(19)

**Europäisches Patentamt**

**European Patent Office**

**Office européen des brevets**

(11) **EP 4 386 384 A1**

(12) **EUROPEAN PATENT APPLICATION**

(43) Date of publication:
**19.06.2024 Bulletin 2024/25**

(21) Application number: **22213839.8**

(22) Date of filing: **15.12.2022**

(51) International Patent Classification (IPC):
***G01N 33/50*** *(2006.01)*

(52) Cooperative Patent Classification (CPC):
**G01N 33/5008; G01N 33/5058; G01N 33/5061**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Georg-August-Universität Göttingen Stiftung Öffentlichen Rechts, Universitätsmedizin 37075 Göttingen (DE)**
• **Repairon Muscle UG (haftungsbeschränkt) 37077 Göttingen (DE)**

(72) Inventors:
• **Zimmermann, Wolfram 37075 Göttingen (DE)**
• **Germeroth, Lothar 37077 Göttingen (DE)**
• **Tiburcy, Malte 37075 Göttingen (DE)**
• **Zafeiriou, Maria-Patapia 37075 Göttingen (DE)**
• **Lutz, Susanne 37075 Göttingen (DE)**

(74) Representative: **Schiweck Weinzierl Koch Patentanwälte Partnerschaft mbB Ganghoferstraße 68 B 80339 München (DE)**

(54) **POTENCY ASSAY FOR PHARMACEUTICAL PRODUCTS**

(57) The present invention describes a method for assessing the potency of a pharmaceutical product assumed to be effective in treating a disorder, preferably a genetic disorder or a non-genetic disorder, wherein the disorder results in an impaired functionality of a physiological bodily function of a subject having the disorder, wherein the method comprises: (i) incubating an engineered tissue with the pharmaceutical product, wherein the engineered tissue is or has been modified to be representative for the disorder; (ii) determining at least one physical parameter of the engineered tissue resulting from step (i), wherein the physical parameter is a measure of the functionality of the physiological bodily function, and (iii) determining whether a value of the at least one physical parameter determined in step (ii) meets a predetermined threshold of said physical parameter, which determines whether the pharmaceutical product has potency, wherein, if said value of the at least one physical parameter exceeds the predetermined threshold, potency of the pharmaceutical product is indicated for treating the disorder by at least partially restoring the functionality of the physiological bodily function.

EP 4 386 384 A1

**Description**

**TECHNICAL FIELD OF THE INVENTION**

The present invention describes a method for assessing the potency of a pharmaceutical product assumed to be effective in treating a disorder, preferably a genetic disorder or a non-genetic disorder, wherein the disorder results in an impaired functionality of a physiological bodily function of a subject having the disorder, wherein the method comprises: (i) incubating an engineered tissue with the pharmaceutical product, wherein the engineered tissue is or has been modified to be representative for the disorder; (ii) determining at least one physical parameter of the engineered tissue resulting from step (i), wherein the physical parameter is a measure of the functionality of the physiological bodily function.

**BACKGROUND**

For testing the clinical application of new therapeutics, especially gene and cell therapeutics in muscle diseases, but also small molecules, the standardized verification of the specific product effect via so-called "potency assays" is of particular importance from a regulatory point of view. Regulatory authorities (EMA, PEI, FDA, etc.) require the establishment and validation of a potency assay for the verification of the quality and especially of the dose-dependent effect of a pharmaceutical product. Especially for Advanced Therapy Medicinal Products (ATMP), variations in potency between manufacturing batches are not uncommon and have a significant impact on clinical application/testing or the interpretation of clinical data. In this regard, the potency assay should test as close as possible to the postulated mode of action of an ATMP.

The verification of the efficacy of ATMPs prior to clinical application is limited with conventional models (animal models, cell lines) and often restricted to surrogate markers with insufficient reference to the postulated mechanism of action (mode of action). Such surrogates usually include protein and gene expression. However, some ATMPs such as Eteplirsen (Exondys 51) have nevertheless been approved by the FDA - even though the FDA does no longer accept surrogate markers: E.g., Golodirsen (Vyondys 53) has only been approved in an appeal procedure due to safety concerns combined with little to no proof of potency. The only surrogate marker used was expression of truncated dystrophin, for which it is unclear whether it is linked to muscle function at all.

Thus, there still is a need for improved potency assays suitable, for example, for regulatory procedures that require showing potency of efficacy of the compounds of interest such as an ATMP. The technical problem therefore is to comply with this need.

**SUMMARY OF THE INVENTION**

The technical problem is solved by the subject matter as defined in the claims. The invention relates to a method for assessing the potency of a pharmaceutical product for treating a disorder, preferably a genetic disorder or a non-genetic disorder. The disorder is preferably characterized by a loss of a bodily function of a tissue, wherein the function can be determined or characterized by a physical parameter such as contractile force, stiffness, and/or one or more multi-electrode array (MEA) measures. For this, an engineered tissue, which is representative for the disorder, is incubated with the pharmaceutical product. After incubation, at least one physical parameter is determined. Since the physical parameter is correlated with the bodily function of the tissue, the physical parameter is a direct measure of the potency of the pharmaceutical product for restoring the bodily function or, in other words, treating the disease.

Accordingly, the present invention relates to a method for assessing the potency of a pharmaceutical product assumed to be effective in treating a disorder, preferably a genetic disorder or a non-genetic disorder, wherein the disorder results in an impaired functionality of a physiological bodily function of a subject having the disorder, wherein the method comprises:

(i) incubating an engineered tissue with the pharmaceutical product, wherein the engineered tissue is or has been modified to be representative for the disorder;
(ii) determining at least one physical parameter of the engineered tissue resulting from step (i), wherein said physical parameter is a measure of the functionality of the physiological bodily function, and
(iii) determining whether a value of the at least one physical parameter determined in step (ii) meets a predetermined threshold of said physical parameter, which determines whether the pharmaceutical product has potency,

wherein, if said value of the at least one physical parameter exceeds the predetermined threshold, potency of the pharmaceutical product is indicated for treating the disorder by at least partially restoring the functionality of the physiological bodily function.

The predetermined threshold is preferably based on and/or obtained by a reference (experiment) using a statistical method, and is more preferably a value deviating from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, optionally expressed as % change of the (mean) reference value.

The engineered tissue preferably comprises one or more of heart tissue, heart muscle, muscle tissue, skeletal muscle tissue, neuronal tissue or connective tissue.

The engineered tissue preferably has been genetically edited to comprise a mutation being seen as causative for the (genetic) disorder. Alternatively, or additionally, the engineered tissue preferably has been subjected to a non-genetic intervention to be representative for the disorder.

The pharmaceutical product preferably is an advanced therapy medicinal product such as a gene therapy medicinal product (GTMP), a cell therapy medicinal product (CTMP), a tissue-engineered product (TEP); a small molecule, a peptide, a protein, a nucleic acid, synthetic RNA, non-coding RNA, a ribonucleoprotein particle (RNP) complex, or a read-through enhancer.

The GTMP preferably is a viral vector, a nanoparticle preferably comprising a nucleic acid and/or proteins, preferably comprised in RNP, or a nucleic acid such as an antisense oligonucleotide. The GTMP preferably is a synthetic or a viral vector, such as a retrovirus such as an adenovirus or an adeno-associated virus (AAV) or a lentivirus, more preferably an AAV is selected from the group consisting of AAV1, AAV2, AAV3, AAV4, AAV5, AAV6, AAV7, AAV8, AAV9, AAV10, AAV11, AAVrh10, AAVrh74 or any combination thereof, preferably AAV2 or AAV9.

The pharmaceutical product preferably comprises a nucleic acid encoding for a protein for gene editing or an expression product thereof such as an endonuclease, e.g., a zinc-finger nuclease (ZFN), a transcription activator-like effector nuclease (TALEN) or a Clustered Regularly Interspaced Short Palindromic Repeats (CRISPR) nuclease.

The GTMP preferably comprises an endonuclease such as Cas9 or Cas13, preferably *Streptococcus pyogenes* Cas9 (SpCas9), *S. aureus* Cas9, *S. auricularis* Cas9, *S. lugdunensis* Cas9, or *N. meningitides* Cas9, and a guide RNA (gRNA). The GTMP preferably comprises a nucleic acid encoding an endonuclease such as Cas9 or Cas13, preferably *Streptococcus pyogenes* Cas9 (SpCas9), *S. aureus* Cas9, *S. auricularis* Cas9, *S. lugdunensis* Cas9, or *N. meningitides* Cas9, and one or more guide RNA (gRNA).

The at least one physical parameter preferably is one or more selected from the group consisting of length, mass, time, electrical current, temperature, luminous intensity, and physical parameters derived from the foregoing.

The at least one physical parameter preferably is one or more from the group consisting of force, movement, electrical current, mass, tissue structure (swelling/condensation, lucidity), and one or more biomechanical parameters (tissue compaction, contraction, stiffness, ultimate strength, elasticity, extensibility, excitability, resilience, toughness, etc).

The at least one physical parameter is preferably one or more selected from the group consisting of tissue contraction, stiffness, force (development), spontaneous beating frequency, contraction time and/or velocity, relaxation time and/or velocity, force (of contraction), contractile force, and one or more multi-electrode array (MEA) measures; preferably measured as pole deflection, Young's modulus, (EHM) shortening, spontaneous beating frequency, contraction time and/or velocity, relaxation time and/or velocity, force of contraction (FOC), twitch tension (TT), resting tension (RT), and one or more from the group consisting of (Weighted) Mean Firing Rate, Burst Frequency (Hz), Interspike Interval in a burst (ms), Network Burst (NB) Frequency, Interspike Interval in a NB (ms), and NB Duration (s), respectively.

The at least one physical parameter preferably is not a gene or protein expression level.

The engineered tissue preferably comprises cells obtained from a patient, who will undergo a treatment with the pharmaceutical product. Alternatively, or additionally, the engineered tissue preferably comprises cells not obtained from the patient, who will undergo a treatment with the pharmaceutical product.

In one embodiment, the engineered tissue that is used herein is heart muscle. Preferably, the at least one physical parameter is force of the engineered tissue and/or movement of the engineered tissue. Preferably, the one or more conditions comprise that the engineered tissue exerts a force of 0.01 mN or more, 0.05 mN or more, 0.1 mN or more, or 1 mN or more. Preferably, the disorder is a genetic heart condition, preferably a genetic heart condition selected from the group consisting of hereditary heart disease such as hereditary forms of dilated, hypertrophic, and arrhythmogenic cardiomyopathies or fibroblastopathies as well as inborn errors of metabolism-associated cardiomyopathies, more preferably a condition selected from the group consisting of Duchenne muscular dystrophy (DMD), Noonan syndrome, diastolic cardiomyopathy, hypertrophic cardiomyopathy, arrhythmogenic right ventricular cardiomyopathy, long and short QT syndrome, Takotsubo syndrome, a lysosomal storage disorder, titinopathy and Barth syndrome. Alternatively, or additionally, the disorder preferably is a non-genetic heart condition, more preferably a non-genetic heart condition induced by simulated neurohormonal and/or pharmacological stimulation (e.g. by a catecholamine, an angiotensin, and/or transforming growth factor (TGF) β), by a drug such as doxorubicin, a tyrosine kinase inhibitor and/or a cardiotoxic drug, by mechanical injury such as crush injury, by thermal injury such as freezing or overheating, by a biophysical injury such as radiation injury, by infection such as a virus infection with cardiotropic virions such as coxsackie, SarsCov, cytomegaly virus and Dengue virus, or a parasitic infection such as Chagas disease with Trypanosoma cruzi, optionally by a bodily fluid such as a serum from a patient with a heart disease, and/or optionally by cells such as blood derived mononuclear cell cytes (e.g., T-cells, B-cells, NK-cells, macrophages) from patients with heart disease.

In one embodiment, the engineered tissue is neuronal tissue, preferably a neuronal organoid. Preferably, the at least one physical parameter is electrical current and/or activity. Preferably, the one or more conditions comprise local and coordinated electrical bursts, and/or clusters. Preferably, wherein the disorder is a neuronal condition, preferably selected from the group consisting of neurodegenerative diseases (e.g., dementia, Parkinson disease, M. Huntington), neuroinflammatory diseases (e.g., multiple sclerosis), brain inflammation disorders (e.g., meningitis), channelopathy (e.g., epilepsy) and psychiatric diseases (including autism and schizophrenia).

In one embodiment, the engineered tissue is muscle tissue, preferably skeletal muscle tissue. Preferably, wherein the at least one physical parameter is force or movement. Preferably, the one or more conditions comprise that the engineered tissue exerts a force of 0.01 mN or more, 0.05 mN or more, 0.1 mN or more, or 1 mN or more. Preferably, the disorder is a genetic muscular disorder, preferably a genetic muscular disorder selected from the group consisting of Duchenne muscular dystrophy, Facioscapulohumeral dystrophy, Becker dystrophy, Emery-Dreifuss dystrophy, myotonic dystrophy, limb-girdle dystrophy, oculopharyngeal muscular dystrophy, congenital dystrophies, congenital myopathies, myotonia congenita, inflammatory myopathies, familial periodic paralysis, and a heritable connective tissue disease such as Marfan syndrome, Ehlers-Danlos syndrome or Loeys-Dietz syndrome.

In one embodiment, the engineered tissue is a composition of different types of tissues, preferably a composition of: (i) neurons and skeletal muscle, or (ii) neurons and heart muscle; wherein more preferably the composition of different types of tissues forms one or more neuro-muscular junctions.

In one particular embodiment,

the engineered tissue is heart muscle or skeletal muscle,
the pharmaceutical product is an AAV2 and/or AAV9 viral vector comprising a nucleic acid encoding Cas9, preferably *S. aureus* Cas9, *S. auricularis* Cas9, *S. lugdunensis* Cas9, or *N. meningitides* Cas9, and one or two guide RNA(s) (gRNA), wherein the Cas9 gene is under the control of a muscle-specific promoter such as CK8e or TNNT2,
the disorder is Duchenne muscular dystrophy, and
said physical parameter is force,
preferably wherein the engineered tissue comprises cells obtained from a patient, who will undergo a treatment with the pharmaceutical product.

In one embodiment, the engineered tissue is connective tissue. Preferably, the at least one physical parameter is a biomechanical parameter such as tissue compaction, contraction, stiffness, ultimate strength, elasticity, extensibility, excitability, resilience, or toughness. Preferably, the disorder is (i) a heart disease which presents with cardiac fibrosis, such as a heart failure (of unknown and/or genetic cause) with reduced or preserved ejection fraction, myocardial infarction, a congenital heart disease, a cardiomyopathy with known mutation(s), age- and/or senescence-induced cardiac fibrosis, a drug-induced cardiac disorder, a metabolic cardiac disorder, a (monogenetic or polygenetic) disorder with contribution of the heart and cardiac fibrosis, a disease involving reactive or replacement fibrosis in an organ such as kidney, liver, lung, and/or skin, etc., (ii) a disorder with impaired scaring and wound healing such as Diabetes mellitus, and/or (iii) a heritable connective tissue disease such as Marfan syndrome, Ehlers-Danlos syndrome or Loeys-Dietz syndrome.

## BRIEF DESCRIPTION OF THE DRAWINGS

The invention will be better understood with reference to the detailed description when considered in conjunction with the non-limiting examples and the accompanying drawings, in which:

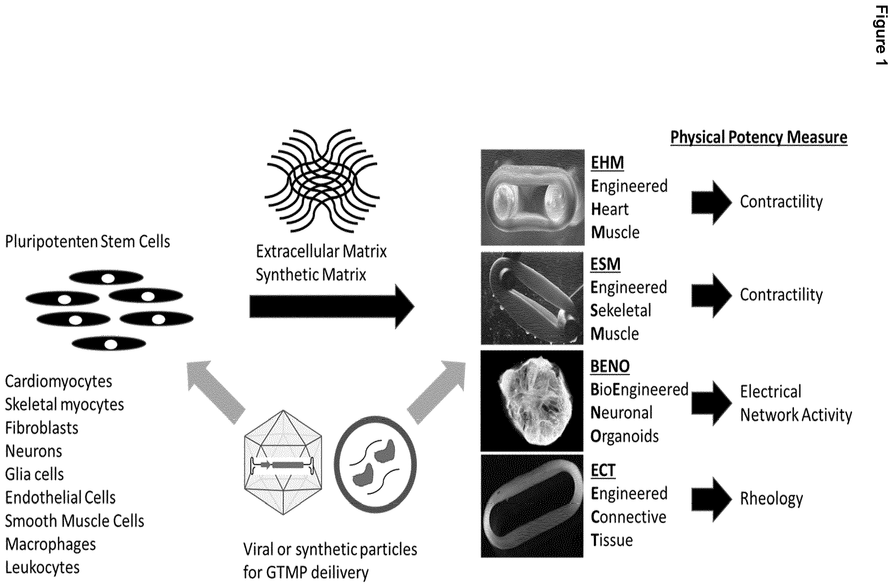

Figure 1 shows exemplary potency assays. Pluripotent stem cells or their differentiated derivatives can be used as the cellular starting material, which may be reconstituted in biological or synthetic extracellular matrix, preferably collagen type I, to facilitate the formation of the desired engineered tissue and/or organoid type, e.g., engineered human myocardium (EHM), engineered skeletal muscle (ESM), bioengineered neuronal organoid (BENO), engineered connective tissue (ECT). Engineered tissue are subject to specific functional assays with physical potency read-outs, such as force of contraction (measured in Newton, typically $\mu$N to mN), network activity (measured in Volt, typically $\mu$V), and viscoelastic and elastic modulus (both measured in Pascal, typically in kPa), can be precisely measured as a function of experimental drug exposure, e.g., after delivery of nucleotide therapeutics by viral (such as AAV) or non-viral (such as synthetic lipid nanoparticles or extracellular vesicles) particles, or small molecules.

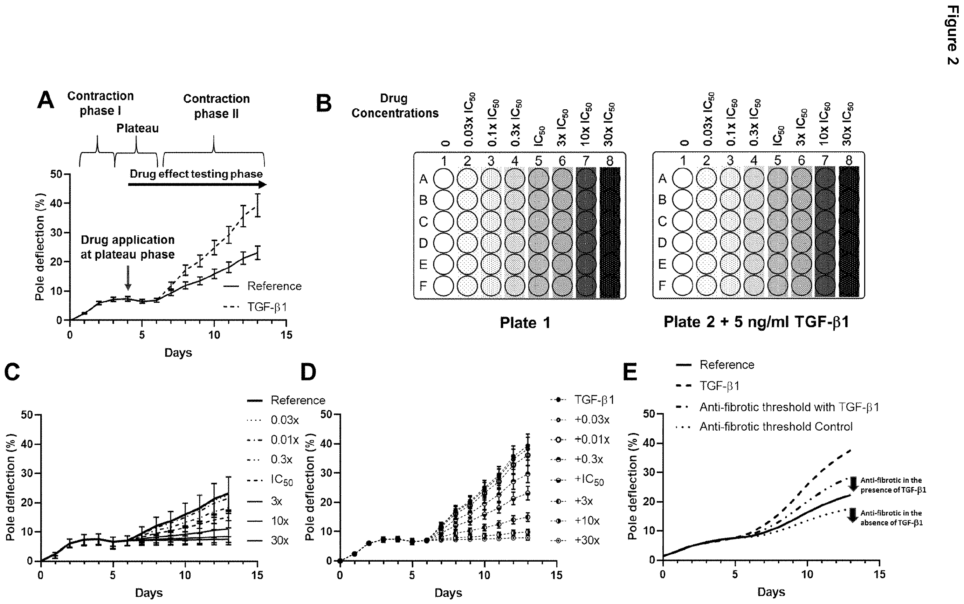

Figure 2 shows pole deflection analyses to determine the anti-fibrotic potency of a drug. A) iPSC-StC/fibroblasts were used to generate ECT. 5 ng/ml TGF-$\beta$1 was applied at day 4. Pole deflection was analyzed every day. A biphasic contraction reaction was observed. B) Exemplary design of an experiment with a drug of which the $IC_{50}$ was known. Theoretical and/or illustrative result of an "ideal" anti-fibrotic drug in the C) absence and D) presence of TGF-β1. E) Schematic illustration of the thresholds for an anti-fibrotic action given in the absence (dotted line) and presence (dotted-dashed line) of TGF-β1 over the drug effect testing phase. It is to be noted for **Figure 2** that the reference trace represents experimental data, and that "Reference" denotes the untreated ECT reference and "TGFβ-1" the TGFβ-1 ECT reference.

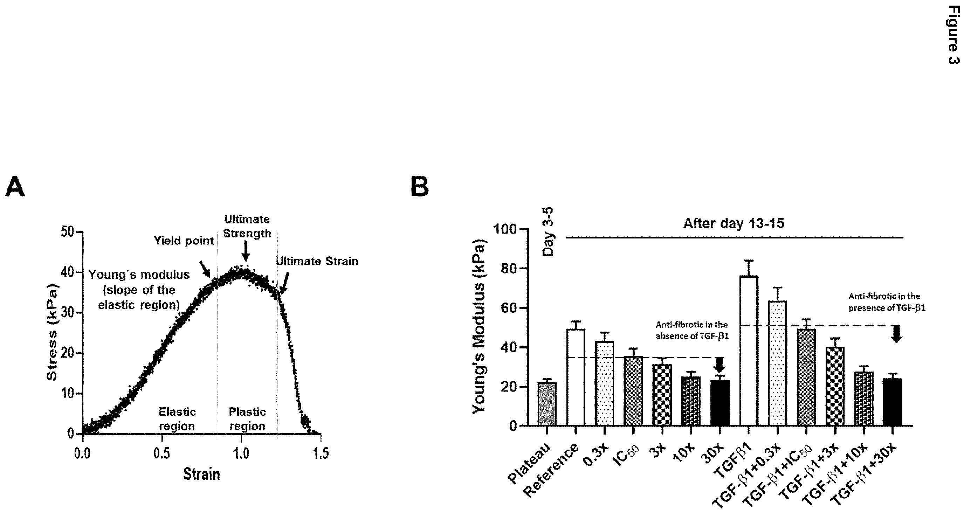

**Figure 3** shows ultimate tensile testing analyses to determine the anti-fibrotic potency of a drug. iPSC-StC / fibroblasts were used to generate ECT. 5 ng/ml TGF-β1 and the drug were applied, when the plateau phase was reached. Ultimate tensile testing of a subset of untreated ECT was performed before drug application and the rest was analyzed 10 days later. A) A representative stress-strain curve with important biomechanical parameters was presented. The Young's modulus (slope of the curve within the elastic phase) indicated the stiffness of an ECT. B) Theoretical and/or illustrative result on the stiffness of an "ideal" anti-fibrotic drug in the absence (bar plots on the left) and presence (bar plots on the right) of TGF-β1, preferably obtained (approximately) on day 10 of the drug effect testing phase. Illustrative significance levels are indicated by dashed lines. It is to be noted for **Figure 3** that plateau and reference stiffness represent experimental data and that "Reference" denotes the untreated ECT reference and "TGFβ-1" the TGFβ-1 ECT reference.

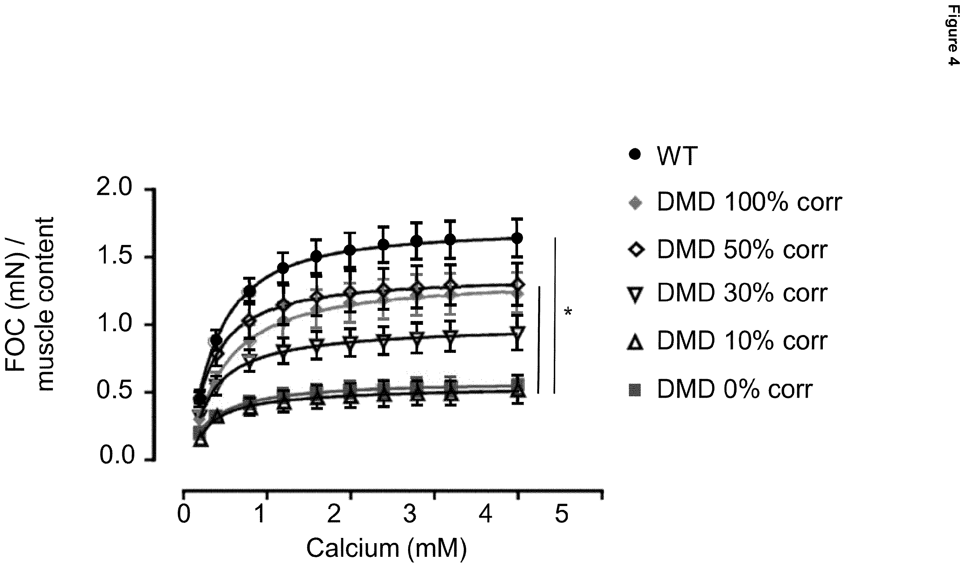

**Figure 4** shows genetic correction of a Duchenne muscular dystrophy (DMD) EHM model. Force of contraction (FOC; mN) normalized to muscle content of EHM titrated to contain 0, 10, 30, 50, or 100% genetically corrected cardiomyocytes; n=4-8, *p<0.05 by 2-way ANOVA and Tukey's multiple comparison test. Source: Long et al. (2018), Sci Adv, 4:eaap9004.

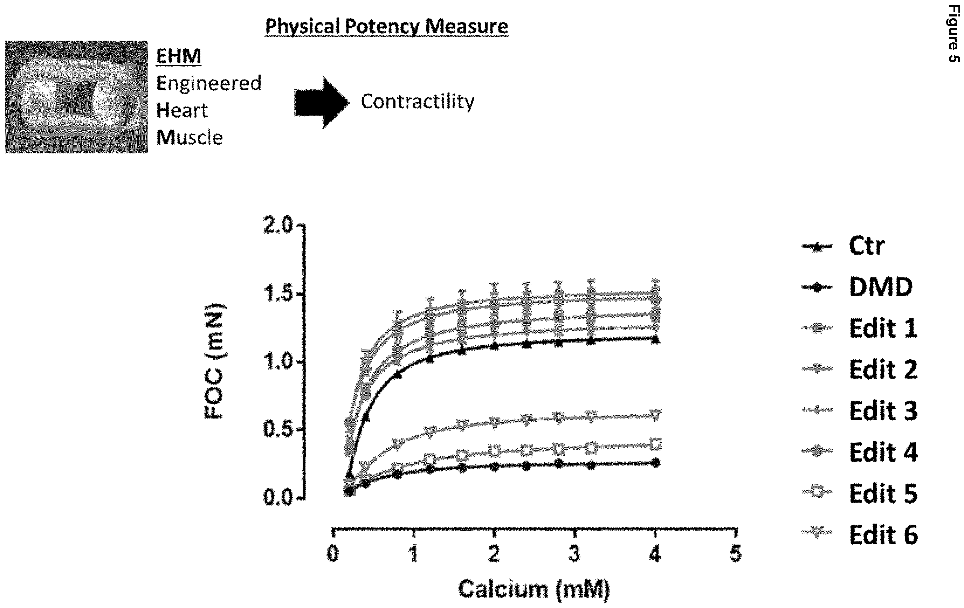

**Figure 5** shows an exemplarily physical potency measure in case of EHM is shown focusing on measuring contractility as force of contraction (FOC; mN) as a function of external Calcium concentration (mM). As it can be seen, in case of a DMD disease model (DMD - black circles), FOC is drastically reduced compared to a reference EHM (black triangles). EDIT 1-6 curves represent EHM from the DMD model after "therapeutic" genome editing with full functional restoration in EDITs 1-4 (filled grey symbols) and only minimal functional restoration in EDITs 5-6 (empty grey symbols), providing strong evidence for "potency" in the CRISPR-edits in the EDIT 1-4 models and only limited "potency" in CRISPR-edits in the EDIT 2-6 models.

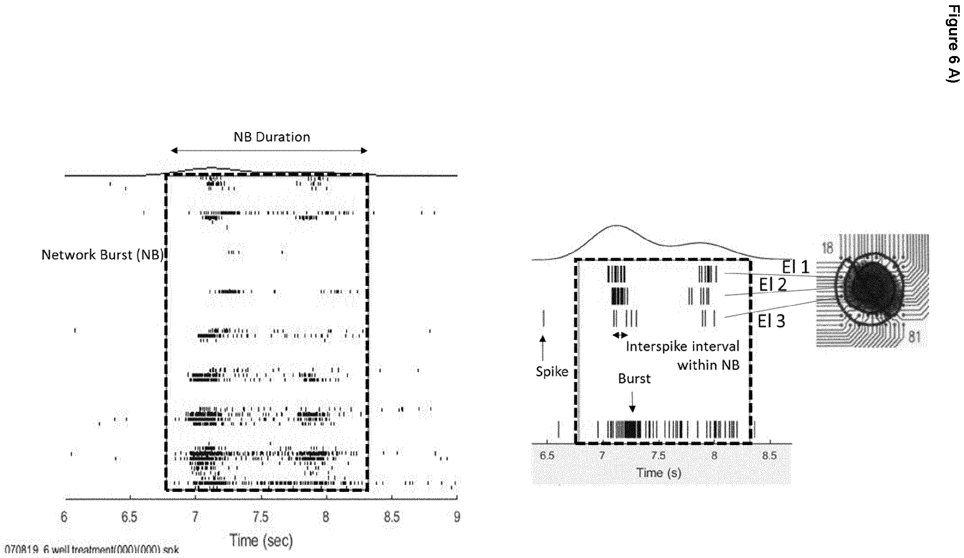

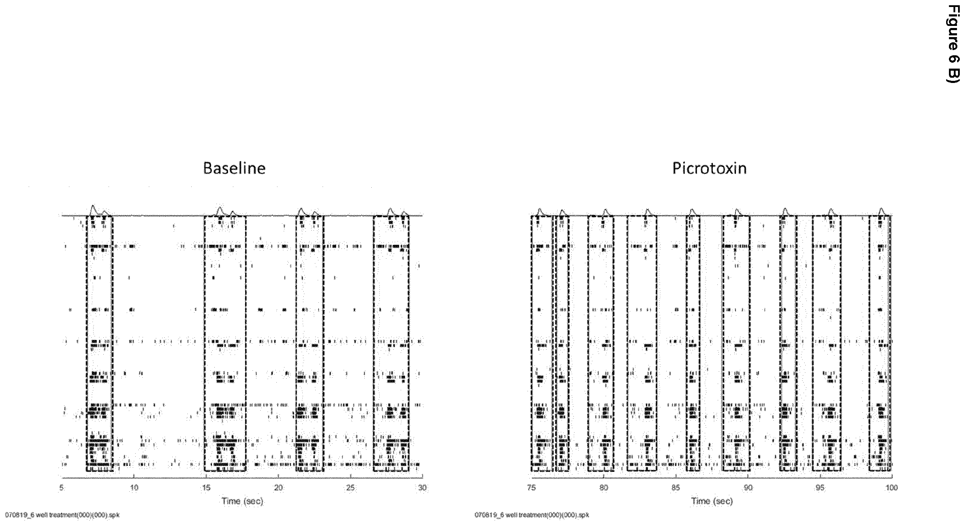

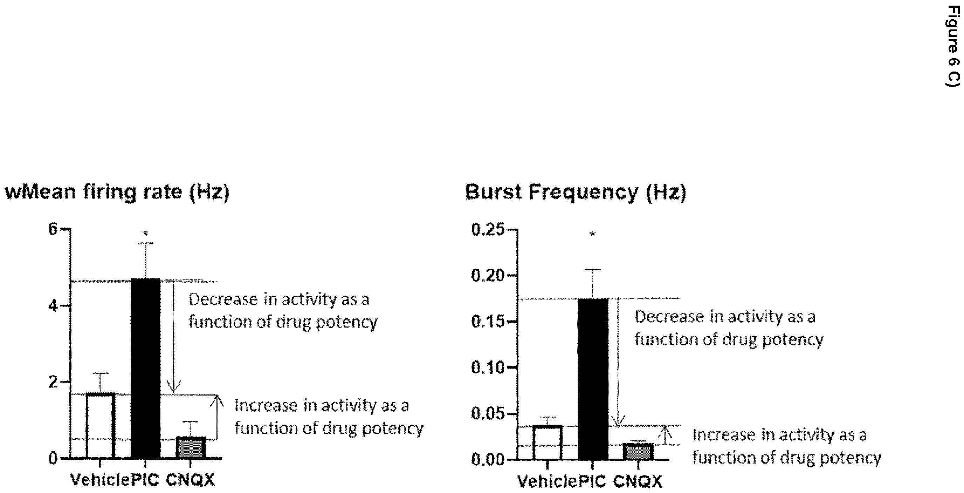

**Figure 6** shows illustrative raster plots of neuronal network activity in a BENO (day 60). A) Example of a raster plot in which every electrode / channel activity is depicted in a row (left and middle). Every spike is indicated with a black line on top of a respective plot, a burst by black lines in the main section of the respective plot and a network burst showing synchronicity between bursts is highlighted by a square with dashed lines. Parameters such as spike, burst, network burst duration, interspike interval, interburst interval are graphically indicated in A). Furthermore, on the right side a picture of a BENO is shown that was placed on a multi-electrode array well and covered by a coil that stabilized the tissue on top of the electrodes. Exemplarily, three electrodes (EL 1-3) are indicated, which record local field potentials. The component activity recorded from each electrode in time is displayed in the raster plot (middle), in which each electrode is represented in a row. B) shows representative activity raster plots of the same well, prior and after picrotoxin treatment. An increase in firing rate and burst frequency was observed that was indicative for a pro-convulsive drug. C) illustrates exemplarily the effect of PIC in case of the two network activity parameters (weighted) mean firing rate (left bar plot) and burst frequency (right bar plot) as shown in **Table 14** (day 60) with horizontal lines indicating the respective means of the PIC treated (middle) and the CNQX treated (right) BENO, respectively, as well as of the empty vehicle treated BENO used as reference (left). Statistics: n=4 tissues / group, one-way ANOVA or non-parametric Students t-test, * p<0.05. The GABA-receptor antagonist Picrotoxin (PIC) was applied as a pro-convulsive compound, resulting in an increased mean (weighted) firing rate and an increases mean burst frequency. In contrast, incubation with the AMPA/kainate receptor antagonist Cyanquixaline (CNQX) resulted in both a reduced mean weighted firing rate and mean burst frequency compared to reference and PIC BENO, indicative for an anti-convulsive activity.

## DETAILED DESCRIPTION OF THE INVENTION

The present invention is described in detail in the following and will also be further illustrated by the appended examples and figures.

In the present invention it is shown that engineered tissues can be used in potency assays for pharmaceutical products. These potency assays may, for example be used to comply with the requirement of the drug approval process to show the claimed efficacy of the drug candidate molecule. These potency assays may also be used, for example, once a drug has been approved, as a potency assay in the GMP manufacturing process of the approved drug. In that case, for example, a batch of the pharmaceutical product may be released for its use as pharmaceutical if this batch meets the pre-determined threshold (value) as described herein. In such a case, the threshold value as described herein may thus serve as the release criterion in the GMP drug manufacturing process. In accordance with the above disclosure, the invention may thus relate to a method for assessing the potency of a pharmaceutical product for treating a disorder, such as a genetic disorder or a non-genetic disorder. The disorder may be characterized by a loss of a bodily function of a tissue, wherein the function of the tissue can be determined or characterized by a physical parameter such as force. For this, an engineered tissue, which is representative for the disorder, is brought into contact (incubated) with the pharmaceutical product. After incubation, at least one physical parameter is determined. Since the physical parameter is correlated with the bodily function of the tissue, the physical parameter is a direct measure of the potency of the pharmaceutical product for restoring the bodily function or, in other words, treating the disease.

Accordingly, the present invention relates to a method for assessing the potency of a pharmaceutical product assumed to be effective in treating a disorder, preferably a genetic disorder or a non-genetic disorder, wherein the disorder results in an impaired functionality of a physiological bodily function of a subject having the disorder, wherein the method comprises:

(i) incubating an engineered tissue with the pharmaceutical product, wherein the engineered tissue is or has been modified to be representative for the disorder;
(ii) determining at least one physical parameter of the engineered tissue resulting from step (i), wherein said physical parameter is a measure of the functionality of the physiological bodily function.

The disorder, preferably but not necessarily a genetic disorder, is characterized by an impaired functionality of a physiological bodily function. A "physiological bodily function" as used herein relates to a function that is typically associated with a given tissue, organ or cell. Illustrative examples include muscles, which exert a force and/or an electrical signal as physiological bodily function, e.g., heart muscle, which pumps blood through the circulatory system by its muscle cell contraction; skeletal muscle, which is essential for all voluntary movements and determined by the force of contraction generated by skeletal muscle myofibers; connective tissue, which is produced predominantly by stromal cells (fibroblasts outside the brain, glia cells inside the brain) and provides mechanical stability to any given tissue by extracellular matrix production (such as collagens) and by their intrinsic contractility (such as well-known for wound healing) or neurons, which provide electrical currents distinguishable as individual neuron and neuronal network activity, which is key for their physiological bodily function. A disorder within the context of the invention causes a partial or complete loss of the physiological bodily function. An "impaired functionality" as used herein refers to the inability of an organ, tissue or cell of a subject to exert its normal physiological bodily function. "Impaired" may in the context of the invention relate to a total loss of function (100% loss), whereas also a partial loss of function, e.g., by at least about 50%, at least about 60%, at least about 70%, at least about 80%, at least about 90% or at least about 95% of function relative to the physiological bodily function (of an engineered tissue) of a healthy subject. The functionality of the physiological bodily function can be expressed in physical parameters. E.g., the force exerted by a muscle. Thus, an impaired functionality of a physiological bodily function may also relate to a reduction of a value of the at least one physical parameter by at least about 15%, at least about 20%, at least about 25%, at least about 30%, at least about 35%, by at least about 40%, at least about 45%, by at least about 50%, at least about 60%, at least about 70%, at least about 80%, at least about 90%, at least about 95% or about 100% relative to a physical parameter value determined, e.g., with (an engineered tissue) of a healthy subject. A "healthy subject" as used herein relates to a subject, which does not suffer from a disorder, preferably does not suffer from the disorder, for which the pharmaceutical product is assumed to be effective in treating.

"Potency" as used herein refers to the ability of a pharmaceutical product to produce an effect of given intensity. Depending on the given intensity, which is within the context of the invention expressed in terms of at least one physical parameter, a partial or full restoration of the bodily function can be seen as the effect. Such a partial or full restoration of the bodily function can also be seen as a successful treatment. I.e., "being effective in treating" may refer to a partial or full restoration of the physiological bodily function. "Treating" or "treatment" or "alleviation" refers to both therapeutic treatment and prophylactic or preventative measures, wherein the object is to prevent, slow down (lessen) or at least partially alleviate or abrogate an abnormal, including pathologic, condition in the organism. Those in need of treatment include those already with the disorder as well as those prone to having the disorder or those in whom the disorder is to be prevented (prophylaxis). Potency can thus be described for example as increasing or decreasing a value of the at least one physical parameter to a value that corresponds to at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 95% or about 100% relative to a value of the at least one physical parameter determined, e.g., with (an engineered tissue) of a healthy subject. Herein, potency of a pharmaceutical product is assessed in step (iii) by determining whether a value of the at least one physical parameter determined in step (ii) meets a predetermined threshold of said physical parameter, which determines whether the pharmaceutical product has potency, wherein, if said value of the at least one physical parameter exceeds the predetermined threshold, potency of the pharmaceutical product is indicated for treating the disorder by at least partially restoring the functionality of the physiological bodily function. Details on the potency determination of a pharmaceutical product are disclosed herein further below.

## Pharmaceutical products

As outlined herein, the method of the present invention is useful in determining whether a pharmaceutical product has potency in treating a disorder. The method of the invention can be applied to a variety of different pharmaceutical products. However, the method of the invention is particularly useful with pharmaceutical products that include gene therapy or alter (i.e., increase or decrease) gene expression or expression of a protein encoded by said gene such as ATMP. Accordingly, the pharmaceutical product preferably is an advanced therapy medicinal product (ATMP) such as a gene therapy medicinal product (GTMP), a cell therapy medicinal product (CTMP), or a tissue-engineered product (TEP); a small molecule, a peptide, a protein, a nucleic acid, synthetic RNA, non-coding RNA, a ribonucleoprotein particle (RNP) complex, or a read-through enhancer. Alternatively, or additionally, the term "pharmaceutical product" preferably encompasses also (chemical and/or pharmaceutical) substances under development, meaning drug candidates that are in preclinical or clinical development.

In one preferred embodiment, the pharmaceutical product is an advanced therapy medicinal product (ATMP). ATMPs are, e.g., described and defined in Article 2 of the regulation (EC) No 1394/2007 of the European Parliament and of the Council of 13 November 2007 on advanced therapy medicinal products and amending Directive 2001/83/EC and Regulation (EC) No 726/2004. In particular, ATMPs include a gene therapy medicinal product, preferably as defined in Part IV of Annex I to Directive 2001/83/EC, a somatic cell therapy medicinal product, preferably as defined in Part IV of Annex I to Directive 2001/83/EC, or a tissue engineered product (TEP). ATMPs are also described, e.g., in Goula et al. 2020, J Clin Med Res, 12(12):780-786.

In one embodiment, the pharmaceutical product is a GTMP. A "gene therapy medicinal product" (GTMP) as used herein relates to a biological medicinal product which has the following characteristics: (a) it contains an active substance which contains or consists of a recombinant nucleic acid used in or administered to a subject or patient with a view to regulating, repairing, replacing, adding or deleting a genetic sequence; and (b) its therapeutic, prophylactic or diagnostic effect relates directly to the recombinant nucleic acid sequence it contains, or to the product of genetic expression of this sequence. Gene therapy medicinal products preferably do not include vaccines against infectious diseases. GTMPs are particularly useful for treating disorders such as inherited/genetic disorders, cancer and in tissue regeneration, e.g., lost of sight. Exemplary GTMPs include, but are not limited to, Idecabtagene vicleucel (sold under the brand name Abecma®), Talimogene laherparepvec (sold under the brand name Imlygic®), Tisagenlecleucel (sold under the brand name Kymriah®), Atidarsagene autotemcel (sold under the brand name Libmeldy®), Voretigene neparvovec (sold under its brand name Luxturna®), Strimvelis (an autologous CD34+ enriched cell fraction that contains CD34+ cells transduced with retroviral vector that encodes for the human ADA cDNA sequence), Brexucabtagene autoleucel (sold under its brand name Tecartus®), Axicabtagene ciloleucel (sold under is brand name Yescarta®), Onasemnogene abeparvovec (sold under its brand name Zolgensma®), or Betibeglogene autotemcel (sold under its brand name Zynteglo®).

In one embodiment, the pharmaceutical product is a CTMP. A "cell therapy medicinal product" (CTMP) as used herein encompasses genetically modified CTMP and/or SCTMP. An example of a genetically modified CTMP may be a CAR-T cell therapeutics CAR-T cell therapeutics may be particularly useful for treating disorders like lymphoma. A "somatic cell therapy medicinal product" (SCTMP) a used herein relates to a biological medicinal product which has the following characteristics: (a) contains or consists of cells or tissues that have been subject to substantial manipulation so that biological characteristics, physiological functions or structural properties relevant for the intended clinical use have been altered, or of cells or tissues that are not intended to be used for the same essential function(s) in the recipient and the donor; and (b) is presented as having properties for, or is used in or administered to human beings with a view to treating, preventing or diagnosing a disease through the pharmacological, immunological or metabolic action of its cells or tissues. SCTMP are particularly useful for treating disorders such as immune disease, Parkinson's disease, Amyotrophic lateral sclerosis (ALS), Alzheimer's disease, cartilage defect, and can be used in cardiac repair, skin replacement or cancer immunotherapy. Exemplary SCTMPs include, but are not limited to, Alofisel or AMESANAR.

In one embodiment the pharmaceutical product is a TEP. A "tissue engineered product" (TEP) and an "engineered tissues" as used herein relates to a product that comprises or consists of engineered cells or tissues, and is presented as having properties for, or is used in or administered to human beings with a view to regenerating, repairing or replacing a human tissue. A tissue engineered product may contain cells or tissues of human or animal origin, or both. The cells or tissues may be viable or non-viable. It may also contain additional substances, such as cellular products, bio-molecules, bio-materials, chemical substances, scaffolds or matrices. Cells or tissues can be considered 'engineered' if they fulfil at least one of the following conditions: the cells or tissues have been subject to substantial manipulation,

so that biological characteristics, physiological functions or structural properties relevant for the intended regeneration, repair or replacement are achieved and/or the cells or tissues are not intended to be used for the same essential function or functions in the recipient as in the donor. Products consisting exclusively of non-viable human or animal cells and/or tissues, which do not contain any viable cells or tissues and which do not act principally by pharmacological, immunological or metabolic action, shall be excluded from this definition. TEPs can be used for small diameter vascular grafts, trachea replacement, tissue-engineered esophagus, liver or kidney implantation or nerve conduit. Exemplary TEPs include, but are not limited to, Holoclar, MACI, MukCell, NOVOCART 3D, NOVOCART Inject, Obnitix, Spherox, t2c001. Alternatively, or optionally, exemplarily TEPs include, but are not limited to, TEPs designed to release biologically active factors like growth factors, cytokines, non-coding RNA for example directly or via exosomes etc. (cf. e.g. Zimmermann WH, Soong PL: Pouch-like structure with paracrine activity and method for its preparation, EP 2842581 A1 and/or US 9801817 B2).

Non-substantial manipulations are, e.g., cutting, grinding, shaping, centrifugation, soaking in antibiotic or antimicrobial solutions, sterilization, irradiation, cell separation, concentration or purification, filtering, lyophylization, freezing, cryopreservation, vitrification.

In one embodiment, the pharmaceutical product is a small molecule. A small molecule or micromolecule is a low molecular weight ($\leq$ 1000 Daltons, preferably $\leq$ 500 Daltons) organic compound that may regulate a biological process, preferably with a size on the order of 1 nm. Larger structures such as nucleic acids and proteins, and many polysaccharides are not small molecules, although their constituent monomers (ribo- or deoxyribonucleotides, amino acids, and monosaccharides, respectively) are often considered small molecules. Small molecules may be used as research tools to probe biological function as well as leads in the development of new therapeutic agents. Some can inhibit a specific function of a protein or disrupt protein-protein interactions. In one particular example, the pharmaceutical product is AMX0035 (Elybrio®), which is a composition comprising sodium phenylbutyrate and taurursodiol and useful for treatment of amyotrophic lateral sclerosis (ALS), Alzheimer's disease, and Wolfram syndrome. When analyzed for potency with a potency assay described herein, the engineered tissue can be neuronal tissue or a neuro-muscular junction. In such an assay, the physical parameter may be electrical activity or, in case a motor plate is employed, also the force exerted by muscle tissue in contact with said neuro-muscular junction. Further exemplary small molecules include Tafamidis in the context of Transthyretin amyloidosis, Ataluren in the context of Duchenne muscular dystrophy (DMD) caused by nonsense mutations and Migalastat in the context of Fabry disease.

In one embodiment, the pharmaceutical product is a peptide. In another embodiment, the pharmaceutical product is a protein. The distinction between peptides and proteins is open for debate, but for the purpose of the present invention, a peptide comprises 20 or less amino acids while a protein comprises more than 20 amino acids in an amino acid chain. Exemplary peptides include insulin. Exemplary proteins include proteins in enzyme-replacement therapy, e.g., in Fabry disease (agalsidase-$\alpha$/$\beta$) or in Pompe disease also known as Glycogen storage disease type II (Alglucosidase-$\alpha$).

In one embodiment, the pharmaceutical product is a nucleic acid. In the present invention, the term "nucleic acid" generally includes both DNA and RNA. However, nucleic acid whose nucleotide is replaced by an artificial derivative or modified nucleic acid from natural DNA or RNA ("artificial" or "synthetic" DNA or RNA) can also be comprised by the nucleic acid in the context of the present invention. The pharmaceutical product may be RNA. The pharmaceutical product may be DNA. The term nucleic acid may also comprise phosphorodiamidate morpholino oligomers (PMO). In one embodiment, the pharmaceutical product is Eteplirsen, e.g., sold as Exondys 510 by Sarepta Therapeutics. In this particular embodiment, the engineered tissue may be (heart) muscle tissue and the physical parameter may be force. The term nucleic acid may also comprise peptide-conjugated PMO (PPMO). The term nucleic acid may also comprise synthetic RNA. The term nucleic acid may also comprise non-coding RNA. The term nucleic acid may also comprise locked nucleic acid (LNA), aptamers. The term nucleic acid may also comprise single-stranded nucleic acids designed to bind and inhibit endogenous microRNA (miRNA) molecules (Anti-miR™). The term nucleic acid may also comprise circular RNA. The term nucleic acid may also comprise decoy RNA. The pharmaceutical product may also be a ribonucleoprotein particle (RNP). An RNP may relate to a complex formed between RNA and RNA-binding proteins. Alternatively, or additionally, an RNP may relate to a gapmer like a short (antisense) DNA strand flanked by one or more RNA-like segments and/or strands of RNA mimics like locked nucleic acids (LNA).

In one particular example, pharmaceutical product is WVE-004 obtainable by Wave Life Sciences USA, Inc. WVE-004 is an antisense oligonucleotide (ASO) targeting the C9ORF72 mutation that causes amyotrophic lateral sclerosis and frontotemporal dementia. The ASO mediates degradation of hexanucleotide expansion-containing C9ORF72 mRNAs. Both the mRNA and the dipeptide proteins produced from the hexanucleotide repeats are believed to be toxic to neurons, hence the rationale that reducing them will be beneficial. WVE-004 can be tested in a potency assay described herein, wherein the engineered tissue is neuronal tissue or a neuro-muscular junction, preferably carrying the human C9ORF72 gene with a repeat expansion. In such an assay, the physical parameter may be electrical activity or, in case a neuro-muscular junction is employed, also the force exerted by muscle tissue in contact with said motor plate.

The pharmaceutical product may also be a read-through enhancer. It has been found that the binding of aminoglycosides, such as gentamicin and G418, to eukaryotic ribosomes alters the architecture of the ribosomal decoding center, thereby enabling pairing of a near-cognate aminoacyl-tRNA complex to a premature termination codon (PTC) and incorporation of an amino acid instead of premature translation termination. This process, termed PTC read-through (PTCR), enables formation of full-length protein from mRNAs bearing a PTC and functional rescue. Examples of read-through enhancers include 2-Aminothiazole-4-carboxamides such as Amlexanox, PTC 124, RTC13, RTC14, RTC204, RTC219, GJ071, GJ072, or Ataluren, which are, e.g., described in Rabea et al. (2019), ACS Med. Chem. Lett., 10(5):726-731; the corresponding chemical structures of the compounds are hereby incorporated by reference.

In a preferred embodiment, the GTMP is a viral vector. Viral vectors may comprise retroviruses such as lentiviruses, adenoviruses or adeno-associated viruses (AAV). Preferably, the viral vector is an AAV, more preferably an AAV selected from the group consisting of AAV1, AAV2, AAV3, AAV4, AAV5, AAV6, AAV7, AAV8, AAV9, AAV10, AAV11 or any combination thereof, most preferably AAV2 or AAV9. Alternatively, AAV with a non-human natural host, such as rhAAV or synthetic vectors may be used.

The GTMP may also be a nanoparticle preferably comprising a nucleic acid and/or proteins, preferably comprised in RNP. A nanoparticle may be a synthetic or a biological nanoparticle. A biological nanoparticle may be for example a microvesicle, an exosome or the like. Nanoparticles are, e.g., described in Li et al. (2007), Pharm Res, 24(3):438-49, hereby incorporated by reference. The pharmaceutical product may also be a nucleic acid such as an antisense oligonucleotide. The pharmaceutical product may also be an extracellular vesicle, e.g., isolated from supernatants of cells such as iPSC, wherein the cells were modified to produce a specific cargo to be included in the extracellular vesicles.

In order to achieve the therapeutic, prophylactic or diagnostic effect, the pharmaceutical product may comprise a protein for gene editing such as an endonuclease, e.g., a zinc-finger nuclease (ZFN), a transcription activator-like effector nuclease (TALEN), a Clustered Regularly Interspaced Short Palindromic Repeats (CRISPR) nuclease, a nuclease-dead endonuclease, or an endonuclease engineered to comprise base and prime editing protein components. Alternatively, or additionally, the pharmaceutical product may comprise a nucleic acid, which when expressed leads to the production of a zinc-finger nuclease (ZFN), a transcription activator-like effector nuclease (TALEN), a Clustered Regularly Interspaced Short Palindromic Repeats (CRISPR) nuclease, a nuclease-dead endonuclease, or an endonuclease engineered to comprise base and prime editing protein components. Preferably, the pharmaceutical product comprises the GTMP comprises an endonuclease such as Cas9 or Cas13, preferably *Streptococcus pyogenes* Cas9 (SpCas9), *S. aureus* Cas9, *Staphylococcus auricularis* Cas9, *Staphylococcus lugdunensis* Cas9, or *N Neisseria meningitides* Cas9, and a guide RNA (gRNA); or comprises a nucleic acid encoding an endonuclease such as Cas9 or Cas13, preferably *Streptococcus pyogenes* Cas9 (SpCas9), *Streptococcus aureus* Cas9, *Streptococcus auricularis* Cas9, *Streptococcus lugdunensis* Cas9, or *Neisseria meningitides* Cas9, and a guide RNA (gRNA).

**Physical parameters**

As described herein, an important advantage of the present invention is that is does not make use of surrogate markers such as protein or gene expression alone but directly measures the functionality of a physiological bodily function of the engineered tissue or organ by determining a physical parameter - e.g., the force exerted by an engineered (heart and/or skeletal) muscle or connective tissue, or network activity in an engineered neuronal tissue like a BENO - after incubating the engineered tissue with a pharmaceutical product. This allows a direct assessment whether the pharmaceutical product has potency to restore the functionality of a physiological bodily function in comparison to, e.g., a diseased state such as observed in muscular dystrophies, fibroblastopathies, or neuro-degeneration. The method of the present invention does not exclude the additional analysis of a biomarker such as gene or protein expression of the biomarker to provide multi-modal information as to a disease phenotype and its pharmacological salvage using an "precision medicine" approach. Accordingly, the method of the invention preferably further comprises the analysis of a biomarker such as gene or protein expression of the biomarker, wherein the biomarker is preferably a biomarker of clinical relevance. The at least one physical parameter however preferably is not gene or protein expression.

A "physical parameter" or a "physical quantity", both of which are used interchangeably throughout this application, relates to a physical property of a material or system that can be quantified by measurement. The physical parameter can be expressed as a value, which is the algebraic multiplication of a numerical value and a unit. For example, the physical parameter mass can be quantified as n kg, where n is the numerical value and kg is the unit.

The physical parameters can be divided into base parameters and derived parameters. The base parameters of a given system of physical quantities is a subset of those quantities, where no base quantity can be expressed in terms of the others, but where every quantity in the system can be expressed in terms of the base quantities. Preferred base parameters can be selected from the group consisting of length, mass, time, electrical current, temperature, and luminous intensity. Accordingly, the physical parameter preferably is one or more selected from the group consisting of length, mass, time, electrical current, temperature, luminous intensity, amount of substance and physical parameters derived from the foregoing. The physical parameter more preferably is one or more selected from the group consisting of length, mass, time, electrical current, temperature, luminous intensity, and physical parameters derived from the foregoing. Preferably, the at least one physical parameter is not an amount of a molecule such as a gene product or a protein.

A derived quantity or physical parameter is a quantity in a system of quantities that is defined in terms of only the base quantities of that system. Accordingly, the physical parameter preferably is one or more selected from the group consisting of force, movement, electrical current, mass, tissue structure (swelling/condensation, lucidity), or a biomechanical parameter such as tissue compaction, contraction, stiffness, ultimate strength, elasticity, extensibility, excitability, resilience, toughness, etc. Herein, rheological parameters encompass biomechanical parameters disclosed herein. Hence, alternatively and/or optionally, the physical parameter may also be a rheological parameter.

The at least one physical parameter is preferably one or more selected from the group consisting of tissue contraction, stiffness, force (development), spontaneous beating frequency, force (of contraction), contractile force, and one or more MEA measures; preferably measured as pole deflection, Young's modulus, (EHM) shortening, spontaneous beating frequency, force of contraction (FOC), twitch tension, and one or more from the group consisting of (Weighted) Mean Firing Rate, Burst Frequency (Hz), Interspike Interval in a burst (ms), Network Burst (NB) Frequency, Interspike Interval in a NB (ms), and NB Duration (s), respectively. Alternatively, or additionally, the at least one physical parameter is preferably one or more selected from the group consisting of pole deflection, Young's modulus, (EHM) shortening, spontaneous beating frequency, force of contraction (FOC), twitch tension, and one or more from the group consisting of (Weighted) Mean Firing Rate, Burst Frequency (Hz), Interspike Interval in a burst (ms), NB Frequency, Interspike Interval in a NB (ms), and/or NB Duration (s).

Alternatively, or additionally, the physical parameter may be one or more selected from those listed in **Table 1**.

**Table 1: List of physical parameters and their definitions.**

| Physical parameter | Symbol | Description | SI base unit | Dimension |
|---|---|---|---|---|
| Length | $L$ | The one-dimensional extent of an object | metre (m) | **L** |
| Mass | $M$ | A measure of resistance to acceleration | kilogram (kg) | **M** |
| Time | $T$ | The duration of an event | second (s) | **T** |
| Electric Current | $I$ | Rate of flow of *electrical* charge per unit time | ampere (A) | **I** |
| Temperature | $T$ | Average kinetic energy per degree of freedom of a system | kelvin (K) | $\Theta$ |
| Luminous intensity | $I_v$ | Wavelength-weighted power of emitted light per unit solid angle | candela (cd) | **J** |

The physical parameter may also be one or more selected from those listed in **Table 2**.

**Table 2: List of physical parameters, here derived physical quantities, and their definitions.**

| Physical parameter | Symbol | Description | SI derived unit | Dimension |
|---|---|---|---|---|
| Absement | A | Measure of sustained displacement: the first integral with respect to time of displacement | m·s | L T |
| Acceleration | $a\rightarrow$ | Rate of change of velocity per unit time: the second time derivative of position | m/s$^2$ | **L T**$^{-2}$ |
| Angular acceleration | $\omega_a$ | Change in angular velocity per unit time | rad/s$^2$ | **T**$^{-2}$ |
| Angular momentum | L | Measure of the extent and direction of an object rotates about a reference point | kg·m$^2$/s | **M L**$^2$ **T**$^{-1}$ |
| Angular velocity | $\omega$ | The angle incremented in a plane by a segment connecting an object and a reference point per unit time | rad/s | **T**$^{-1}$ |
| Area | A | Extent of a surface | m$^2$ | **L**$^2$ |
| Area density | $\rho_A$ | Mass per unit area | kg·m$^{-2}$ | **M L**$^{-2}$ |

(continued)

| Physical parameter | Symbol | Description | SI derived unit | Dimension |
|---|---|---|---|---|
| Burst frequency | | See Frequency | | |
| Capacitance | C | Stored charge per unit electric potential | farad (F = C/V) | $M^{-1} L^{-2} T^4 I^2$ |
| Current density | $J \rightarrow$ | Electric current per unit cross-section area | A/m² | $L^{-2} I$ |
| Dynamic viscosity | v | Measure for the resistance of an incompressible fluid to stress | Pa·s | $M L^{-1} T^{-1}$ |
| Electric charge | Q | The force per unit electric field strength | coulomb (C = A·s) | $T I$ |
| Electric charge density | $\rho_Q$ | Electric charge per unit volume | C/m³ | $L^{-3} T I$ |
| Electric dipole moment | p | Measure of the separation of equal and opposite electric charges | C·m | $L T I$ |
| Electric displacement field | $D \rightarrow$ | Strength of the electric displacement | C/m² | $L^{-2} T I$ |
| Electric field strength | $E \rightarrow$ | Strength of the electric field | V/m | $M L T^{-3} I^{-1}$ |
| Electrical conductance | G | Measure for how easily current flows through a material | siemens (S = $\Omega^{-1}$) | $M^{-1} L^{-2} T^3 I^2$ |
| Electrical conductivity | $\sigma$ | Measure of a material's ability to conduct an electric current | S/m | $M^{-1} L^{-3} T^3 I^2$ |
| Electric potential | $\varphi$ | Energy required to move a unit charge through an electric field from a reference point | volt (V = J/C) | $M L^2 T^{-3} I^{-1}$ |
| Electrical resistance | R | Electric potential per unit electric current | ohm ($\Omega$ = V/A) | $M L^2 T^{-3} I^{-2}$ |
| Electrical resistivity | $\rho_e$ | Bulk property equivalent of electrical resistance | ohm-metre ($\Omega$·m) | $M L^3 T^3 I^{-2}$ |
| Energy | E | Energy | J | $M L^2 T^{-2}$ |
| Energy density | $\rho_E$ | Energy per unit volume | j·m⁻³ | $M L^{-1} T^{-2}$ |
| Entropy | S | Logarithmic measure of the number of available states of a system | J/K | $M L^2 T^{-2} \Theta^{-1}$ |
| Firing rate | | See Frequency | | |
| Force | $F \rightarrow$ | Transfer of momentum per unit time | newton (N = kg·m·s⁻²) | $M L T^{-2}$ |
| Force of contraction (FOC) or twitch tension (TT) | F | Force required to move a tissue from a relaxed to a contracted state | newton (N = kg·m·s⁻²) | $M L T^{-2}$ |
| Frequency | f | Number of (periodic) occurrences per unit time | hertz (Hz = s⁻¹) | $T^{-1}$ |
| Half-life | $t_{1/2}$ | Time for a quantity to decay to half its initial value | s | $T$ |

(continued)

| Physical parameter | Symbol | Description | SI derived unit | Dimension |
|---|---|---|---|---|
| Heat | Q | Thermal energy | joule (J) | $\mathbf{M\ L^2\ T^{-2}}$ |
| Heat capacity | $C_p$ | Energy per unit temperature change | J/K | $\mathbf{M\ L^2\ T^{-2}\ \Theta^{-1}}$ |
| Heat flux density | $\phi_Q$ | Heat flow per unit time per unit surface area | W/m$^2$ | $\mathbf{M\ T^{-3}}$ |
| Illuminance | $E_v$ | Luminous flux per unit surface area | lux (lx = cd·sr/m$^2$) | $\mathbf{L^{-2}\ J}$ |
| Impedance | Z | Resistance to an alternating current of a given frequency, including effect on phase | ohm ($\Omega$) | $\mathbf{M\ L^2\ T^{-3}\ I^{-2}}$ |
| Impulse | J | Transferred momentum | newton-second (N.s = kg·m/s) | $\mathbf{M\ L\ T^{-1}}$ |
| Inductance | L | Magnetic flux generated per unit current through a circuit | henry (H) | $\mathbf{M\ L^2\ T^{-2}\ I^{-2}}$ |
| Interspike interval | ISI | Time between two electrical activites recorded as spikes | s | |
| Intensity | I | Power per unit cross sectional area | W/m$^2$ | $\mathbf{M\ T^{-3}}$ |
| Jerk | $j\to$ | Change of acceleration per unit time: the third time derivative of position | m/s$^3$ | $\mathbf{L\ T^{-3}}$ |
| Jounce (or *snap*) | $s\to$ | Change of jerk per unit time: the fourth time derivative of position | m/s$^4$ | $\mathbf{L\ T^{-4}}$ |
| Linear density | $\rho_l$ | Mass per unit length | kg·m$^{-1}$ | $\mathbf{M\ L^{-1}}$ |
| | | | | |
| Mach number (or *mach*) | M | Ratio of flow velocity to the local speed of sound | unitless | 1 |
| Magnetic field strength | H | Strength of a magnetic field | A/m | $\mathbf{L^{-1}\ I}$ |
| Magnetic flux | $\Phi$ | Measure of magnetism, taking account of the strength and the extent of a magnetic field | weber (Wb) | $\mathbf{M\ L^2\ T^{-2}\ I^{-1}}$ |
| Magnetic flux density | B | Measure for the strength of the magnetic field | tesla (T = Wb/m$^2$) | $\mathbf{M\ T^{-2}\ I^{-1}}$ |
| Magnetization | M | Amount of magnetic moment per unit volume | A/m | $\mathbf{L^{-1}\ I}$ |
| Mass fraction | x | Mass of a substance as a fraction of the total mass | kg/kg | 1 |
| (Mass)Density (or *volume density*) | $\rho$ | Mass per unit volume | kg/m$^3$ | $\mathbf{M\ L^{-3}}$ |
| Mean lifetime | T | Average time for a particle of a substance to decay | s | T |
| Molar concentration | C | Amount of substance per unit volume | mol·m$^{-3}$ | $\mathbf{L^{-3}\ N}$ |
| Molar energy | | Amount of energy present in a system per unit amount of substance | J/mol | $\mathbf{M\ L^2\ T^{-2}\ N^{-1}}$ |

(continued)

| Physical parameter | Symbol | Description | SI derived unit | Dimension |
|---|---|---|---|---|
| Molar entropy | | Entropy per unit amount of substance | J/(K·mol) | $M\,L^2\,T^{-2}\,\Theta^{-1}\,N^{-1}$ |
| Molar heat capacity | c | Heat capacity of a material per unit amount of substance | J/(K·mol) | $M\,L^2\,T^{-2}\,\Theta^{-1}\,N^{-1}$ |
| Moment of inertia | I | Inertia of an object with respect to angular acceleration | kg·m² | $M\,L^2$ |
| Momentum | $p\rightarrow$ | Product of an object's mass and velocity | kg·m/s | $M\,L\,T^{-1}$ |
| Network Burst (NB) Frequency | | See Frequency | | |
| NB duration | | See Time | | |
| Permeability | $\mu_s$ | Measure for how the magnetization of material is affected by the application of an external magnetic field | H/m | $M\,L\,T^{-2}\,I^{-2}$ |
| Permittivity | $\varepsilon_s$ | ' Measure for how the polarization of a material is affected by the application of an external electric field | F/m | $M^{-1}\,L^{-3}\,T^{4}\,I^{2}$ |
| Plane angle | θ | Ratio of circular arc length to radius | radian (rad) | 1 |
| Pole deflection | | Pixel distance (pole distance in a relaxed versus a contracted state) or % change of pole distance in a relaxed versus a contracted state; in the Examples herein calculated as % as follows: 100 x (pole distance$_{day\ 0}$-pole distance$_{day\ x}$)/pole distance$_{day\ 0}$; see also Length with day 0 representing a relaxed state and day x a contracted state | | |
| Power | P | Rate of transfer of energy per unit time | watt (W) | $M\,L^2\,T^{-3}$ |
| Pressure | p | Force per unit area | pascal (Pa = N/m²) | $M\,L^{-1}\,T^{-2}$ |
| Pop | $p\rightarrow$ | Rate of change of crackle per unit time: the sixth time derivative of position | m/s⁶ | $L\,T^{-6}$ |
| Radiance | L | Power of emitted electromagnetic radiation per unit solid angle per emitting source area | W/(m²·sr) | $M\,T^{-3}$ |
| Radiant intensity | I | Power of emitted electromagnetic radiation per unit solid angle | W/sr | $M\,L^2\,T^{-3}$ |
| Reaction rate | r | Rate of a chemical reaction for unit time | mol/(m³·s) | $L^{-3}\,T^{-1}\,N$ |
| Refractive index | n | Factor by which the phase velocity of light is reduced in a medium | unitless | 1 |
| Reluctance | | resistance to the flow of magnetic flux | H⁻¹ | $M^{-1}\,L^{-2}\,T^{2}\,I^{2}$ |
| Resting tension (RT) | F | Force developed at a resting state | newton (N = kg·m·s⁻²) | $M\,L\,T^{-2}$ |
| Solid angle | Ω | Ratio of area on a sphere to its radius squared | steradian (sr) | 1 |

(continued)

| Physical parameter | Symbol | Description | SI derived unit | Dimension |
|---|---|---|---|---|
| Specific energy | | Energy density per unit mass | $J \cdot kg^{-1}$ | $L^2\,T^{-2}$ |
| Specific heat capacity | c | Heat capacity per unit mass | $J/(K \cdot kg)$ | $L^2\,T^{-2}\,\Theta^{-1}$ |
| Specific volume | v | Volume per unit mass (reciprocal of density) | $m^3 \cdot kg^{-1}$ | $M^{-1}\,L^3$ |
| Spin | S | Quantum-mechanically defined angular momentum of a particle | $kg \cdot m^2 \cdot s^{-1}$ | $M\,L^2\,T^{-1}$ |
| Strain | $\varepsilon$ | Extension per unit length | unitless | 1 |
| Stress | $\sigma$ | Force per unit oriented surface area | Pa | $M\,L^{-1}\,T^{-2}$ |
| Surface tension | $\gamma$ | Energy change per unit change in surface area | N/m or $J/m^2$ | $M\,T^{-2}$ |
| Temperature gradient | $\{\displaystyle \nabla T\}$ | steepest rate of temperature change at a particular location | K/m | $\Theta\,L^{-1}$ |
| Thermal conductance | | Measure for the ease with which an object conducts heat | W/K | $M\,L^2\,T^{-3}\,\Theta^{-1}$ |
| Thermal conductivity | $\lambda$ | Measure for the ease with which a material conducts heat | $W/(m \cdot K)$ | $M\,L\,T^{-3}\,\Theta^{-1}$ |
| Thermal resistance | R | Measure for the ease with which an object resists conduction of heat | K/W | $M^{-1}\,L^{-2}\,T^3\,\Theta$ |
| Thermal resistivity | $R_\lambda$ | Measure for the ease with which a material resists conduction of heat | $K \cdot m/W$ | $M^{-1}\,L^{-1}\,T^3\,\Theta$ |
| Torque | $\tau$ | Product of a force and the perpendicular distance of the force from the point about which it is exerted | newton-metre (N·m) | $M\,L^2\,T^{-2}$ |
| Twitch tension | | See Force of contraction (FOC) | | |
| Velocity | $v\rightarrow$ | Moved distance per unit time: the first time derivative of position | m/s | $L\,T^{-1}$ |
| Volume | V | Three dimensional extent of an object | $m^3$ | $L^3$ |
| Volumetric flow rate | Q | Rate of change of volume with respect to time | $m^3 \cdot s^{-1}$ | $L^3\,T^{-1}$ |
| Wavelength | $\lambda$ | Perpendicular distance between repeating units of a wave | m | L |
| Wavenumber | k | Repetency or spatial frequency: the number of cycles per unit distance | $m^{-1}$ | $L^{-1}$ |
| Wavevector | $k\rightarrow$ | Repetency or spatial frequency vector: the number of cycles per unit distance | $m^{-1}$ | $L^{-1}$ |
| Weight | w | Gravitational force on an object | newton (N $= kg \cdot m/s^2$) | $M\,L\,T^{-2}$ |
| Work | W | Transferred energy | joule (J) | $M\,L^2\,T^{-2}$ |
| Young's modulus | E | Ratio of stress to strain | pascal (Pa $= N/m^2$) | $M\,L^{-1}\,T^{-2}$ |

(continued)

| Physical parameter | Symbol | Description | SI derived unit | Dimension |
|---|---|---|---|---|
| Elastic modulus | G' | Elastic component of the viscoelastic behavior of a material | pascal (Pa = N/m$^2$ | $M\,L^{-1}\,T^{-2}$ |
| Viscous modulus | G" | Viscous component of the viscoelastic behavior of a material | pascal (Pa = N/m$^2$ | $M\,L^{-1}\,T^{-2}$ |
| Complex modulus | G* | $G^* = T_A / \gamma_A$<br>with complex shear modulus G* (G star, in Pa), shear-stress amplitude $\tau_A$ (in Pa), and strain amplitude $\gamma_A$ (dimensionless, or expressed in %). G* describes the entire viscoelastic behavior of a sample and is called the complex shear modulus G* | pascal (Pa = N/m$^2$ | $M\,L^{-1}\,T^{-2}$ |
| Shear stress | $\tau$ | $\tau = F / A$ with shear stress $\tau$ (pronounced: tau), shear force F (in N) and shear area A (in m$^2$). | pascal | $M\,L^{-1}\,T^{-2}$ |
| Shear strain or shear deformation | $\gamma$ | $\gamma = s / h$ with shear strain $\gamma$, deflection path s (in m), and shear gap h (in m) | m/m | none |
| Shear modulus | G | $G = \tau / \gamma$ with shear modulus G, shear stress $\tau$ (in Pa), and shear strain or • shear deformation $\gamma$ (with the unit 1) | none | none |
| Shortening (e.g. EHM shortening) | | Calculated herein as % as follows: $100 \times$ (pole distance$_{complete\ relaxation}$ -pole distance$_{maximal\ contraction}$)/pole distance$_{complete\ relaxation}$; measured per contraction | | |
| Spontaneous beating frequency | | See Frequency | | |

Further shear moduli are shown in the following: G'=G*cos($\delta$) - this is the "storage" or "elastic" modulus; G"=G*sin($\delta$) - this is the "loss" or "plastic" modulus; and tan$\delta$=G"/G' - a measure of how elastic (tan$\delta$<1) or plastic (tan$\delta$>1). Those can also be employed and measured as physical parameters within the context of the present invention. A further exemplary physical parameter is the strain rate expressed as dF/dt max = maximal force increase over time of the contraction amplitude (measure of contraction) or dF/dt min = maximal force decrease over time of the contraction amplitude (measure of relaxation).

The method of the present invention may include a step of comparing the (value of the) physical parameter obtained in step (ii) of the method matches a certain predetermined threshold. Accordingly, the present invention relates to a method for assessing the potency of a pharmaceutical product assumed to be effective in treating a disorder, preferably a genetic disorder or a non-genetic disorder, wherein the disorder results in an impaired functionality of a physiological bodily function of a subject having the disorder, wherein the method comprises:

(i) incubating an engineered tissue with the pharmaceutical product, wherein the engineered tissue is or has been modified to be representative for the disorder;
(ii) determining at least one physical parameter of the engineered tissue resulting from step (i), wherein the physical parameter is a measure of the functionality of the physiological bodily function, and
(iii) determining whether a value of the at least one physical parameter determined in step (ii) meets a predetermined threshold of said physical parameter, which determines whether the pharmaceutical product has potency,

wherein, if said value of the at least one physical parameter exceeds the predetermined threshold, potency of the pharmaceutical product is indicated for treating the disorder by at least partially restoring the functionality of the physiological bodily function.

Depending on the nature of the disorder and the physical parameter, the pharmaceutical product may aim at increasing the physical parameter (e.g., force in muscular dystrophies) or at decreasing the physical parameter (e.g., heart rate in tachycardias). Such a distinction is well within the ability of a person skilled in the art. Thus, one could also say that a value of the at least one physical parameter that meets, fulfils and/or matches the predetermined threshold indicates that the pharmaceutical product has potency for treating the disorder. More specifically, a value of the at least one determined physical parameter that exceeds the predetermined threshold refers herein to a value of the at least one physical parameter that is at least equal to, preferably, dependent on the disorder, pharmaceutical product and/or physical parameter under study, (preferably significantly) higher or lower than said predetermined threshold, which indicates that the pharmaceutical product has potency for treating the disorder by at least partially restoring the functionality of the physiological bodily function. For example, in case of an engineered tissue being a bioengineered neuronal organoid (BENO), that is incubated with a competitive AMPA/Kainate receptor antagonist to the glutamatergic receptor (AMPA), like Cyanquixalin (CNQX), a diminishment of synaptic activity can be expected that may be exemplified by a decrease in the physical parameter "weighted mean firing rate" and/or an increase in the physical parameter "interspike interval in a burst" due to excitatory network silencing (see, e.g., also **Figure 6C** and **Experiment 9**). Thus, meeting the respective predetermined threshold, e.g., for weighted mean firing rate would relate to a comparison of the determined value of weighted mean firing rate and the value of the predetermined threshold for said physical parameter, wherein potency of said receptor antagonist would be indicated in case the determined value of the weighted mean firing rate would exceed the respective predetermined threshold and thus, in case the determined value of the weighted mean firing rate would be below the respective predetermined threshold. In case of the at least one physical parameter comprising or being, e.g., interspike interval in a burst, potency of said receptor antagonist would be indicated in case the determined value of the interspike interval in a burst would exceed the respective predetermined threshold and thus, in case the determined value of the interspike interval in a burst would be higher than the respective predetermined threshold.

Herein, the term "predetermined" refers to an a priori determination, preferably in the context of a threshold. As an example, such a predetermined threshold may relate to a value of the at least one physical parameter that is determined by the skilled artisan a priori, preferably based on a reference (experiment). Thus, a predetermined threshold may be determined in an a priori performed reference (example). Alternatively, or additionally, the predetermined threshold may relate to a value of the at least one physical parameter that is determined by the skilled artisan based on a reference (experiment) investigated in parallel and/or at least partially temporarily overlapping with a potency assay as described herein. Thus, said reference (experiment) and the potency assay can be performed at least partially temporarily overlapping and/or in parallel. As regards said reference (experiment) it is preferred that said reference (experiment) reflects a potency assay as disclosed herein in the sense that it preferably comprises step (i) and step (ii), wherein in step (i) no pharmaceutical product is used and/or a buffer and/or an empty vector is used instead of a pharmaceutical product. The value(s) of the at least one physical parameter obtained in the reference (experiment) is/are preferably investigated to determine a respective predetermined threshold that can be used in the method according to the present invention to assess the potency of the pharmaceutical product under study for treating a disorder. The described reference (experiment) has the advantage that the experimental setting and/or conditions between reference (experiment) and potency assay are comparable, preferably identical except incubation with a pharmaceutical product. Thus, the effect of the pharmaceutical product on the at least one physical parameter can be assessed without or only with limited bias that may result from differences in the experimental setting and/or condition. Such a predetermined threshold can thus be considered optimized in view of the characteristics of engineered tissue, disease, pharmaceutical product and/or physical parameter(s) used in the method according to the present invention for assessing the pharmaceutical product's potency. Illustrative and/or preferred examples of suitable combinations of engineered tissue, disease and physical parameter are given in the respective engineered tissue section further below.

Preferably, the predetermined threshold is determined based on a reference (experiment), wherein the at least one physical parameter of an engineered tissue and/or engineered organoid is determined in the absence of a pharmaceutical product or by comparing values of the at least one physical parameter obtained in the absence and presence of a pharmaceutical product, respectively. Said reference (experiment) may encompass one or more conditions and thus, refer to a reference (experiment) based on

i) an engineered tissue and/or engineered organoid that is derived from ia) a healthy individual and/or ib) well-characterized iPSC representing a healthy wildtype,
ii) an untreated engineered tissue and/or an untreated engineered organoid that iia) is or has been modified to be representative for the disorder or iib) is derived from a subject having the disorder, wherein said disorder is a, preferably genetic or non-genetic, disorder for the treatment of which the potency of the pharmaceutical product is assessed using the method according to the present invention, and/or
iii) an engineered tissue and/or engineered organoid with a permanent genetic repair of a disorder-associated, preferably disorder-causing, mutation.

Option i) may be referred to herein also as "healthy" reference. This option may be advantageous to investigate the effect of the pharmaceutical product in a healthy wildtype and thus, without disease specific confounding effects. Such an effect may be an increase or a decrease of a physical parameter, like force of contraction, upon treatment compared to said "healthy" reference. Alternatively, or optionally, option i) may be advantageous, e.g., in studies, wherein focus is put on assessing the level of restoration of the functionality of the physiological bodily function under study upon treatment with a given drug compared to a healthy individual. Option ii) may be referred to herein also as "diseased" reference. This option may be advantageous, e.g., for studies focusing on the level of restoration of the functionality of the physiological bodily function under study upon treatment with a given drug compared to an untreated patient suffering from a given disease. Alternatively, or additionally, said option may be advantageous to assess time course information about the potency of a pharmaceutical product such as time until when potency can be assessed upon treatment and/or effect length. Thus, such a "diseased" reference may be the identical or a different engineered tissue and/or organoid than the engineered tissue and/or organoid used for a potency assay as disclosed herein. In view of these considerations, option ii) may be preferred herein for a reference (experiment) to determine a predetermined threshold for a potency assay as described herein, in particular when the primary aim is to ensure alleviation of symptoms and/or improvement of a physiological bodily function upon treatment with a pharmaceutical product. Thus, pharmaceutical products can be identified that have the potency to shift a physical parameters (mean) value away from the (mean) value of said physical parameter observed in the "diseased" reference. Option iii) may be advantageous to determine the maximal dynamic range of a pharmaceutical product and/or substance that can be investigated as a reference (experiment). As regards options ia) and/or iia), investigation of treated and untreated engineered tissues modelling a disease may be advantageous to assess a drug's potency for a group of patients having in common a specific mutation underlying the disease. In such a scenario, the respective mutation(s) is(/are) preferably newly introduced into well-characterized iPSC cells. This offers the advantage that the effect of the drug on the disease caused by said specific mutation(s) can be assessed in a well-defined setting. Options ib) and/or iib) may be particularly advantageous, when a drug's potency is to be assessed in the context of an individualized therapy of said patient. In such a case, taking an engineered connective tissue (ECT) as an example, iPSC-derived stromal cells for ECT generation are preferably generated from somatic cells obtained from said patient. Thus, the effect of a specific drug treatment and more specifically, the potency of the drug can be assessed in a patient-specific genetic background, thus reflecting potential in vivo effects of the pharmaceutical product. Thus, the predetermined threshold is preferably determined based on and/or obtained by a reference (experiment), in which preferably the at least one physical parameter of an engineered tissue is at least determined in the absence of the pharmaceutical product. It is preferred that said engineered tissue used in the reference (experiment) is derived from a healthy individual and/or well-characterized iPSC representing a healthy wildtype. Alternatively, or additionally, said engineered tissue used in the reference (experiment) is preferably an untreated engineered tissue modified to be representative for the disorder or derived from a subject having said disorder, wherein said disorder is a, preferably genetic or non-genetic, disease for the treatment of which the potency of the pharmaceutical product is assessed using the herein disclosed potency assay. In any of the cases described herein, it is envisioned that a pharmaceutical product that is associated with a, preferably statistically significant, change in the (mean) value of at least one physical parameter compared to the respective (mean) parameter value in at least one reference (experiment and/or condition), preferably in at least the "diseased" reference (experiment and/or condition), is preferably assessed potent for treating the respective disorder. Preferably, in case of more than one reference (experiment), potency of a pharmaceutical product is indicated if a value of the at least one physical parameter exceeds the predetermined threshold for said parameter in at least one of the references (experiments and/or conditions), preferably in at least the "diseased" reference (experiment and/or condition).

As regards the value of the predetermined threshold, the person skilled in the art is capable of determining a predetermined threshold. An exemplary method would include the determination of the at least one physical parameter of an engineered tissue obtained from a healthy subject, i.e. a subject not suffering from the respective disease. Advantageously, in such a comparison engineered tissue does not contain a mutation that can be seen as causative for the disorder (isogenic reference). The value of the at least one physical parameter obtained from such a reference engineered tissue can be seen as a predetermined threshold. In some cases, a complete treatment, i.e. the restoration of the physiological bodily function to that of a healthy person, is not (yet) possible. In such a case, the predetermined threshold can be 10%, 15%, 20%, 25%, 30%, 35%, 40%, 45%, 50%, 55%, 60%, 65%, 70%, 75%. 80%, 85% or 90%, preferably at least 20%, 25%, 30%, 35% and/ 40%, of the value of the at least one physical parameter of a healthy subject.

Preferably, the predetermined threshold is determined based on and/or obtained by a reference (experiment), wherein said reference (experiment) is performed in at least one replicate and thus at least once, more preferably in at least three, four, five, fix, seven, eight, nine or ten replicates. This has the advantage that a statistical method can be performed and the potency of pharmaceutical product under study assessed statistically, while minimizing potential biasing effects. It is to be noted that Alternatively, or additionally, a potency assay as described herein is performed in at least one replicate and thus at least once, more preferably in at least three, four, five, fix, seven, eight, nine or ten replicates. Ideally, both the reference experiment(s) and the potency assay(s) are performed in at least one replicate and thus at least once, more preferably in at least three, four, five, fix, seven, eight, nine or ten replicates.

Preferably, the predetermined threshold is determined using a statistical method. For example, the statistical method may be a significance test such as a t-test, f-test, chi-square test, (non-parametric) Students t-test one-way ANOVA analysis (preferably in combination with a Dunnet's multiple comparison test) or two-way ANOVA analysis (preferably in combination with a Tukey's multiple comparison test). This is preferably the case, when the predetermined threshold is determined based on and/or obtained by a reference (experiment), in which the at least one physical parameter of an engineered tissue is determined both in the presence (treated) and the absence (untreated) of the pharmaceutical product, and respective physical parameter values are compared for determining said predetermined threshold. In such a scenario, the predetermined threshold can represent a minimal value of the at least one physical parameter that has been determined to differ significantly between values obtained from treated and untreated engineered tissues under comparable, preferable otherwise identical, experimental conditions. As another example, the statistical method may comprise determination of standard deviation(s) (SD). This is preferably the case, when the predetermined threshold is determined based on and/or obtained by a reference (experiment), in which the at least one physical parameter of an engineered tissue is determined in the absence of the pharmaceutical product, wherein the reference (experiment) comprises at least three, four, five, fix, seven, eight, nine or ten replicates. In such a scenario, the predetermined threshold preferably represents a value of the at least one physical parameter that differs, .e.g., by 1x SD, 1.5x SD, 2x SD, 2.5x SD or 3x SD from the respective (mean) physical parameter value observed in the replicates of the reference (experiment). Alternatively, or optionally, a predetermined threshold determined using a statistical method a may be expressed as a percentage change (% change) of a respective (mean) reference value of said physical parameter. In any of these scenarios, a value of the at least one physical parameter that exceeds the predetermined threshold, preferably significantly, deviates from a respective (mean) value obtained in the reference (experiment) with the direction of the deviation from the respective (mean) reference (experiment) value to be assessed by the skilled artisan in view of disease, engineered tissue and/or physical parameter under study.

Preferably, the predetermined threshold is based on and/or obtained by a reference (experiment) using a statistical method, preferably wherein the reference (experiment) is performed in at least one replicate and thus at least once, more preferably in at least three, four, five, fix, seven, eight, nine or ten replicates. It is particularly preferred that the predetermined threshold is a value deviating from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD. Alternatively, or additionally, the predetermined threshold is a value deviating from a (mean) reference value obtained for the at least one physical parameter in a reference (experiment) by (at least) between 0.5x and 2.5x standard deviations (SD) and/or by (at least) 0.5x SD, 1x SD (standard deviation), 1.5x SD or 2x SD that is expressed as % change of the respective (mean) reference value.

The skilled artisan is aware of the fact that for a pharmaceutical product, the potency of which is to be assessed using the method according to the present invention, may affect more than one physical parameter. In this case, the skilled person in the art is capable of identifying the most relevant physical parameter(s) to be determined for the potency assay. The skilled person in the art is further capable of interpreting obtained results in case only a portion, e.g. one, two, three, half etc., of the determined physical parameter values are values that fulfil or match the respective predetermined threshold. For example, there may be differences in effect sizes of a specific drug treatment on physical parameters depending on the target of the respective physical parameter. If, e.g., a pharmaceutical product is a general excitatory neuron inhibitor, investigation of its treatment effect may reveal a silencing of the excitatory network as its primary effect. This may be reflected, e.g., by the at least one physical parameter being and/or comprising (weighted) mean firing rate, burst frequency, and/or network burst (NB) frequency. Said treatment may have additionally secondary effects resulting from said primary effect and that may be reflected, e.g., by the at least one physical parameter being and/or comprising interspike interval in a burst. The skilled artisan is aware that such a secondary effect may exhibit for example a reduced effect size compared to a primary effect, which may lead to a physical parameter relating to primary effect detection that exceeds a respective threshold in contrast to a physical parameter relating to a secondary effect. A comparable scenario may be observed vice versa for a secondary effect exhibiting a larger effect size than a primary effect. Thus, depending on the disease, the pharmaceutical product, the engineered tissue and the at least one physical parameter determined for the potency assay, the skilled person may consider potency of a pharmaceutical product indicated in case one or more values of the at least one physical parameter exceeding the respective thresholds. Thus, in case of more than one physical parameter, a value of at least one of said more than one physical parameter is preferably required to exceed a respective threshold for indicating potency, preferably at least in view of a "diseased" reference (condition and/or experiment).

The skilled artisan is aware that potency of a pharmaceutical product assumed to be effective in treating a disorder may be assessed under constraints such as the concentration of the pharmaceutical product used during incubation and/or duration of incubation. Generally, a suitable concentration to be investigated using a potency assay as disclosed herein may be a clinically relevant concentration, wherein potency of a pharmaceutical product is assessed when at least a clinically relevant concentration of the pharmaceutical product is associated with at least a (mean) value of a physical parameter that exceeds the respective predetermined threshold obtained by at least one reference (experiment), preferably at least a "diseased" reference. For example, in case of small molecules, the EC50/IC50 may be used as an indication of a suitable concentration as a pharmaceutical product preferably shows an effect within its biophysical solubility range as well as a range from, e.g., 100-fold higher to 100-fold lower than the EC50/IC50. For non-classical pharmaceutical products, e.g. gene therapy products such as AAV mediated pharmaceutical products, a titer-based approach may be advantageous to determine clinically relevant dosages (concentrations) such as, e.g. $10^{12}$-$10^{14}$ vector genomes per kg (vg/kg). Thus, the person skilled in the art is aware that using the method according to the present invention for example a minimal amount, concentration, incubation time and/or treatment time of the pharmaceutical product can be determined that for which the pharmaceutical product can be assessed potent for treating a disorder. Consequently, the method according to the present invention can advantageously be used to determine suitable (e.g. minimal) potent drug concentrations for an intended treatment of a disease and/or suitable (e.g. minimal) treatment durations, wherein the drug is assessed potent for treating the disease by at least partially restoring the functionality of the physiological bodily function.

## Engineered tissues

To be suitable for a potency assay, the engineered tissue used in the method of the invention should be representative for the disorder, which shall be treated with the pharmaceutical product. "Representative for the disorder" as used herein relates to an engineered tissue, which carries a deviation from a healthy state. This deviation leads to an impaired functionality of the physiological bodily function, which can be measured by determining at least one physical parameter. As an illustrative example, mutations (the "deviation") cause the expression of non-functional dystrophin proteins in Duchenne muscular dystrophy (DMD), which results in the loss of muscular force (the "impaired functionality of a physiological bodily function"). Such a mutation can be seen as "being causative for the (genetic) disorder". This loss of muscular force can be determined by measuring the force exerted by a muscle of a patient (the "at least one physical parameter") and/or mimicked using an engineered tissue. In this illustrative example, a pharmaceutical product could be designed to restore the mutations in such a way that functional dystrophin is functional again, e.g. by gene therapy. Now turning to an exemplary potency assay: The engineered tissue is or has been modified to be representative for the disorder, e.g., DMD. While there are several ways to create an engineered (DMD) muscle tissue showing no or reduced ability to exert a force or any other function that can be measured as a physical quantity, those treatments do not reflect the actual or real cause of the disease. Such tissues would not be representative for the disorder because the impaired functionality of a physiological bodily function (here reduced or lost ability to exert a force) is not causatively linked to the deviation from the healthy state but merely an effect of a non-disease-related treatment. In a corresponding exemplary potency assay, the engineered tissue - in order to be representative for the disorder - should carry the mutation causing the disorder, e.g., DMD. This way, the pharmaceutical product, which aims at removing (treating) said deviation (e.g., by repairing the dysfunctional dystrophin gene) or at least bringing the subject into a state, wherein the initial deviation has no consequence anymore (e.g., by introducing a functional replacement of the dystrophin gene or correction of the genetic reading frame), can be assayed for its potency. Thus, there is interplay between the pharmaceutical product, its mode of action, the disorder and how the engineered tissue mimics the disorder. Exemplarily potency assays are schematically illustrated in **Figure 1.**

Engineered tissues as such are known to a person skilled in the art. Examples of engineered tissues suitable for the present invention include heart muscle (see e.g., WO 2015/040142), skeletal muscle (see e.g., WO 2021/0741236), connective tissue (see e.g., EP 3945133), neurons or neuronal organoids (see e.g., WO 2018/228948), or human myocardium (see e.g., WO 2015/025030), all of which are hereby incorporated by reference. Furthermore, methods of genetically engineering engineered tissues or the pluripotent stem cells prior to differentiation into the engineered tissue are well known to a person skilled in the art. An exemplary procedure is described in Long et al. (2018), Sci Adv, 4:eaap9004.

Accordingly, the engineered tissue may comprise one or more of heart tissue, heart muscle, muscle tissue, skeletal muscle tissue, neuronal tissue or connective tissue.

As outlined herein, the engineered tissue is or has been modified to be representative for the disorder. This can be achieved by various means. In one embodiment, the engineered tissue has been genetically edited to comprise a mutation being seen as causative for the (genetic) disease.

In addition to genetic disorders, the potency assay of the present invention can also be used for analyzing the potency of treatments of non-genetic disorders. Also in such non-genetic disorders gene therapy may be helpful. Accordingly, the engineered tissue can be subjected to a non-genetic intervention to be representative for the disorder. Such a non-genetic intervention may be a drug exposure, factor exposure (with the factor being e.g. a growth factor, a cytokine, etc.), a bodily fluid exposure (e.g. patient serum), a mechanical intervention (e.g. cutting injury), a biophysical intervention (e.g. thermal or radiation injury), and/or cell exposure (e.g., PBMCs isolated from patients).

The species of the cells of the engineered tissue is not particularly limited. Advantageously, it is however identical to the species of the subject, who will undergo a treatment with the pharmaceutical product. The engineered tissue may be human, from a non-human primate such as macaque or marmoset, from a pig, from a rodent such as mouse, rat or guinea pig. In a preferred embodiment, the engineered tissue is human. Non-human animal models are however still important as bridging study models. Thus, the engineered tissue may also be from a humanized animal model.

One further advantage of the present invention is that the engineered tissue can be universal, i.e. initially obtained from one particular donor used in various potency assays for different subjects, or individualized, i.e. directly obtained from the patient, who will undergo a treatment with the pharmaceutical product. Thus, there is great flexibility.

In one embodiment, the engineered tissue comprises cells obtained from a patient, who will undergo a treatment with the pharmaceutical product. This does not necessarily exclude the presence of material such as extracellular matrix, e.g., a collagen matrix, obtained from a subject other than said patient. In other words, all cells comprised in the engineered tissue may have been initially obtained from the patient, who will undergo a treatment with the pharmaceutical product. This approach is a further step towards individualized medicine. It allows the direct assessment of the potency of the pharmaceutical product within a particular patient. Thereby, other influence factors such as additional mutations that might have an influence on the (genetic) disorder can be tested. Furthermore, the potency of pharmaceutical products can be more thoroughly assayed in multifactorial genetic diseases such as asthma, autoimmune diseases such as multiple sclerosis, cancers, ciliopathies, diabetes (mellitus), heart disease, hypertension, inflammatory bowel disease, intellectual disability, mood disorder, obesity, refractive error or infertility.

As outlined before, the method of the present invention also allows the universal application of engineered tissues derived from cells of one subject in assaying the potency. Accordingly, the engineered tissue may comprise cells not obtained from the patient, who will undergo a treatment with the pharmaceutical product.

## Disorders

The method of the present invention is suitable for a variety of disorders. This includes genetic disorders but also non-genetic disorders. In one embodiment the disorder is a genetic disorder. In another embodiment, the disorder is a non-genetic disorder.

Herein, the terms "disorder" and "disease" are used herein interchangeably. Furthermore, a "genetic disorder" as used herein relates to a health problem caused by one or more abnormalities in the genome. It can be caused by a mutation in a single gene (monogenic) or multiple genes (polygenic) or by a chromosomal abnormality. The mutation responsible can occur spontaneously before embryonic development (a *de novo* mutation), or it can be inherited from two parents, who are carriers of a faulty gene (autosomal recessive inheritance) or from a parent with the disorder (autosomal dominant inheritance). When the genetic disorder is inherited from one or both parents, it is also classified as a hereditary disease. Some disorders are caused by a mutation on the X chromosome and have X-linked inheritance. Very few disorders are inherited on the Y chromosome or mitochondrial DNA (due to their size). Genetic disorders are present before birth. The opposite of a hereditary disease is an acquired disease, i.e. a "non-genetic disorder". Most cancers, although they involve genetic mutations to a small proportion of cells in the body, are acquired diseases. Some cancer syndromes, however, such as BRCA mutations, are hereditary genetic disorders.

In one embodiment, the disorder is a genetic heart condition, preferably a genetic heart condition selected from the group consisting of hereditary heart disease such as hereditary forms of dilated, hypertrophic, and arrhythmogenic cardiomyopathies or fibroblastopathies as well as inborn errors of metabolism-associated cardiomyopathies, more preferably a condition selected from the group consisting of Duchenne muscular dystrophy (DMD), Noonan syndrome, diastolic cardiomyopathy (preferably associated with a mutation in a myosin heavy chain (e.g., MHY7), a troponin (e.g., TNNT2, TNNI3, TNNC1), dystrophin (DMD), desmin (DES), phospholamban (PLB), a presenilin (e.g., PSEN1 and PSEN2), an actinin (e.g., ACTN2), a tropomyosin (e.g., TPM1), titin (TTN), an actin (e.g., ACTC1), and/or a laminin (e.g., LMNA)), hypertrophic cardiomyopathy (preferably associated with a mutation in a myosin heavy chain (e.g., MHY7), a troponin (e.g., TNNT2, TNNI3), a myosin binding protein (e.g. MYBPC3), a tropomyosin (e.g., TPM1), a myosin light chain (e.g., MYL2, MYL3), titin (TTN), an actin (e.g., ACTC1), and/or an AMP-activated protein kinase (e.g., PRKAG2)), arrhythmogenic right ventricular cardiomyopathy (preferably associated with to a mutation in desmoplakin (DSP), a plakophilin (e.g., PKP2), plakoglobin (JUP), a desmoglein (e.g., DSG2), a desmocollin (e.g., DSC2), a ryanodine receptor (e.g., RYR2), and/or laminin (e.g., LMNA)), long and short QT syndrome (preferably associated with a mutation in a sodium channel (e.g., SCN5A, SCN4B, SNTA1), a potassium channel (e.g., KCNH2, KCNQ1, KCNJ2, KCNJ5, KCNE1, KCNE2) and/or a calcium channel (e.g., CACNA1A, CACNA1C , CACNA2D1), a bicarbonate / chloride exchanger (e.g., SLC4A3), a caveolin (e.g., CAV3) and/or a calmodulin (e.g., CALM1, CALM2, CALM3)), Takotsubo syndrome, a lysosomal storage disorder, titinopathy and Barth syndrome (preferably associated with a mutation in tafazzin (TAZ)). Alternatively, or additionally, the disorder is associated with and/or caused by a mutation in a gene and/or protein selected from the group consisting of a myosin heavy chain (e.g., MHY7), a troponin (e.g., TNNT2, TNNI3, TNNC1), dystrophin (DMD), desmin (DES), phospholamban (PLB), a presenilin (e.g., PSEN1 and PSEN2), an actinin (e.g., ACTN2), a tropomyosin (e.g., TPM1), titin (TTN), an actin (e.g., ACTC1), a laminin (e.g., LMNA), a myosin binding protein (e.g. MYBPC3), a myosin light chain (e.g., MYL2, MYL3), an AMP-activated protein kinase (e.g., PRKAG2), desmoplakin (DSP), a plakophilin (e.g., PKP2), plakoglobin (JUP), a desmoglein (e.g., DSG2), a desmocollin (e.g., DSC2), a ryanodine receptor (e.g., RYR2), a sodium channel (e.g., SCN5A, SCN4B, SNTA1), a potassium channel (e.g., KCNH2, KCNQ1, KCNJ2, KCNJ5, KCNE1, KCNE2), a calcium channel (e.g., CACNA1A, CACNA1C , CACNA2D1), a bicarbonate / chloride exchanger (e.g., SLC4A3), a caveolin (e.g., CAV3), a calmodulin (e.g., CALM1, CALM2, CALM3), and tafazzin (TAZ).

In a further embodiment, the disorder is a non-genetic heart condition, preferably a non-genetic heart condition induced by simulated neurohormonal and/or pharmacological stimulation (e.g. by a catecholamine, angiotensin, and/or transforming growth factor (TGF) β), by a drug such as doxorubicin, a tyrosine kinase inhibitor and/or a cardiotoxic drug, by mechanical injury such as crush injury, by thermal injury such as freezing or overheating, by a biophysical injury such as radiation injury, by infection such as a virus infection with cardiotropic virions such as coxsackie, SarsCov, cytomegaly virus and Dengue virus, or a parasitic infection such as Chagas disease with Trypanosoma cruzi, optionally by a bodily fluid such as a serum from a patient with a heart disease, and/or optionally by cells such as blood derived mononuclear cell cytes (e.g., T-cells, B-cells, NK-cells, macrophages) from patients with heart disease.. Examples of a cardiotoxic drug are well known tho the person skilled in the art, see, e.g. Mladenka et al. 2018 (Med Res Rev 2018;38:1332-1403, PMID: 29315692, DOI: 10.1002/med.21476).

In one embodiment, the disorder is a neuronal condition, preferably selected from the group consisting of neurodegenerative diseases (e.g., dementia, Parkinson disease, M. Huntington), neuroinflammatory diseases (e.g., multiple sclerosis), brain inflammation disorders (e.g., meningitis), channelopathy (e.g., epilepsy) and psychiatric diseases (including autism and schizophrenia).

In one embodiment, the disorder is a genetic muscular disorder, preferably a genetic muscular disorder selected from the group consisting of Duchenne muscular dystrophy, Facioscapulohumeral dystrophy, Becker dystrophy, Emery-Dreifuss dystrophy, myotonic dystrophy, limb-girdle dystrophy, oculopharyngeal muscular dystrophy, congenital dystrophies, congenital myopathies, myotonia congenita, familial periodic paralysis, and a heritable connective tissue disease such as Marfan syndrome, Ehlers-Danlos syndrome or Loeys-Dietz syndrome. Alternatively, or additionally, the disorder may be a non-genetic muscular disorder.

In one embodiment, the disorder is (i) a heart disease which presents with cardiac fibrosis, such as a heart failure (of unknown and/or genetic cause) with reduced or preserved ejection fraction, myocardial infarction, a congenital heart disease, a cardiomyopathy with known mutation(s), age- and/or senescence-induced cardiac fibrosis, a drug-induced cardiac disorder, a metabolic cardiac disorder, a (monogenetic or polygenetic) disorder with contribution of the heart and cardiac fibrosis, a disease involving reactive or replacement fibrosis in an organ such as kidney, liver, lung, and/or skin, etc., (ii) a disorder with impaired scaring and wound healing such as Diabetes mellitus, and/or (iii) a heritable connective tissue disease such as Marfan syndrome, Ehlers-Danlos syndrome or Loeys-Dietz syndrome .

## Heart tissue

The method of the invention can be used to assay the potency of the pharmaceutical product assumed to be effective in treating a disorder involving the heart muscle or heart muscle tissue. Accordingly, the engineered tissue may be heart muscle or heart muscle tissue.

The physiological bodily function of heart muscle (tissue) is to exert a force in order to contract the heart chambers, thereby pumping blood through the blood vessels of the circulatory system. To do so, the cardiomyocytes present in heart muscle contract in a coordinated, synchronized manner. Accordingly, the method of the invention is particularly useful when the at least one physical parameter determined in the method is force, e.g., expressed in Newton (N), or change of volume. Accordingly, in one embodiment, the at least one physical parameter is force. In another embodiment, the at least one physical parameter is change of volume, e.g., of a chamber formed by the engineered tissue. In another embodiment, the at least one physical parameter is velocity of the muscle contraction-relaxation cycle.

The person skilled in the art is capable of preparing engineered heart (muscle) tissue. Methods for producing engineered heart muscle are, e.g., disclosed in WO 2015/025030, WO 2015/040142 or Long et al. (2018), Sci Adv, 4:eaap9004. Cardiomyocytes can be obtained from pluripotent stem cells by making use of the following exemplary method: iPSCs can be adapted and maintained in TESR-E8 (STEMCELL Technologies) on 1:120 Matrigel in PBS-coated plates and passaged using EDTA solution (Versene, Thermo Fisher Scientific) twice weekly. For cardiac differentiation, iPSCs can be plated at $5 \times 10^4$ to $1 \times 10^5$ cells/cm$^2$ and induced with RPMI, 2% B27, 200 mM L-ascorbic acid-2-phosphate sesquimagnesium salt hydrate (Asc; Sigma-Aldrich), activin A(9 ng/ml; R&D Systems), BMP4 (5 ng/ml; R&D Systems), 1 mM CHIR99021 (Stemgent), and FGF-2 (5 ng/ml; Miltenyi Biotec) for 3 days; following another wash with RPMI medium, cells may be cultured from days 4 to 13 with 5 mM IWP4 (Stemgent) in RPMI supplemented with 2% B27 and 200 mM Ascorbic acid. Cardiomyocytes may then be metabolically purified by glucose deprivation from days 13 to 17 in glucose-free RPMI (Thermo Fisher Scientific) with 2.2 mM sodium lactate (Sigma-Aldrich), 100 mM β-mercaptoethanol (Sigma-Aldrich), penicillin (100 U/ml), and streptomycin (100 mg/ml). To generate defined, serum-free engineered heart muscle (EHM), the purified cardiomyocytes can be mixed with HFFs (American Type Culture Collection) at a 70%:30% ratio. The cell mixture can be reconstituted in a mixture of pH-neutralized medical-grade bovine collagen (0.4 mg per EHM; LLC Collagen Solutions) and concentrated serum-free medium [$2\times$ RPMI, 8% B27 without insulin, penicillin (200 U/ml), and streptomycin (200 mg/ml)] and cultured for 3 days in Iscove medium with 4% B27 without insulin, 1% nonessential amino acids, 2 mM glutamine, 300 mM ascorbic acid, IGF1 (100 ng/ml; AF-100-11), FGF-2 (10 ng/ml; AF-100-18B), VEGF165 (5 ng/ml; AF-100-20), TGF-b1 (5 ng/ml; AF-100-21C; all growth factors can be obtained from PeproTech), penicillin (100 U/ml), and streptomycin (100 mg/ml). After a 3-day condensation period, EHM may then be transferred to flexible holders to support auxotonic contractions. The manufacturers listed are only to show that all components are commercially available and should not be construed as limiting.

Engineered (heart) muscle tissue typically exerts a force of 0.01 mN or more when it is healthy, e. g., in an assay as described in WO 2015/025030, WO 2015/040142, Tiburcy et al. (2017), Circulation, 135(19): 1832-1847, or Long et al. (2018), Sci Adv, 4:eaap9004. Accordingly, the one or more conditions preferably comprise that the engineered tissue exerts a force of 0.01 mN or more, 0.05 mN or more, 0.1 mN or more, or 1 mN or more. However, more important is the recovery of muscle function relative to a healthy reference. Force exerted by an engineered (heart) muscle tissue can be measured making use of the following exemplary experiment: Contraction experiments can be performed under isometric conditions, e.g., in organ baths at 37°C in gassed (5% CO2/95% O$_2$) Tyrode's solution (containing 120 mM NaCl, 1 mM MgCl$_2$, 0.2 mM CaCl$_2$, 5.4 mM KCl, 22.6 mM NaHCO$_3$, 4.2 mM NaH$_2$PO$_4$, 5.6 mM glucose, and 0.56 mM ascorbate). The engineered tissue can be electrically stimulated at 1.5 Hz with 5 ms square pulses of 200 mA. The engineered tissue can be mechanically stretched at intervals of 125 mm until the maximum systolic force amplitude (FOC) can be observed according to the Frank-Starling law. Responses to increasing extracellular calcium (e.g., 0.2 to 6 mM) can be investigated to determine maximal inotropic capacity. Forces can be normalized to muscle content (sarcomeric $\alpha$-actinin-positive cell content, as determined by flow cytometry).

The physiological bodily function of (heart) muscle tissue may be impaired by a variety of disorders - may it be hereditary (genetic) or non-genetic. In one embodiment, the disorder is a genetic heart condition, preferably a genetic heart condition selected from the group consisting of hereditary heart disease such as hereditary forms of dilated, hypertrophic, and arrhythmogenic cardiomyopathies or fibroblastopathies as well as inborn errors of metabolism-associated cardiomyopathies, more preferably a condition selected from the group consisting of Duchenne muscular dystrophy (DMD), Noonan syndrome, diastolic cardiomyopathy, hypertrophic cardiomyopathy, arrhythmogenic right ventricular cardiomyopathy, long and short QT syndrome, Takotsubo syndrome, a lysosomal storage disorder, titinopathy and Barth syndrome. Preferably, the condition is Duchenne muscular dystrophy. In another embodiment, the disorder is a non-genetic heart condition, preferably a non-genetic heart condition induced by simulated neurohormonal and/or pharmacological stimulation (e.g. by a catecholamine, an angiotensin, and/or transforming growth factor $\beta$), by a drug such as doxorubicin, a tyrosine kinase inhibitor and/or a cardiotoxic drug, by mechanical injury such as crush injury, by thermal injury such as freezing or overheating, by a biophysical injury such as radiation injury, by infection such as a virus infection with cardiotropic virions such as coxsackie, SarsCov, cytomegaly virus and Dengue virus, or a parasitic infection such as Chagas disease with Trypanosoma cruzi, optionally by a bodily fluid such as a serum from a patient with a heart disease, and/or optionally by cells such as blood derived mononuclear cell cytes (e.g., T-cells, B-cells, NK-cells, macrophages) from patients with heart disease..

Preferably, the at least one physical parameter is selected from the group consisting of (EHM) shortening, spontaneous beating frequency, contraction time/velocity, relaxation time/velocity, RT, FOC and TT.

Preferably, the at least one physical parameter is selected from the group consisting of (EHM) shortening, spontaneous beating frequency and FOC. It is preferred that the at least one physical parameter is determined approximately on day 14 of incubation of the engineered tissue, wherein the engineered tissue is preferably an EHM. It is further preferred that the predetermined threshold is determined based on and/or obtained by a reference (experiment), in which preferably the at least one physical parameter of an engineered tissue is at least determined in the absence of the pharmaceutical product. It is preferred that, the predetermined threshold is based on and/or obtained by a reference (experiment) using a statistical method and deviates from a (mean) reference value obtained for the at least one physical parameter in a reference (experiment) by 1x SD (standard deviation), preferably 2x SD. Alternatively, or additionally, said deviation is expressed as % change of the respective (mean) reference value.

Preferably, the at least one physical parameter comprises or is (EHM) shortening and the predetermined threshold deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1x SD or 2x SD. Alternatively, or additionally, said predetermined threshold is an at least 15%, preferably at least 20%, more preferably at least 25%, at least 35% or at least 40% change of a (mean) reference value obtained for the at least one physical parameter in a reference (experiment), in which (EHM) shortening is preferably determined in the absence of the pharmaceutical product using an engineered tissue that is derived from a healthy individual or a subject having the disorder and/or well-characterized iPSC representing a healthy wildtype or modified to be representative for the disorder. Thus, a pharmaceutical product is preferably assessed and/or determined to be potent for treating the disorder by at least partially restoring the functionality of the physiological bodily function if a value of the at least one physical parameter determined in a potency assay as disclosed herein exceeds the predetermined threshold of preferably 2x SD and/or (approximately) 35% or 40% change of the respective (mean) reference value.

Preferably, the at least one physical parameter comprises or is spontaneous beating frequency (in Hz) and the predetermined threshold deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1x SD or 2x SD. Alternatively, or additionally, said predetermined threshold is an at least 10%, preferably at least 15%, more preferably at least 20%, at least 25% or at least 30% change of a (mean) reference value obtained for the at least one physical parameter in a reference (experiment), in which spontaneous beating frequency is preferably determined in the absence of the pharmaceutical product using an engineered tissue that is derived from a healthy individual or a subject having the disorder and/or well-characterized iPSC representing a healthy wildtype or modified to be representative for the disorder. Thus, a pharmaceutical product is preferably assessed and/or determined to be potent for treating the disorder by at least partially restoring the functionality of the physiological bodily function if a value of the at least one physical parameter determined in a potency assay as disclosed herein exceeds the predetermined threshold of preferably 2x SD and/or (approximately) 20%, 25% or 30% change of the respective (mean) reference value.

Preferably, the at least one physical parameter comprises or is FOC and the predetermined threshold deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1x SD or 2x SD. Alternatively, or additionally, said deviation is expressed as % change of the respective (mean) reference value obtained for the at least one physical parameter in a reference (experiment), in which FOC is preferably determined in the absence of the pharmaceutical product using an engineered tissue that is derived from a healthy individual or a subject having the disorder and/or well-characterized iPSC representing a healthy wildtype or modified to be representative for the disorder.

### Neuronal tissue

The method of the invention can be used to assay the potency of the pharmaceutical product assumed to be effective in treating a disorder involving the nervous system. Accordingly, the engineered tissue may be neuronal tissue such as a neuronal organoid, or a neuron. The neuronal organoid may be a bioengineered neuronal organoid (BENO). "Nervous tissue" and "neuronal tissue" may be used interchangeably. Nervous tissue, also called neural or neuronal tissue, is the main tissue component of the nervous system. The nervous system regulates and controls bodily functions and activity. It consists of two parts: the central nervous system (CNS) comprising the brain and spinal cord, and the peripheral nervous system (PNS) comprising the branching peripheral nerves. It typically is composed of neurons, also known as nerve cells, which receive and transmit impulses, and neuroglia, also known as glial cells or glia or further specified as macroglia (e.g., astrocytes, oligodendrocytes, ependymal cells, radial cells, Schwann cells, satellite glial cells, enteric glial cells) and microglia (i.e., specialized macrophages of the central nervous system), which assist the propagation of the nerve impulse, provide nutrients to the neurons, and/or exhibit a phagocytosing and immune modulatory function.. Nervous tissue can be made up of different types of neurons, all of which have an axon. An axon is the long stem-like part of the cell that sends action potentials to the next cell. Bundles of axons make up the nerves in the PNS and tracts in the CNS. Physiological bodily functions of the nervous system may include sensory input, integration, control of muscles and glands, homeostasis, and mental activity.

Numerous protocols for inducing neural differentiation of human stem cells exist in prior art. One such method involves an induction of the neuroectoderm by dual SMAD (Sma and Mad Related Family) signaling pathway inhibition (i.e., by BMP and TGF beta inhibition) for about 8-12 days in culture (Chambers, Fasano et al., Nat. Biotechnol., 2009). At this time point, the majority of the stem cells transform into neural progenitor cells (NPCs). After day 12, several protocols (Lancaster and Knoblich, Science, 2014) allow spontaneous differentiation of the cells to various neuronal and glial cells, while other protocols (Qian, Nguyen et al., Cell, 2016; Birey, Andersen et al., Nature, 2017) apply various patterning factors to pattern the tissues, or neurotrophic factors (BDNF, GDNF) to enhance neuronal survival. Moreover, it has been observed that dbcAMP addition or notch inhibition by DAPT can enhance neuronal differentiation of such pluripotent cells (Crawford and Roelink, Dev. Dyn., 2007; Kriks, Shim et al, Nature, 2011). Differences in the factors used for neural induction and differentiation as well as the timing of the treatments define the differentiation potential afforded by these procedures. In another report, human cerebral organoids were produced as a model for microcephaly, a disease that is not easily reproducible using mouse models (Lancaster et al., Nature, 2013, Qian, Nguyen et al., Cell, 2016).

Protocols for generating neuronal organoids are also well known to a person skilled in the art, see e.g., WO 2018/228948 or Zafeiriou et al. (2020), Nature Communications, 11:3791, both of which are hereby incorporated by reference. An exemplary method for preparing BENOs is described in the following: The GMP hiPS line (TC1133, Lonza) or cell line hiPS-G1 can be used for BENO generation. One day prior to neuronal differentiation, a 1:1 mixture of acid-solubilized bovine collagen I (Collagen Solutions) and serum free 2× DMEM (Thermoscientific) can be prepared and neutralized by 0.1M NaOH addition. iPSC suspensions (4500 cells/μl) may be prepared in StemMACS™ iPS-Brew XF medium (Miltenyi) complemented with 20 ng/ml of FGF-2 (Miltenyi) and 10 μmol/L Y-27632 (Stemgent). iPSCs were added to the collagen/DMEM mixture to achieve a final concentration of 3,000 cells/μl and 1 mg/ml of collagen !. Thirty microliters of cell-collagen mixtures may be aliquoted into 96-well plates (U-bottom, low attachment) and placed in an incubator for 30 min. Upon collagen polymerization, 250 μl of StemMACS™ iPS-Brew XF supplemented with 10 ng/ml FGF and 10 μmol/L Y-27632 may be added in each well. From day 0 to 10 BENOs may be cultured in neuronal commitment medium (NCM, Basal medium [Neurobasal-A containing 2 mmol/L glutamine, 100 U/ml penicillin, 100 μg/ml streptomycin, 2% B27, 1% N2 supplement, 200 μmol/L ascorbic acid] complemented with 10 μmol/L SB431542 [Tocris], 50 ng/ml noggin [R&D systems] and 1 μmol/L RA [Sigma]). On day 3, BENOs may be transferred into six-well plates (10 BENOs/well). From day 10 to 15 BENOs may be cultured in neural progenitor expansion medium (NPEM, Basal medium complemented with 10 ng/ml FGF-2 and 5 ng/ml TGFB1 [Peprotech]). Finally, from day 15 to 28 BENOs may be cultured in Neural Differentiation Medium (Basal medium complemented with 2.5 μmol/L DAPT [Tocris] and 5 ng/ml TGFB1). From day 29, BENOs may be cultured in Basal medium. Medium change may be performed every second day. The manufacturers listed are only to show that all components are commercially available and should not be construed as limiting.

As used herein, the term "organoid" refers to a tissue culture forming a three-dimensional assembly, which mimics, at least partially, the structure and/or function of an organ, such as a human organ. Organoids can be generated from pluripotent stem cells in, for example, a three-dimensional (3D) environment. One such 3D environment for organoids is a spheroid-shaped 3D environment. An organoid may further be regarded as a miniaturized and simplified version of an organ.

As used herein, the term "bioengineered neuronal organoid" (BENO) is an organoid derived from neural tissue that has been produced according to the methods of the present invention. A BENO may be regarded as a miniaturized and simplified model of a neural organ, including the brain, and/or of neural tissue existing within an organ or controlling an organ, for instance, neural tissue present within the heart (e.g., sympathetic nervous system) and skeletal muscle (e.g., nicotinergic nerve endings at skeletal neuromuscular junctions). Herein, a BENO may preferably be regarded as a miniaturized and simplified model of a neural organ, including the brain. BENO are especially well suited as forebrain models. As BENO self-pattern into midbrain and hindbrain identities, BENO are also suitable as midbrain and/or hindbrain models.

Neuronal tissue or nervous tissue is known to transport information via electrical currents or chemical compounds. Accordingly, the at least one physical parameter preferably is electrical current and/or electrical activity. In one embodiment, the at least one physical parameter is electrical current, which can be expressed in Ampere (A). In another embodiment, the at least one physical parameter is electrical activity. It is also possible to monitor the changes in electrical potential (action potentials) caused by neuronal activity. Accordingly, the at least one physical parameter may be electrical potential, which can be expressed in Volt (V).

A "neuronal network" as used herein represents a group of one or more interconnected neurons. The connection between the neurons in a neuronal network permits a transmission of information from one neuron to the other(s). In a neuronal network, the connection between neurons can be via synapses. The presence of a neuronal network can be readily confirmed, for example, through the use calcium imaging or multielectrode array analyses of the neurons. In case of a disorder, the neuronal network can be impaired or absent.

A "functional neuronal network" as used herein refers to a neuronal network, which displays a transmission of electrochemical information from one neuron to the other. Functional neuronal networks require the formation of functional synapses. Functional neuronal networks are characterized by activity patterns of the network which may include synchronized electrical activity of more than one neuron of the network or patterns showing functional interdependencies between neurons of the network, including neuron activation or inhibition patterns. The presence of a neuronal network can be confirmed, for example, by calcium imaging of the neurons. In particular, the reaction of a group of neurons to signaling molecules or inhibition of receptors of signaling molecules (e.g., GABA receptors) can confirm the presence of a functional neuronal network. One example evidencing a functional neuronal network is where the neurons in the network demonstrate synchronized calcium signals (e.g., calcium spikes), which become un-synchronized following the addition of a receptor inhibitor that inhibits a neuronal signaling molecule and subsequently re-synchronize upon inhibitor removal. Accordingly, the functionality of the physiological bodily function may be characterized by the presence of a functional neuronal network.

A functional neuronal network can also be characterized by the presence of local and coordinated electrical bursts and/or clusters. Accordingly, the one or more conditions preferably comprise local and coordinated electrical bursts, and/or clusters. This can be assessed, e.g., by multri-electrode arrays, e.g., as described in Zafeiriou et al. (2020), Nature Communications, 11:3791. An exemplary method is described in the following: Six-well plates with 64 platinum microelectrodes arrays per well (MEA; 0.04 MΩ/microelectrode, 30 μm microelectrode diameter, 200 μm spacing) can

be coated with Matrigel™ 1:120 diluted in PBS for 1 h at room temperature prior seeding. One or two slices (thickness: 300 μm) may be placed into a MEA well and immobilized by a concentrically coiled tungsten ring. 10-15 min recordings/2 h may be performed every other day for up to 60 days using a Maestro pro MEA system (Axion Biosystems). Data recordings may be automatically scheduled with the Axion Software (AxIS Navigator) using the manufacturer's Spontaneous Neural Configuration. Data analysis may be performed using the manufacturer's standalone tools, Neural Metric Tool, and Axis Metric Plotting Tool (Axion Biosystems). The spike detecting threshold may be set to 5.5 standard deviations, and the electrodes that detected at least 5 spikes per min can be classified as active. Spike bursts may be identified using an ISI threshold requiring a minimum number of five spikes with a maximum ISI of 100 ms. NB may be identified by an envelope algorithm with a threshold of 1.25, minimum IBI interval of 100 ms, 75% burst inclusion with a minimum of 10% active electrodes. Firing synchrony may be estimated by the area under the normalized synchrony cross-correlogram for a time window of 20 ms. For potentiation measurements, multichannel fEPSP recordings may be performed with a MEA2100 device (MC_Rack 3.2.1.0 software, Multi Channel Systems, Reutlingen, FRG). To reduce noise, the bath may be grounded via an extra custom-made Ag/AgCl electrode attached to the MEA amplifier ground socket. LTP may be induced by 3 trains of HFS (20 pulses 100 Hz, 50 μs, 100 μA) in 20 s interval. Prior to HFS, neurons may be stimulated by single pulses (100 μA, 50 μs pulse width, 30 s inter-pulse interval). After stimulation the same protocol may be performed for 1 h in order to quantify differences in synaptic strength. The manufacturers listed are only to show that all components are commercially available and should not be construed as limiting.

"Neuronal network function" as used herein is the transmission of electrochemical information from one neuron to the other. Furthermore, herein a "network burst" relates to the synchronized firing of multiple neurons in different regions of a BENO and represent a measure of network bursting. Network bursting or oscillation is defined herein by alternating periods of high and low synchronized activity, and represents a hallmark of functional networks.

.Preferably, the at least one physical parameter is a MEA measure, preferably one or more selected from the group consisting of (Weighted) Mean Firing Rate, Burst Frequency (Hz), Interspike Interval in a burst (ms), Network Burst (NB) Frequency, Interspike Interval in a NB (ms), and NB Duration (s). It is preferred that the at least one physical parameter is determined approximately after 10 minutes of incubation of the engineered tissue, wherein the engineered tissue is preferably a BENO. It is further preferred that the predetermined threshold is determined based on and/or obtained by a reference (experiment), in which preferably the at least one physical parameter of an engineered tissue is at least determined in the absence of the pharmaceutical product. It is preferred that, the predetermined threshold is based on and/or obtained by a reference (experiment) using a statistical method and deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1x SD or 2x SD. Alternatively, or additionally, said deviation is expressed as % change of the respective (mean) reference value. Alternatively, or additionally, the at least one physical parameter may be plasticity.

For assessing a pro-convulsive potency of a pharmaceutical product, it is preferred that the at least one physical parameter is one or more selected from the group consisting of (Weighted) Mean Firing Rate, Burst Frequency (Hz), Network Burst (NB), and NB Duration (s), and that the predetermined threshold deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1x SD or 2x SD. Alternatively, or additionally, said predetermined threshold is an at least 5% or 10%, preferably at least 25% or 30%, more preferably at least 50% or 55%, at least 60% or 100% change of a (mean) reference value obtained for the at least one physical parameter in a reference (experiment), in which the at least one physical parameter is preferably determined in the absence of the pharmaceutical product using an engineered tissue that is derived from a healthy individual or a subject having the disorder and/or well-characterized iPSC representing a healthy wildtype or modified to be representative for the disorder. Thus, a pharmaceutical product is preferably assessed and/or determined to be, preferably pro-convulsive, potent for treating the disorder by at least partially restoring the functionality of the physiological bodily function if a value of the at least one physical parameter determined in a potency assay as disclosed herein exceeds the predetermined threshold of preferably 2x SD and/or (approximately) 10%, 50%, 55%, 60% or 100% change of said (mean) reference value. More specifically, it is preferred that in case of the at least one physical parameter being (Weighted) Mean Firing Rate said predetermined threshold is preferably 2x SD and/or (approximately) 55% or 60% change of said (mean) reference value; in case of the at least one physical parameter being Burst Frequency (Hz) said predetermined threshold is preferably 2x SD and/or (approximately) 50% change of said (mean) reference value; in case of the at least one physical parameter being Network Burst (NB) said predetermined threshold is preferably 2x SD and/or (approximately) 100% change of said (mean) reference value; in case of the at least one physical parameter being NB Duration (s) said predetermined threshold is preferably 2x SD and/or (approximately) 10% change of the respective (mean) reference value.

A pro-convulsive drug offers the opportunity to assess, e.g., potential drug effect ranges and/or to determine potential effects in a contra-indication study. Such a contra-indication study may, e.g., be of performed by administering or when a pharmaceutical product is to be administered to a patient without neurological disorder. On the other side, a potency assay as disclosed herein may be particularly advantageous to assess an anti-convulsive potency of a pharmaceutical product. Examples of (novel) anti-convulsive pharmaceutical drugs comprise Carbamazepine, Eslicarbazepine acetate, Valproate, Levetiracetam, Lamotrigine, Lacosamide, Perampanel, Topiramate, Pregabalin, and/or Cenobamate. When assessing an anti-convulsive potency of a pharmaceutical product, a reference (experiment) may be especially suitable that comprises a healthy BENO reference and/or a permanently genetically repaired BENO reference, e.g., using CRISPR corrected cells.

For assessing an anti-convulsive potency of a pharmaceutical product, it is preferred that the at least one physical parameter is one or more selected from the group consisting of (Weighted) Mean Firing Rate and Interspike Interval in a burst (s), and that the predetermined threshold deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1x SD or 2x SD. Alternatively, or additionally, said predetermined threshold is an at least 15%, preferably at least 25% or 30%, more preferably at least 55% or 60% change of a (mean) reference value obtained for the at least one physical parameter in a reference (experiment), in which the at least one physical parameter is preferably determined in the absence of the pharmaceutical product using an engineered tissue that is derived from a healthy individual or a subject having the disorder and/or well-characterized iPSC representing a healthy wildtype or modified to be representative for the disorder. Thus, a pharmaceutical product is preferably assessed and/or determined to be, preferably anti-convulsive, potent for treating the disorder by at least partially restoring the functionality of the physiological bodily function if a value of the at least one physical parameter determined in a potency assay as disclosed herein exceeds the predetermined threshold of preferably 2x SD and/or (approximately) 15%, 25%, 30%, 55% or 60% change of said (mean) reference value. More specifically, it is preferred that in case of the at least one physical parameter being (Weighted) Mean Firing Rate said predetermined threshold is preferably 2x SD and/or (approximately) 55% or 60% change of said (mean) reference value; in case of the at least one physical parameter being Interspike Interval in a burst (s) said predetermined threshold is preferably 2x SD and/or (approximately) 30% change of the respective (mean) reference value.

It is particularly preferred that the at least one physical parameter is or comprises (Weighted) Mean Firing Rate and that the predetermined threshold deviates from a (mean) reference value obtained for the at least one physical parameter in a reference (experiment) by 2x SD and/or (approximately) 60% change of the respective (mean) reference value.

## Muscle tissue

The method of the invention further can be used to assay the potency of the pharmaceutical product assumed to be effective in treating a disorder involving muscle tissue. Accordingly, the engineered tissue may be muscle tissue, preferably skeletal muscle tissue, or a muscle.

The person skilled in the art is capable of preparing an engineered muscle tissue. Methods for producing muscle tissue are, e.g., disclosed in WO 2021/074126, hereby incorporated by reference in its entirety. In particular, engineered skeletal muscle tissue in an exemplary embodiment can be produced from pluripotent stem cells as follows: (i) inducing mesoderm differentiation of the pluripotent stem cells by culturing pluripotent stem cells in a basal medium comprising an effective amount of (a) FGF2, (b) a GSK3 inhibitor, (c) a SMAD inhibitor, and (d) a serum-free additive comprising transferrin, insulin, progesterone, putrescine, and selenium or a bioavailable salt thereof; (ii) inducing myogenic specification by culturing the cells obtained in step (i) in a basal medium comprising an effective amount of (a) a gamma-secretase/ NOTCH inhibitor, (b) FGF2, and (c) a serum-free additive as in (i), followed by continuing culturing in said medium with the addition of an effective amount of (d) HGF, followed by culturing said cells in a basal medium comprising an effective amount of (a) a gamma secretase/NOTCH inhibitor, (b) HGF, (c) a serum-free additive as in (i), and (d) knockout serum replacement (KSR); (iii) expanding and maturing the cells into skeletal myoblasts and satellite cells by culturing the cells obtained in step (ii) in a basal medium comprising an effective amount of (a) HGF, (b) a serum-free additive as in (i), and (c) knockout serum replacement (KSR); and (iv) maturing the cells into skeletal myotubes and satellite cells by culturing the cells obtained in step (iii) dispersed in an extracellular matrix under mechanical stimulation in a basal medium, comprising an effective amount of (a) a serum-free additive as in step (i) and (b) an additional serum-free additive comprising albumin, transferrin, ethanolamine, selenium or a bioavailable salt thereof, L-carnitine, fatty acid additive, and triod-L-thyronine (T3); thereby producing artificial skeletal muscle tissue. The pluripotent stem cells may have been obtained from the patient, who will undergo a treatment with the pharmaceutical product. The pluripotent stem cells may have not been obtained from the patient, who will undergo a treatment with the pharmaceutical product.

The physiological bodily function of (skeletal) muscle tissue is to exert a force in order to move a part of the body. Accordingly, the method of the invention is particularly useful when the at least one physical parameter determined in the method is force, e.g., expressed in Newton (N), or movement, e.g., expressed as the velocity of the moving entity in meter per second (m/s). Accordingly, in one embodiment, the at least one physical parameter is force. In another embodiment, the at least one physical parameter is movement. In another embodiment, the at least one physical parameter is velocity.

Methods for determining the force exerted by a (skeletal) muscle tissue are known by the person skilled in the art and are, e.g., described in WO 2021/074126, Tiburcy et al. (2019, FASEB Bioadv, 1(12):731-746) or Shahriyari et al. (2022 Cachexia Sarcopenia Muscle, 13(6):3106-3121, doi: 10.1002/jcsm.13094), hereby incorporated by reference. Preferably, the force is determined isometrically. In an exemplary embodiment, the measurement can be carried out as follows: The contractile function of engineered skeletal muscle tissue can be measured under isometric conditions in an organ bath filled with gassed (5% $CO_2$/95% $O_2$) Tyrode solution (containing in mmol/L: 120 NaCl, 1 $MgCl_2$, 0.2 $CaCl_2$, 5.4 KCl, 22.6 $NaHCO_3$, 4.2 $NaH_2PO_4$, 5.6 glucose, and 0.56 ascorbate) at 37°C. To verify the force-length relationship, while ESMs can be electrically stimulated at 1 Hz with 5 ms square pulses of 200 mA, muscle length can be increased by mechanical stretch at intervals of 125 $\mu$m until the maximum contraction force was observed. At the length of maximal force generation, tetanic contraction force can be assessed under defined stimulation frequencies (4-second stimulation at 10, 20, 40, 60, 80, and 100 Hz).

Engineered (skeletal) muscle tissue typically exerts a force of 0.01 mN or more when it is healthy. Accordingly, the one or more conditions preferably comprise that the engineered tissue exerts a force of 0.01 mN or more, 0.05 mN or more, 0.1 mN or more, or 1 mN or more.

The physiological bodily function of (skeletal) muscle tissue may be impaired by a variety of disorders. Exemplary disorders include genetic muscular disorder, preferably a genetic muscular disorder selected from the group consisting of Duchenne muscular dystrophy, Facioscapulohumeral dystrophy, Becker dystrophy, Emery-Dreifuss dystrophy, myotonic dystrophy, limb-girdle dystrophy, oculopharyngeal muscular dystrophy, congenital dystrophies, congenital myopathies, myotonia congenita, familial periodic paralysis, and a heritable connective tissue disease such as Marfan syndrome, Ehlers-Danlos syndrome or Loeys-Dietz syndrome. Preferably, the disorder is Duchenne muscular dystrophy. Alternatively, or additionally, the disorder may be a non-genetic muscular disorder.

In one particular embodiment,

the engineered tissue is heart muscle,
the pharmaceutical product is an AAV2 and/or AAV9 viral vector comprising a nucleic acid encoding Cas9, preferably *S. aureus* Cas9, *S. auricularis* Cas9, *S. lugdunensis* Cas9, or *N. meningitides* Cas9, and one or two guide RNA(s) (gRNA), wherein the Cas9 gene is under the control of a muscle-specific promoter such as CK8e or TNNT2,
the disorder is Duchenne muscular dystrophy, and
the physical parameter is force,
preferably wherein the engineered tissue comprises cells obtained from a patient, who will undergo a treatment with the pharmaceutical product.

In one particular embodiment,

the engineered tissue is skeletal muscle,
the pharmaceutical product is an AAV2 and/or AAV9 viral vector comprising a nucleic acid encoding Cas9, preferably *S. aureus* Cas9, *S. auricularis* Cas9, *S. lugdunensis* Cas9, or *N. meningitides* Cas9, and one or two guide RNA(s) (gRNA), wherein the Cas9 gene is under the control of a muscle-specific promoter such as CK8e or TNNT2,
the disorder is Duchenne muscular dystrophy, and
the physical parameter is force,
preferably wherein the engineered tissue comprises cells obtained from a patient, who will undergo a treatment with the pharmaceutical product.

In some embodiments, the engineered tissue is muscle tissue and the disorder is a muscle tissue disorder, preferably DMD.

Preferably, the at least one physical parameter is tissue contraction force, preferably determined as twitch tension and/or using a stimulation frequency of 40 Hz or 100 Hz. It is preferred that the at least one physical parameter is determined approximately on day 10 or 14 of the drug effect testing phase and thus, day 10 or 14 of incubation of the engineered tissue, wherein the engineered tissue is preferably an ESM. It is further preferred that the predetermined threshold is determined based on and/or obtained by a reference (experiment), in which preferably the at least one physical parameter of an engineered tissue is at least determined in the absence of the pharmaceutical product. It is preferred that, the predetermined threshold is based on and/or obtained by a reference (experiment) using a statistical method and deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1.5x or 2x SD. Alternatively, or additionally, said deviation is expressed as % change of the respective (mean) reference value.

**Neuronal junctions**

The versatility of the method of the present invention also allows assaying the potency of pharmaceutical products for treating a disorder based on a mixture of different types of engineered tissues. In one example, such a mixture of includes neurons and skeletal or heart muscle tissue. This can resemble a neuronal junction and the method of the invention can in this embodiment be used to assay the potency to treat disorders present at neuromuscular junctions or more particular disorders, which impair the function of the neuromuscular junctions. Here, the engineered tissue can comprise neurons such as BENOs described herein and skeletal or heart muscle tissue, which have been co-cultured during preparation of the engineered tissue (see also WO 2018/228948, hereby incorporated by reference in its entirety).

Accordingly, the engineered tissue may be a composition of different types of tissues. Preferably, the engineered tissue may be a composition of neurons and skeletal muscle tissue. Preferably, the engineered tissue may be a composition of neurons and heart muscle tissue. Preferably, the composition of different types of tissues forms one or more neuro-muscular junction. A "neuro-muscular junction" (or myoneural junction) is a chemical synapse between a motor neuron and a muscle fiber.

**Connective tissue**

Connective tissue is one of the many basic types of animal tissue, along with epithelial tissue, muscle tissue, and nervous tissue. In embryology it develops from the mesoderm. Connective tissue typically is found in between other tissues everywhere in the body, including the nervous system. The three outer membranes (the meninges) that envelop the brain and spinal cord are composed of dense inert connective tissue. Connective tissue typically comprises three main components: fibers (elastic and collagen fibers), ground substance and cells. All are immersed in the body water. The cells of connective tissue may comprise fibroblasts, adipocytes, macrophages, mast cells and leucocytes. Accordingly, the engineered tissue may in one embodiment be connective tissue.

Connective tissue may suffer from disorders that can be treated by a pharmaceutical product as defined herein. Accordingly, the disorder preferably is (i) a heart disease which presents with cardiac fibrosis, such as a heart failure (of unknown and/or genetic cause) with reduced or preserved ejection fraction, myocardial infarction, a congenital heart disease, a cardiomyopathy with known mutation(s), age- and/or senescence-induced cardiac fibrosis, a drug-induced cardiac disorder, a metabolic cardiac disorder, a (monogenetic or polygenetic) disorder with contribution of the heart and cardiac fibrosis, a disease involving reactive or replacement fibrosis in an organ such as kidney, liver, lung, and/or skin, etc., (ii) a disorder with impaired scaring and wound healing such as Diabetes mellitus, and/or (iii) a heritable connective tissue disease such as Marfan syndrome, Ehlers-Danlos syndrome or Loeys-Dietz syndrome.

Connective tissue has a wide variety of physiological bodily functions that depend on the types of cells and the different classes of fibers involved. Loose and dense irregular connective tissue, formed mainly by fibroblasts and collagen fibers, have an important role in providing a medium for oxygen and nutrients to diffuse from capillaries to cells, and carbon dioxide and waste substances to diffuse from cells back into circulation. They also allow organs to resist stretching and tearing forces. Dense regular connective tissue, which forms organized structures, is a major functional component of tendons, ligaments and aponeuroses, and is also found in highly specialized organs such as the cornea. Elastic fibers, made from elastin and fibrillin, also provide resistance to stretch forces. They typically are found in the walls of large blood vessels and in certain ligaments, particularly in the ligamenta flava. In hematopoietic and lymphatic tissues, reticular fibers made by reticular cells provide the stroma-or structural support-for the parenchyma-or functional part-of the organ. Mesenchyme is a type of connective tissue found in developing organs of embryos that is capable of differentiation into all types of mature connective tissue. Another type of relatively undifferentiated connective tissue is the mucous connective tissue known as Wharton's jelly, found inside the umbilical cord. Various types of specialized tissues and cells are classified under the spectrum of connective tissue, and are as diverse as brown and white adipose tissue, blood, cartilage and bone.

Wound healing is characterized by connective tissue formation, i.e., scarring as replacement for damaged organ tissue also called replacement fibrosis. Accordingly, the disorder can be fibrosis, e.g., fibrosis in or of an organ. Connective tissue as replacement fibrosis is observed for example after myocardial infarction, in cardiomyopathies, in muscle damage, such as fibrofatty replacement in DMD, in stroke patients, toxic liver damage, kidney disease, or skin injury.

Connective tissue can be characterized by its biomechanical parameters. Biomechanical parameters include parameters such as tissue compaction, contraction, stiffness, ultimate strength, elasticity, extensibility, excitability, resilience, or toughness. Accordingly, the at least one physical parameter may be tissue compaction. The at least one physical parameter may be (tissue) contraction. The at least one physical parameter may be stiffness. Stiffness is the extent to which an object resists deformation in response to an applied force. The at least one physical parameter may be ultimate strength. The at least one physical parameter may be elasticity. Elasticity is the ability of a body to resist a distorting influence and to return to its original size and shape when that influence or force is removed. The at least one physical parameter may be extensibility. Extensibility is the ability of a body to resist a distorting influence before fracturing (and to return to its original size and shape when that influence or force is removed). The at least one parameter may be excitability. The at least one physical parameter may be resilience. The at least one physical parameter may be toughness. Toughness is the ability of a material to absorb energy and elastically and plastically deform without fracturing.

Methods for producing engineered connective tissue are known to a person skilled in the art. Cardiac stromal cells, a type of connective tissue, can be produced as described in WO 2022/023451 A1 or Santos et al. (2021), J. Vis. Exp., 174:e62700, which are hereby incorporated by reference in its entirety. An exemplary method of producing engineered connective tissue is laid out in the following: First, prepare stromal cells, e.g., as described in WO 2022/023451 A1, or fibroblast. Then, dissociated stromal cells or fibroblasts are casted together with an extracellular matrix, e.g., collagen, into a suitable mold.

In some embodiments, the engineered tissue is connective tissue and the disorder is (i) a heart disease which presents with cardiac fibrosis, such as a heart failure (of unknown and/or genetic cause) with reduced or preserved ejection fraction, myocardial infarction, a congenital heart disease, a cardiomyopathy with known mutation(s), age- and/or senescence-induced cardiac fibrosis, a drug-induced cardiac disorder, a metabolic cardiac disorder, a (monogenetic or polygenetic) disorder with contribution of the heart and cardiac fibrosis, a disease involving reactive or replacement fibrosis in an organ such as kidney, liver, lung, and/or skin, etc., (ii) a disorder with impaired scaring and wound healing such as Diabetes mellitus, and/or (iii) a heritable connective tissue disease such as Marfan syndrome, Ehlers-Danlos syndrome or Loeys-Dietz syndrome.

As regards the choice of a suitable reference (experiment), two scenarios may be exemplarily illustrated that the skilled artisan is aware of. In case of a non-genetic disorder, like a non-genetically caused (cardiac) fibrosis, potency of an anti-fibrotic acting pharmaceutical product may be indicated in case a, preferably statistically significant, difference of at least one physical parameter value is observed between a treated engineered tissue or organoid and at least a reference (experiment) being or comprising a healthy wildtype engineered tissue or organoid. However, in case a genetic mutation is associated with a disease and the pharmaceutical product may be acting in this respect and/or is an individualized therapy medicament, then anti-fibrotic potency may be indicated in case a, preferably statistically significant, difference of at least one physical parameter value is observed between a treated engineered tissue or organoid and at least a reference (experiment) being or comprising an untreated though mutated engineered tissue or organoid.

Preferably, the at least one physical parameter is tissue contraction and/or stiffness, preferably measured as pole deflection and/or Young's modulus. It is preferred that the at least one physical parameter is determined approximately on day 10 of the drug effect testing phase and thus, day 10 of incubation of the engineered tissue, wherein the engineered tissue is preferably an ECT. It is further preferred that the predetermined threshold is determined based on and/or obtained by a reference (experiment), in which preferably the at least one physical parameter of an engineered tissue is at least determined in the absence of the pharmaceutical product. It is preferred that, the predetermined threshold is based on and/or obtained by a reference (experiment) using a statistical method and deviates from a (mean) reference value obtained for the at least one physical parameter in a reference (experiment) by (at least) between 0.5x and 2.5x standard deviations (SD) and/or by (at least) 0.5x SD, 1x SD, 1.5x SD or 2x SD, preferably by 1x SD or 1.5x SD. Alternatively, or additionally, said deviation is expressed as % change of the respective (mean) reference value.

For assessing a pro-fibrotic potency of a pharmaceutical product, it is preferred that the at least one physical parameter is measured as pole deflection and/or Young's modulus, and that the predetermined threshold deviates from a (mean) reference value obtained for the at least one physical parameter in a reference (experiment) by 1x SD or 1.5x SD. Alternatively, or additionally, said predetermined threshold is an at least 30% or 35%, or 55% or 60% change of a (mean) reference value obtained for the at least one physical parameter in a reference (experiment) in absence of TGF-$\beta$1, in which the at least one physical parameter is preferably determined in the absence of the pharmaceutical product using an engineered tissue that is derived from a healthy individual or a subject having the disorder and/or well-characterized iPSC representing a healthy wildtype or modified to be representative for the disorder. Thus, in case of the at least one physical parameter being or comprising pole deflection, the pharmaceutical product is preferably assessed and/or determined to be, preferably pro-fibrotic, potent for treating the disorder by at least partially restoring the functionality of the physiological bodily function if a value of the at least one physical parameter determined in a potency assay as disclosed herein exceeds the predetermined threshold of preferably 1x SD and/or (approximately) 35% change of said (mean) reference value in the absence of TGF-$\beta$ 1. In case of the at least one physical parameter being or comprising Young's modulus, the pharmaceutical product is preferably assessed and/or determined to be, preferably pro-fibrotic, potent for treating the disorder by at least partially restoring the functionality of the physiological bodily function if a value of the at least one physical parameter determined in a potency assay as disclosed herein exceeds the predetermined threshold of preferably 1.5x SD and/or (approximately) 60% change of the respective (mean) reference value in the absence of TGF-$\beta$ 1.

For assessing an anti-fibrotic potency of a pharmaceutical product, it is preferred that the at least one physical parameter is measured as pole deflection and/or Young's modulus, and that the predetermined threshold deviates from a (mean) reference value obtained for the at least one physical parameter in a reference (experiment) by 1x SD or 1.5x SD. Alternatively, or additionally, said predetermined threshold is an at least 30% or 35%, or 55% or 60% change of a (mean) reference value obtained for the at least one physical parameter in a reference (experiment) in presence and/or absence of TGF-$\beta$1, in which the at least one physical parameter is preferably determined in the absence of the pharmaceutical product using an engineered tissue that is derived from a healthy individual or a subject having the disorder and/or well-characterized iPSC representing a healthy wildtype or modified to be representative for the disorder. Thus, in case of the at least one physical parameter being or comprising pole deflection, the pharmaceutical product is preferably assessed and/or determined to be, preferably anti-fibrotic, potent for treating the disorder by at least partially restoring the functionality of the physiological bodily function if a value of the at least one physical parameter determined in a potency assay as disclosed herein exceeds the predetermined threshold of preferably 1x SD and/or (approximately) 35% change of said (mean) reference value in the absence of TGF-$\beta$1 and/or (approximately) 30% change of said (mean) reference value in the presence of TGF-$\beta$1. In case of the at least one physical parameter being or comprising Young's modulus, the pharmaceutical product is preferably assessed and/or determined to be, preferably anti-fibrotic, potent for treating the disorder by at least partially restoring the functionality of the physiological bodily function if a value of the at least one physical parameter determined in a potency assay as disclosed herein exceeds the predetermined threshold of preferably 1.5x SD and/or (approximately) 60% change of said (mean) reference value in the absence of TGF-$\beta$1 and/or (approximately) 55% or 60% change of the respective (mean) reference value in the presence of TGF-$\beta$1.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "including" and "including but not limited to" are used interchangeably.

As used herein the term "about" or "approximately" means within 20%, preferably within 15%, preferably within 10%, and more preferably within 5% of a given value or range. It also includes the concrete number, i.e. "about 20" includes the number of 20.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

## EXPERIMENTAL EXAMPLES

An even better understanding of the present invention and of its advantages will be evident from the following experimental examples, offered for illustrative purposes only. The examples are not intended to limit the scope of the present invention in any way.

**Example 1: Drug effect assessment on ECT using pole deflection**

Background

Engineered connective tissues (ECT) may be generated with different types of fibroblasts, including induced human pluripotent stem cell-derived stromal cells (iPSC-StC) and primary human fibroblasts of different origin, e.g., skin, gingiva, heart. Dependent on the disease context under study, pro-fibrotic and anti-fibrotic drugs can be tested and characterized with regard to their potency. For example, in the context of cardiac fibrosis, anti-fibrotic drugs are needed to prevent excessive extracellular matrix deposition into the myocardium mainly by cardiac fibroblasts. Oppositely, Diabetes mellitus is usually associated with an impaired wound healing of the skin, and thus, drugs that are able to induce a temporary conversion of skin fibroblasts into more contractile myofibroblasts are of interest as they could support and/or enhance wound healing.

ECT generation

The general experimental procedure was identical for all used cells, including primary fibroblasts and iPSC-StC. Supporting information in view of the ECT model, in particular its generation, may also be found in Santos et al. (2021, Fibroblast Derived Human Engineered Connective Tissue for Screening Applications. J. Vis. Exp. e62700, doi:10.3791/62700, PMID: 34487119), Santos et al. (2022, Using different geometries to modulate the cardiac fibroblast phenotype and the biomechanical properties of engineered connective tissues. Biomater Adv. 139:213041, PMID: 35909053), and EP20188364. In particular, acid solubilized, collagen type 1 was mixed 1:1 with 2x DMEM containing 20% fetal calf serum and antibiotics. The pH of the collagen mixture was neutralized with NaOH, then combined with the iPSC-StCs or fibroblasts and carefully mixed to generate ECT composed of 0.3 mg collagen and $0.75 \times 10^6$ cells per 180 $\mu$l per ECT. The cell-collagen mixture was distributed in molds of a 48-well myrPlate (Tiburcy et al. 2020 STAR Protocols and WO2017/207431) holding two poles made of pliable polymer (e.g., TM5MED) material. After casting, ECT were allowed to solidify for 1h in a cell incubator, then the appropriate cell culture medium was added and replaced every second day afterwards.

Pole deflection analysis

Pole deflection measurement and platforms are well-established in the art, cf. e.g. WO2017/207431 (also granted as DE 10 2016 110 328 B3). For the examples presented herein, poles were imaged under UV light directly after the first medium addition and in the following once a day. Pole deflection as a function of ECT compaction was calculated as follows: $100 \times$ (pole distance$_{day\ 0}$-pole distance$_{day\ X}$)/pole distance$_{day\ 0}$.

Study design

Fibrotic processes are triggered by mechanical and/or biochemical cues, which induce specific signaling cascades that are partly interlinked. Therefore, "ideal" anti-fibrotic drugs should interfere with both stressor types and thus, testing is preferably performed in conditions where only the mechanical constraint induces cell activation versus conditions in which mechanical activation is combined with the application of a pro-fibrotic cytokine, preferably of TGF-$\beta$1. Preferably, drug testing is performed when a stable ECT has formed and initial processes not related to fibrosis are completed. Thus, drug testing is preferably performed when formerly 2D-cultured cells adapted to a 3D environment as in case of an ECT model. In the ECT model, cell adaptation may occur within the first few days in parallel to ECT compaction and contraction phase I. Therefore, drug application is preferably performed when the ECT reaches the contractile plateau and before contraction phase II is executed, which demarcates cell reactivation. According to the current understanding of fibrotic mechanisms, the mechanical-driven contraction phase II can be further enhanced by the application of a pro-fibrotic cytokine like TGF-$\beta$1, when the ECT has reached the contractile plateau.

Methodological aspects

For pole deflection, absolute detection limits were as follows: upper limit (theoretically) 100%, lower limit (theoretically) 0%. However, without being bound to theory practically observed detection limits can be approximately 60-70% (upper limit) and 2% (lower limit), respectively. Relative detection limits were approximately 5-fold to 10-fold of the plateau value (upper limit) and approximately 0.25-fold of the plateau value (lower limit), respectively.

ECT treatment

Reference ECT were analyzed, which were treated with a drug solvent in absence or presence of TGF-β1, with TGF-β1 application serving as additional reference with respect to pro-fibrotic drug characterization. Both reference conditions were performed in 6 technical replicates. Pole deflection was observed to reach a maximum between day 3 and day 5. Thus, mechanical-driven contraction phase II was further enhanced in case of the TGF-β1 reference ECT by the application of 5 ng/ml TGF-β1 at day 4 (**Figure 2A**).

In the treatment conditions, the drug of interest will be applied when the pole deflection reaches a maximum between day 3 and day 5. Herein, the day on which during the plateau phase a drug will be added may also be referred to as day 1 of a drug effect testing phase. Thus, in the illustrative examples disclosed herein, day 1 of the drug effect testing phase will represent day 4 of the ECT culture. Said drug effect testing phase may be as long as the ECT culture time. Drug concentrations will be chosen based on the $IC_{50}/EC_{50}$ values of the respective drug, if available (**Figure 2B**, left side). Used concentrations will range between $IC_{50}/EC_{50} \times \pm$ 30-fold and will be applied in half log steps, e.g. when an $IC_{50}$ will be 1 μM then concentrations of 0.03, 0.1, 0.3, 1, 3, 10, and 30 μM will be tested. As above, reference ECT will be analyzed, which will be treated with the drug solvent (in the absence of TGF-β1). All conditions will be performed in 6 technical replicates. On a second plate, the same experimental setting will be used; however, 5 ng/ml TGF-β1 will be additionally added to the samples (**Figure 2B**, right side). When the $IC_{50}/EC_{50}$ values of a given drug will be unknown, then the highest concentration will be a 100-fold dilution of the drug's solubility, e.g. when the maximal soluble concentration will be 100 mM, the highest used final concentration will be 1 mM. In addition, 6 other concentrations descending in log steps and the solvent will be tested. The medium with the drugs will be exchanged every second day during the next, e.g., 10 days of culture.

Classification of drugs based on their effects on contraction phase II

Statistical analysis of the reference ECT revealed that lowering contraction by a minimum of 1xSD is significant in a two-way ANOVA analysis with a Tukey's multiple comparison test. Table 3 summarizes the (mean) contraction (in %) in reference ECT (without TGFβ-1) and TGFβ-1 reference ECT determined by pole deflection analysis with a deviation by 1x SD equating to a change in the inhibition of the contraction increase between plateau and day 13 (absolute; day 10 of the drug effect testing phase) of 35% (reference without TGFβ-1) and 30% (reference with TGFβ-1), respectively.

**Table 3: Values from day 10 of the drug effect testing phase (absolute day 13)**

| Reference | Contraction (%) | 1xSD | Inhibition of the contraction increase between the plateau and day 13 |
|---|---|---|---|
| Without TGFβ-1 | 23.2 | 5.7 | 35% |
| With TGF-β1 | 39.4 | 9.6 | 30% |

Expected interference of a theoretically and/or illustratively "ideal" anti-fibrotic drug with contraction phase II in the absence or presence of TGF-β1 is presented in **Figure 2C and 2D**, respectively. Application of the $IC_{50}$ concentration will result in a 50% inhibition of the contraction phase II without impeding the degree of contraction resulting from contraction phase I. Thus, said inhibition of contraction phase II will be assessed based on pole deflection observed at the end of culture and the plateau and thus, herein at day 10 and day 1 of the drug effect testing phase, respectively, and the resulting difference compared between ECT models with drug treatment (treatment condition(s)) and without drug treatment (reference condition(s)). In this illustrative example, pole deflection at day 13 is expected to be significantly inhibited by approximately 30% and approximately 35% with and without TGF-β1, respectively, already by 0.3x of the $IC_{50}$ concentration. Said day 13 corresponds within this illustrative example to the last culture day and/or day 10 of the drug effect testing phase.

Accordingly, a drug with an anti-fibrotic activity is expected to display in an ECT model, e.g. (approximately) on day 10 of a drug effect testing phase, in at least one of both settings (+/-TGF-β1) an inhibition of contraction phase II by a minimum of 1x SD compared to the respective reference (**Figure 2E**). A drug, which will enhance in an ECT model contraction phase II in the absence of TGF-β1 by a minimum of 1x SD compared to the respective reference, e.g. (approximately) on day 10 of a drug effect testing phase, may be considered to be pro-fibrotic.

In this illustrative example as well as the following ones, ECT culture time under study will end on day 13, which thus will correspond to the last culture day and/or day 10 of the drug effect testing phase. However, the skilled artisan is aware that culture times may depend on the cell type and ECT under study and hence, the last culture day and/or the herein used day 10 of the drug effect testing phase may be an earlier or later day than mentioned herein. For example, a longer culture period may be advantageous, when cells and/or ECT under study react slower than in case of the observations made herein.

**Example 2: Drug effect assessment on ECT using ultimate tensile testing**

Background

Interferences with the contractile properties of fibroblasts represented a potential action of an anti- or pro-fibrotic drug. However, it does not necessarily indicate that the drug can also interfere with the production or degradation of extracellular matrix. Therefore, plateau and reference stiffness were assessed and drugs will be tested with respect to their influence on ECT stiffness by performing in a follow-up analysis of **Example 1** ultimate tensile testing.

ECT generation and study design

Reference ECT were generated and treated similar as described herein in **Example 1.** In contrast to the pole deflection analysis disclosed in **Example 1** herein, 8 ECT were harvested for the ultimate tensile testing at the moment when the plateau was reached and before a drug solvent was applied with or without TGF-$\beta$1. Residual ECT were cultured for further 10 days. Then, macroscopic images were taken from the ECT on the poles to estimate their cross-sectional areas based on the observed ECT thicknesses. Ultimate tensile testing was performed with a dynamic mechanical analyzer (DMA). Therefore, ECT were loaded onto two hooks clamped to a DMA in a 37°C tempered organ bath filled with PBS. Then, uniaxial stretch at a constant linear rate of approximately 1% (0.03 mm/s) was applied until the ECT ruptured. The recorded force traces were converted into stress-strain curves. The Young's modulus representing the stiffness of the ECT was analyzed by determining the slope of the linear part of curve within the elastic region (**Figure 3A**). All measurements were performed in 8 replicates.

In the treatment conditions, the study design will be as described in the paragraph above for the reference ECT except that effective drug concentrations will be added with or without TGF-$\beta$1 at the moment when the plateau will be reached. In the herein described illustrative example, considered concentrations will range from 0.3x to 30x of the $IC_{50}$ concentration. All measurements will be performed in 8 replicates.

Methodological aspects

For Young's modulus, absolute detection limits were 1 MPa (upper limit) and 10 kPa (lower limit), respectively. Relative detection limits were approximately 50-fold of the plateau value (upper limit) and approximately 0.5-fold of the plateau value (lower limit), respectively.

Classification of drugs based on their effects on stiffness (Young's modulus)

Statistical analysis of the reference ECT revealed that lowering contraction by 1.5xSD was significant in a one-way ANOVA analysis with a Dunnet's multiple comparison test. **Table 4** summarizes (mean) Young's modulus (kPa) values in reference ECT (without TGF$\beta$-1) and TGF$\beta$-1 reference ECT determined by ultimate tensile testing with a deviation by 1.5x SD equating to a change in the inhibition of Young's modulus increase between plateau and day 13 (absolute; day 10 of the drug effect testing phase) of 59% (reference without TGF$\beta$-1) and 57% (reference with TGF$\beta$-1), respectively.

**Table 4: Values from day 10 of the drug effect testing phase (absolute day13)**

| Reference | Young's modulus (kPa) | SD | 1.5xSD | Inhibition of Young's modulus increase between the plateau and day 13 |
|---|---|---|---|---|
| without TGF-$\beta$1 | 49.4 | 10.6 | 15.9 | 59% |
| With TGF-$\beta$1 | 76.4 | 21.6 | 32.4 | 57% |

Ultimate tensile testing of treatment condition ECT is expected to reveal an increase in stiffness at the end of the drug effect testing phase compared to measurements executed during the plateau phase. The increase will be further enhanced by the application of 5 ng/ml TGF-$\beta$ 1 at the beginning of the drug effect testing phase. Expected interference of a theoretically and/or illustratively "ideal" anti-fibrotic drug with the stiffness of an ECT in the absence and presence of TGF-$\beta$1 is presented in **Figure 3B.** Application of 0.3x of the $IC_{50}$ concentration is expected to result in an approximately 30% inhibition of the stiffness increase and application of the $IC_{50}$ concentration to result in a 50% inhibition of the stiffness increase, with said inhibition occurring between the plateau and the end of culture. E.g. in case of the $IC_{50}$

concentration, inhibition of the stiffness increase will be determined as follows:

$$IC_{50} \text{ stiffness} = \text{Stiffness}_{Plateau} + (\text{Stiffness}_{End} - \text{Stiffness}_{Plateau})/2$$

with Stiffness$_{Plateau}$ being determined in this illustrative example on day 1 of the drug effect testing phase and Stiffness$_{End}$ at culture end and thus, in this illustrative example on day 10 of the drug effect testing phase.

Accordingly, a drug with anti-fibrotic activity should display in an ECT model, e.g. (approximately) on day 10 of a drug effect testing phase, in at least one of both settings (+/-TGF-β1) an inhibition of the stiffness increase by a minimum of 1.5x SD compared to a respective reference. A drug, which enhances in an ECT model the stiffness increase in the absence of TGF-β1 by a minimum of 1.5x SD compared to a respective reference, e.g. (approximately) on day 10 of a drug effect testing phase, may be considered pro-fibrotic.

**Example 3: Potency assay for Pirfenidone in heart failure with preserved ejection fraction using ECT and both pole deflection and ultimate tensile testing**

Background

In view of the results obtained in **Examples 1 and 2,** a potency assay will be performed for Pirfenidone. Pirfenidone is an orally administered, synthetic small-molecule derivative of pyridine with approved therapeutic efficacy in idiopathic pulmonary fibrosis. Pirfenidone appears to have a broad range of effects on inflammation and other fibrotic pathways, as well as an inhibitory effect on TGFβ signaling. As Pirfenidone is under study in clinical testing as anti-fibrotic drug for patients with heart failure with preserved ejection fraction, which is characterized by a diastolic dysfunction of the heart that correlates with the degree of fibrosis in the myocardium. Thus, cardiac fibrosis is an important therapeutic target in heart failure with preserved ejection fraction. Hence, Pirfenidone will be investigated using an ECT based potency assay. Pole deflection and ultimate tensile testing will be chosen as representative physical parameters as both contractility and stiffness of the cardiac extracellular matrix may be considered essential for reliably studying biochemical properties of ECT upon Pirfinidone treatment in vitro. Thus, pole deflection and/or ultimate tensile testing will represent for the potency assay herein a measure of the functionality of the physiological bodily function.

Study design

ECT will be generated and pole deflection and ultimate tensile testing performed as described herein in case of **Examples 1 and 2,** except that ECT will be generated from well-characterized iPSC representing a healthy wildtype or iPSC obtained from healthy individuals as reference ("healthy" +/-TGF-β1) as well as from iPSC obtained from patients with heart failure with preserved ejection fraction. More specifically, ECT derived from patients will be divided into a further reference ("diseased" +/-TGF-β1), a "low" treatment group with 0.3 mg/ml Pirfenidone treatment (+/-TGF-β1) and a "high" treatment group with 1 mg/ml Pirfenidone treatment (+/-TGF-β1). For treatment, Pirfenidone will be dissolved in the respective culture medium in the concentration given for the respective group. The drug's IC$_{50}$ was determined to be 0.43 mg/ml in vitro and thus, the "high" treatment group will receive a treatment of Pirfenidone in a concentration being at least at the drug's IC$_{50}$ concentration. Investigated concentrations will represent supratherapeutic concentrations in a range that is commonly used for Pirfenidone analyses in vitro as Pirfenidone is commonly found to be less potent in in vitro compared to in vivo settings. All physical parameter values will be obtained on day 10 of the drug effect testing phase (absolute day 13) for 8 to 10 replicates per group. The predetermined threshold of the at least one physical parameter will be in case of pole deflection 1x SD and in case of ultimate tensile testing 1.5x SD of the respective (mean) value of said parameter observed in the respective reference ECT. Pirfenidone will be assumed to be potent for treating heart failure with preserved ejection fraction if a value of the at least one physical parameter (pole deflection and/or ultimate tensile) exceeds in at least one of the two settings (+/- TGF-β1) the predetermined threshold for said parameter obtained in at least one of the two respective reference conditions ("healthy" +/- TGF-β1 and "diseased" +/- TGF-β1, respectively), preferably in at least the "diseased" +/- TGF-β1 reference condition.

(Expected) Outcome

It is expected that Pirfenidone shows comparable effects in both settings (+/- TGF-β1). Furthermore, it is expected that at the end of culture and thus, herein on day 10 of the drug effect testing phase, Pirfenidone treatment at least in the "high" treatment group is expected to result in a markedly inhibited contraction phase II as determined by pole deflection and/or in a markedly inhibited stiffness increase as determined using ultimate tensile testing. The (mean) contraction (in %) value in at least one of the two settings determined by pole deflection analysis is expected to meet the predetermined threshold obtained in at least one of the reference conditions in at least one of the two settings, indicating that Pirfenidone may have potency for treating heart failure with preserved ejection fraction by at least partially restoring the functionality of the physiological bodily function in view of (ECT) tissue contraction. However, in case the (mean) contraction (in %) value determined by pole deflection analysis will not meet, in neither of the two settings and/or for none of the respective reference conditions, the predetermined threshold, Pirfenidone may not be assessed to have potency for treating heart failure with preserved ejection fraction by at least partially restoring the functionality of the physiological bodily function in view of (ECT) tissue contraction. Furthermore, the (mean) Young's modulus values determined by ultimate tensile testing are not expected to meet the respective predetermined thresholds obtained in at least one of the reference conditions in any of the two settings, indicating that Pirfenidone may not have potency for treating heart failure with preserved ejection fraction as regards ECT stiffness. However, in case (mean) Young's modulus values in at least one of the two settings will meet the predetermined threshold at least obtained in one of the reference conditions, Pirfenidone will be assessed to have potency for treating heart failure with preserved ejection fraction by at least partially restoring the functionality of the physiological bodily function in view of (ECT) stiffness.

**Example 4: Genetic rescue of muscle function in tissue engineered human muscle - titration experiments**

Several muscle diseases are linked to malfunction and/or absence of a specific protein. To address the efficacy of a potential therapy, a human muscle model with depletion of dystrophin as exemplarily target protein was generated. Thus, said human muscle model can be considered as a DMD model. Genetic rescue experiments were then applied to determine the degree of genetic repair required to identify a discernable effect on muscle function as also described in more detail in Long et al. (2018), Sci Adv, 4:eaap9004.

Engineered myocardium (more specifically, Engineered Heart Muscle; EHM) was generated from induced pluripotent stem cell (iPSC)-derived cardiomyocytes with lack of dystrophin and genetically corrected, isogenic cardiomyocytes. EHM were titrated to contain 0%, 10%, 30%, 50%, and 100% corrected cardiomyocytes. EHM function was assessed by contraction experiments after 4 weeks of maturation on flexible holders. Therefore, contraction experiments were performed in organ bath experiments. Organ bath as well as multiwall plate experiments are routine methods well known to the skilled person in the art (see, e.g., Tiburcy et al., 2017, Defined Engineered Human Myocardium with Advanced Maturation for Applications in Heart Failure Modelling and Repair. Circulation 135:1832-1847; Tiburcy et al., 2020, Generation of Engineered Human Myocardium in a Multi-well Format. STAR Protoc. 1:100032; Zimmermann et al., 2005, Engineering Heart Tissue for In Vitro and In Vivo Studies, In: Dhein, S., Mohr, F.W., Delmar, M. (eds) Practical Methods in Cardiovascular Research, Springer, Berlin, Heidelberg, https://doi.org/10.1007/3-540-26574-0_34).

In particular, contraction experiments were performed under isometric conditions in organ baths at 37°C in gassed (5% $CO_2$/95% $O_2$) Tyrode's solution (containing 120 mM NaCl, 1 mM $MgCl_2$, 0.2 mM $CaCl_2$, 5.4 mM KCl, 22.6 mM $NaHCO_3$, 4.2 mM $NaH_2PO_4$, 5.6 mM glucose, and 0.56 mM ascorbate). EHM were electrically stimulated at 1.5 Hz with 5-ms square pulses of 200 mA. EHMs were mechanically stretched at intervals of 125 mm until the maximum systolic force amplitude (FOC) was observed according to the Frank-Starling law. Responses to increasing extracellular calcium (0.2 to 4 mM) were investigated to determine maximal inotropic capacity. Where indicated, forces were normalized to muscle content (sarcomeric alpha-actinin-positive cell content, as determined by flow cytometry).

Obtained results indicated that (approximately) 30% of cardiomyocytes were needed to be repaired to partially rescue contractile force, and (approximately) 50% of cardiomyocytes to fully rescue the phenotype. Repair of (approximately) 10% cardiomyocytes did not impact force production (**Figure 4;** see also Long et al. (2018), Sci Adv, 4:eaap9004).

**Example 5: Genetic rescue of muscle function in EHM - genome editing**

Having determined the degree of genetic repair required to identify a discernable effect on muscle function, genome editing conditions were compared in view of their impact in the context of DMD. DMD is often caused and/or impacted by frame-shift mutations due to deletions of one or more exons of the dystrophin gene. This can prevent translation of the dystrophin protein in its functional (wildtype) form, while a structurally compromised dystrophin is often generated that can lead to muscle cell degeneration, inflammation and fibrosis. However, there may be exons in the dystrophin gene that, when skipped, can convert DMD to a milder phenotype and/or can even restore functional (wildtype) dystrophin generation in cases of some duplications. Thus, different genome editing conditions were investigated to assess the potential of exon skipping on correcting human DMD frame-shift mutations.

Engineered heart muscle (EHM) was created from induced pluripotent stem cells obtained from a patient with Duchenne Muscular Dystrophy using a method described by Tiburcy et al. (2017, Defined Engineered Human Myocardium with Advanced Maturation for Applications in Heart Failure Modelling and Repair. Circulation 135:1832-1847). Organ bath as well as multiwall plate experiments are routine methods well known to the skilled person in the art (see, e.g., Tiburcy et al., 2017, Defined Engineered Human Myocardium with Advanced Maturation for Applications in Heart Failure Modelling and Repair. Circulation 135:1832-1847; Tiburcy et al., 2020, Generation of Engineered Human Myocardium in a Multi-well Format. STAR Protoc. 1:100032) and were performed to obtain data on force of contraction (FOC) in EHM from a DMD patient compared to EHM from induced pluripotent stem cells from an unrelated healthy donor. Force of contraction was markedly reduced in the DMD-patient derived EHM. Genome editing was applied by targeted CRISPR/Cas9 insertion of insertion/deletion mutations (indels) in an attempt to rescue the DMD phenotype and test a potential therapeutic strategy.

Comparing in total 6 different editing conditions, resulted in the identification of 4 edits associated with normalization of contractile function and 2 edits which did not result in a considerable rescue (**Figure 5**). Thus, this Example demonstrated the application of engineered muscle tissue in the identification of therapeutically efficient genome editing strategies. The at least one physical parameter may differ from a predetermined (mean) threshold obtained in a reference (experiment; i.e., for example a patient-derived DMD-EHM) by (at least) 1x SD or 1.5x SD. Alternatively, or additionally, said predetermined threshold may be at least 30% or 35%, or 55% or 60% change of a (mean) value obtained for the at least one physical parameter in a reference (experiment), e.g., performed with a DMD model, which may be patient derived or genetically engineered to resemble a clinically relevant DMD model.

Hence, the Example exemplarily illustrated two extremes, namely healthy and diseased. Any treatment, e.g. with a biologic, a drug, an ATMP, an LNP, a therapeutic such as an all-in-one AAV-CAS9-gRNA vector, etc., is expected to result in a respective potency assay as disclosed herein in a physical parameter value between said respective extremes. In case a pharmaceutical product is associated with a, preferably statistically significant, change in the value of a physical parameter compared to the respective parameter in an untreated diseased model is preferably evaluated as an indication (threshold value) that the respective pharmaceutical product is potent for treating the disorder under study. Thus, an ideal pharmaceutical product is expected to result in an at least partial, preferably a full, recovery (i.e. reversion of the determined (mean) value of the at least one physical parameter to a respective (mean) value obtained in a "healthy" reference (experiment) and/or an optimally edited reference condition (such as in case of EDIT 1-4 in this Example). It is expected that an approximately 10% change, preferably an at least approximately 12.5% or 15% change, will be found to be statistically significant when applying a potency assay according to the present invention. Deviations from this hypothesis may be caused by low sample sizes and/or technical detection limits, the person skilled in the art is aware of and capable of assessing.

### Example 6: Screening of drugs with unknown cardioactive profile using EHM

A mutation in the titin (TTN) gene can contribute to and/or cause dilated cardiomyopathy (DCM). In view of this, in this Example mutated EHM were generated and used to assess potency of several substances with unknown cardioactive profile. More specifically, the TTNtv iPSC-derived cardiomyocytes used in this Example were mutated in exon 326 of the TTN gene (c.43628insAT, p.Ser14450fsX). Said mutation in the sarcomere protein titin can cause building-up of truncated titin protein, which may contribute to (mainly dilated) cardiomyopathies (cf. e.g. Fomin et al., Science Translational Medicine, 2021, Vol 13, Issue 618, doi: 10.1126/scitranslmed.abd3079; and Gramlich et al., EMBO Mol Med (2015)7:562-576https://doi.org/10.15252/emmm.201505047). Thus, said mutation may represent an interesting candidate for targeted therapies to treat DCM.

Engineered Human Myocardium (EHM) was generated from TC1133 TTN-wt (wildtype, non-edited) and TTNtv iPSC-derived cardiomyocytes (edited, with a mutation in the TTn gene in exon 326 (c.43628insAT, p.Ser14450fsX) causing dilated cardiomyopathy (DCM)) and human fibroblasts in a 48 well plate as described in Tiburcy et al. STAR Protocols 2020. After maturing EHMs for 28 days, they were treated with 10 $\mu$M of the respective substance (2 EHM per substance; treatment conditions) or vehicle (0.2% DMSO; reference conditions) for 14 days (until day 42) with daily media changes. EHM function was monitored by weekly optical recording of pole bending. Thus, day 29 can be seen as day 1 of the drug effect testing phase, and day 42 as day 14 of the drug effect testing phase applied in this illustrative Example. The substances tested in this Example were developed with the aim to reduce the amount of abnormal protein aggregates and were thus investigated in view of their potency to treat DCM caused by the TTN c.43628insAT mutation using an EHM model.

The effects of 19 different substances (Z1-19; provided by Prof. C. Griesinger, Max-Planck for Multidisciplinary Sciences; Department NMR-based Structural Biology) on EHM function were determined by evaluating EHM shortening as a parameter for force development as well as spontaneous beating frequency. EHM shortening refers herein to pole deflection per contraction, determined as $100 \times$ (pole distance$_{complete\ relaxation}$ -pole distance$_{maximal\ contraction}$)/pole distance$_{complete\ relaxation}$. Thus, EHM shortening was determined as a physical parameter and hence, as a measure of the functionality of the physiological bodily function, which determines whether a given substance in this Example has potency for treating DCM by at least partially restoring the functionality of the measured physiological bodily function.

**Table 5** and **Table 6** summarize the mean EHM shortening (in %) of vehicle-treated reference EHM. To determine a relevant effect on EHM shortening, thresholds of 1 standard deviation (SD) or of 2 SD were defined equating to a change in EHM shortening of 18% (1x SD) or 36% (2x SD) in TTN-wt EHM and of 20% (1x SD) or 40% (2x SD) in TTNtv EHM, respectively.

**Table 5:** EHM shortening in TTN-wt reference EHM ("healthy" reference; day 42).

| EHM shortening | Mean | SD | n | 1xSD | % change | 2xSD | % change |
|---|---|---|---|---|---|---|---|
| | | | | | Thresholds | | |
| Mean (references) | 10.96% | 2.00% | 7 | 2.00% | 18% | 4.00% | 36% |

**Table 6:** EHM shortening in TTNtv reference EHM ("diseased" reference; day 42).

| EHM shortening | Mean | SD | n | 1xSD | % change | 2xSD | % change |
|---|---|---|---|---|---|---|---|
| | | | | | Thresholds | | |
| Mean (references) | 5.65% | 1.13% | 7 | 1.13% | 20% | 2.25% | 40% |

By applying a threshold of 2 standard deviations (SD) of respective reference EHM samples, a number of substances were identified, that increased or decreased EHM shortening as summarized in **Table 7.** A substance was assumed to be potent for treating DCM if a value of the at least one physical parameter (EHM shortening) exceeded the predetermined threshold for said parameter obtained in at least one of the two reference conditions (non-edited/"healthy" and/or edited/"diseased"), preferably at least in the "diseased" reference condition.

**Table 7:** Summary of effects on EHM shortening; ↑: >2SD increase, ↓: >2SD decrease, ↔: <2SD change.

| Substance | TTN-wt EHM | TTNtv EHM |
|---|---|---|
| Z1 | ↑ | ↑ |
| Z2 | ↔ | ↔ |
| Z3 | ↔ | ↔ |
| Z4 | ↔ | ↔ |
| Z5 | ↔ | ↔ |
| Z6 | ↔ | ↔ |
| Z7 | ↑ | ↔ |
| Z8 | ↔ | ↔ |
| Z9 | ↔ | ↔ |
| Z10 | ↓ | ↓ |
| Z11 | ↔ | ↔ |
| Z12 | ↑ | ↔ |
| Z13 | ↓ | ↓ |
| Z14 | ↔ | ↑ |
| Z15 | ↑ | ↔ |
| Z16 | ↑ | ↑ |
| Z17 | ↑ | ↑ |
| Z18 | ↔ | ↔ |
| Z19 | ↔ | ↔ |

**Table 8** and **Table 9** summarize spontaneous beating frequency of vehicle-treated reference EHM. To determine a relevant effect on EHM beating frequency, thresholds of 1 SD or of 2 SD were defined equating to a change in EHM beating frequency of 15% (1x SD) or 30% (2x SD) in TTN-wt EHM and of 11% (1x SD) or 22% (2x SD) in TTNtv EHM, respectively.

**Table 8:** Spontaneous beating frequency (Hz) in reference TTN-wt EHM ("healthy" reference).

| | | | | Thresholds | | | |
|---|---|---|---|---|---|---|---|
| Beating frequency | Mean | SD | n | 1xSD | % change | 2xSD | % change |
| Mean (references) | 0.37 | 0.05 | 7 | 0.05 | 15% | 0.11 | 30% |

**Table 9:** Spontaneous beating frequency (Hz) in reference TTNtv EHM ("diseased" reference).

| | | | | Thresholds | | | |
|---|---|---|---|---|---|---|---|
| Beating frequency | Mean | SD | n | 1xSD | % change | 2xSD | % change |
| Mean (references) | 0.36 | 0.04 | 7 | 0.04 | 11% | 0.08 | 22% |

By applying a threshold of 2x SD of respective reference EHM samples, substances will be identified, that increase or decrease spontaneous beating frequency. In this case, a substance will be assumed to be potent for treating DCM if a value of the at least one physical parameter (spontaneous beating frequency) exceeds the predetermined threshold for said parameter obtained in at least one of the two reference conditions (non-edited TTN-wt and/or edited TTNtv), preferably at least in the "diseased" reference condition. Furthermore, substances will be identified that increase or decrease spontaneous beating frequency and/or EHM shortening, when considering both as physical parameters to be determined for assessing potency of a given substance. In this case, a substance will be assumed to be potent for treating DCM if a value of the at least one physical parameter (EHM shortening and/or spontaneous beating frequency) exceeds the predetermined threshold for said parameter obtained in at least one of the two reference conditions (non-edited and/or edited), preferably at least in the "diseased" reference condition.

**Example 7: Potency assay for DMD rescue using EHM**

Background

In view of the results obtained from **Examples 4 to 6,** exon skipping approaches may bear the potential to at least partially alleviate a fraction of DMD phenotypes. One candidate will be investigated further as skipping exon 51 of the dystrophin gene may allow correcting more than 10% of DMD phenotypes. Eteplirsen is a commercially available drug based on an exon 51 skipping approach and received an accelerated FDA approval based on a "surrogate endpoint" rather than a direct measure of clinical benefit, as required for traditional FDA approval. Hence, a potency assay for DMD rescue will be performed for Eteplirsen to illustrate the commercial, regulatory and scientific relevance of the method according to the present invention, exemplified in the context of DMD and using EHM. The physical parameters assessed herein illustratively for the assays for Eteplirsen in the DMD EHM model will be EHM shortening (cf. **Example 6**), spontaneous beating frequency (cf. **Example 6**) and maximum systolic force amplitude (FOC) (cf. **Examples 4 and 5**). The focus physical parameter will be FOC as force of contraction will represent the expected primary target of Eteplirsen.

Eteplirsen will be chosen as an illustrative drug and in particular as an example of a nucleotide therapeutic. Eteplirsen is indicated for DMD patients with a confirmed mutation in the dystrophin gene that is amenable to exon 51 skipping. Said indication has been approved under accelerated FDA approval based on an increase in dystrophin in skeletal muscle observed in some patients treated with Eteplirsen. Continued approval may be contingent upon verification of a clinical benefit in confirmatory trials, which are time consuming and cost intensive. Publicly available clinical data currently suggest that treatment with Eteplirsen can increase expression of dystrophin protein by only approximately 1% (cf. e.g. EMA/691796/2018; and/or Aartsma-Rus and Krieg, Nucleic Acid Therapeutics, 2017, Vol. 27, Number 1, doi: 10.1089/nat.2016.0657). Thus, in this Example it is hypothesized that in a DMD EHM model, no significant improvement of any one of the chosen physical parameters, including the assumed primary target physical parameter FOC, will be observed.

Study Design

Engineered Human Myocardium (EHM) will be generated from induced pluripotent stem cells obtained from healthy individuals as well as from patients with DMD amenable to exon 51 skipping using a method described by Tiburcy et al. (2017, Defined Engineered Human Myocardium with Advanced Maturation for Applications in Heart Failure Modelling and Repair. Circulation 135:1832-1847). To assess the three physical parameters, five groups with seven replicates each will be investigated representing reference and treatment conditions, respectively. In particular, EHM of healthy individuals and EHM ("healthy" reference) of DMD patients without Eteplirsen treatment ("diseased" reference) will represent references (reference conditions), while treatment conditions will comprise EHM of DMD patients treated with "low", "standard" and "high" Eteplirsen dosages. To decide on respective concentrations, serum concentrations after clinically applied Eteplirsen concentrations, e.g. as reported in the CHMP assessment report (EMEA/H/C/004355/0000)), may be used as an estimate for the "standard" treatment, while for additionally a broader range will be tested, e.g. ranging from approximately 1 log higher to approximately 1 log lower than such (reported) serum concentrations and/or clinically effective concentrations. Thus, for example the "high" treatment may be 1 log higher and the "low" treatment 1 log lower than the concentration used for the "standard" treatment". For the potency assays, EHM will be incubated with Eteplirsen for 14 days in the indicated concentration of the assigned group. Physical parameter determination will be done as described in **Examples 4 to 6**. All physical parameter values will be obtained on day 14 of incubation with or without Eteplirsen. The predetermined threshold of the at least one physical parameter (EHM shortening, spontaneous beating frequency and/or FOC) will be 2x SD of the (mean) value of said parameter observed in respective reference EHM not incubated with the pharmaceutical product Eteplirsen.

All three physical parameters are expected to showed ascending values within treatment concentrations, with Eteplirsen in its "standard" treatment concentration showing a higher (mean) value than in its "low" treatment concentration, though lower than its "high" treatment concentration. Overall, incubation with Eteplirsen, independent of the chosen concentration, is not expected to result in a determined (mean) EHM shortening value that will meet the respective predetermined threshold obtained in at least one of the two reference conditions, preferably at least in the "diseased" reference condition, which would indicate that Eteplirsen has no potency for treating DMD by at least partially restoring the functionality of the physiological bodily function, measured as EHM shortening. However, in case at least the "high" treatment group will show a (mean) EHM shortening value that will meet the respective predetermined threshold obtained in at least one of the two reference conditions, preferably at least in the "diseased" reference condition, Eteplirsen will be assessed as having the potency for treating DMD by at least partially restoring the functionality of the physiological bodily function, measured as EHM shortening. Also in case of spontaneous beating frequency (in Hz), incubation with Eteplirsen is not expected to result in any determined (mean) spontaneous beating frequency value that will meet the respective predetermined threshold obtained in at least one of the two reference conditions, preferably at least in the "diseased" reference condition, which would indicate that Eteplirsen has no potency for treating DMD by at least partially restoring the functionality of the physiological bodily function, measured as spontaneous beating frequency (in Hz). However, in case at least the "high" treatment group will show a (mean) spontaneous beating frequency value that will meet the respective predetermined threshold obtained in at least one of the two reference conditions, preferably at least in the "diseased" reference condition, Eteplirsen will be assessed as having the potency for treating DMD by at least partially restoring the functionality of the physiological bodily function, measured as spontaneous beating frequency. Also in case of FOC, incubation with Eteplirsen in any of the investigated concentrations is not expected to result in a determined (mean) FOC value that will meet the predetermined threshold obtained in at least one of the two reference conditions, preferably at least in the "diseased" reference condition, which would indicate that Eteplirsen has no potency for treating DMD by at least partially restoring the functionality of the physiological bodily function, measured as FOC. However, in case at least the "high" treatment group will show a (mean) FOC value that will meet the respective predetermined threshold obtained in at least one of the two reference conditions, preferably at least in the "diseased" reference condition, Eteplirsen will be assessed as having the potency for treating DMD by at least partially restoring the functionality of the physiological bodily function, measured as FOC. In case expectations will be met, Eteplirsen will not be considered potent for treating DMD. In particular, Eteplirsen, will not be assumed to be potent for treating DMD as it is not expected that a value of any one of the at least one physical parameter (EHM shortening, spontaneous beating frequency and/or FOC) will exceed the predetermined threshold for said parameter obtained in at least one of the two reference conditions, preferably at least in the "diseased" reference condition.

Generally, a pharmaceutical product (like Eteplirsen) is preferably considered to be potent for treating a given disorder (like DMD) using a potency assay disclosed herein, if a concentration dependent effect can be observed and/or if, in case of one or more (tested) concentrations of the pharmaceutical product, at least one determined (mean) value of the at least one physical parameter exceeds the respective predetermined threshold, preferably at least in case of the "diseased" reference condition.

Furthermore, without being bound to theory it is believed that a lack of efficacy of Eteplirsen may result from the fact that it may not reach (at least not in an effective amount) the intended target cells to exert its effect. Alternative formulations (e.g. by chemical and/or nanopharmacological modifications such as packaging in nanoparticles with or without target organ/cell specificity) or alternative nucleotide therapeutics may be able to enhance therapeutic activity of Eteplirsen in situ. However, the identification of such formulations requires suitable platforms and approaches that have been lacking so far. This problem is solved by the potency assay as disclosed herein. The potency assay disclosed herein offers the opportunity to test the potency of a pharmaceutical product and/or its alternative formulations, e.g., in engineered skeletal and heart muscle. This will allow for the determination of patient-specific utility and dosing. As regards Eteplirsen as illustrative example, this will be enabled and/or supported by the fact that the potency assay disclosed herein can use as read-out -and thus a physical parameter- e.g. force of contraction instead of currently investigated surrogates like dystrophin protein levels. The importance of a direct read-out is also shown by **Example 5** in which DMD was expressed "again" in EDIT 5-6, but without clear and/or detectable functional improvement.

### Example 8: Potency assays using Engineered Skeletal Muscle (ESM)

Another major effect of DMD can be observed in skeletal muscles. Thus, Engineered Skeletal Muscle (ESM) will be used for another Eteplirsen potency assay in the context of DMD.

ESM will be generated as disclosed in Shahriyari et al. (Engineered skeletal muscle recapitulates human muscle development, regeneration, and dystrophy, 2022, Journal of Cachexia, Sarcopenia and Muscle, DOI: 10.1002/jcsm.13094) using iPSCs derived from healthy subjects and patients with DMD amenable to exon 51 skipping. ESM will be incubated with different Eteplirsen concentrations as described in **Experiment 7.** The physical parameter assessed for the potency assay for Eteplirsen in the DMD ESM model will be contraction force as described in Shahriyari et al. 2022, preferably determined using a stimulation frequency of 40 Hz or 100 Hz.

In Shahriyari et al. 2022, it is disclosed that absence of dystrophin in DMD ESM caused a marked reduction of contractile force determined as twitch tension (-35 $\pm$ 7%, P < 0.05) compared to a rescued phenotype after genome editing with a genome editing approach as described in **Example 5.** Thus, approximately 40% change may be expected for a genetically repaired model. However, a pharmaceutically relevant change of a physical parameter is expected to be much lower, such as about 10% compared to a reference (experiment). In view of this consideration, the predetermined threshold for the determined twitch tension value observed in Eteplirsen incubated DMD ESM models is expected to relate to an approximately 0.5x SD or 1x SD, and/or to an x-fold SD in the approximate range of 0.5x to 1x SD, increase in twitch tension compared to the respective twitch tension value obtained and/or determined for a genetically rescued and thus, permanent genetic repair engineered ESM, an untreated DMD ESM reference ("diseased" reference) and/or a healthy subject ("healthy" reference).

As in case of **Example 7,** it is not expected that Eteplirsen will result in a considerable change of a physical parameter and hence, also determined twitch tension values in Eteplirsen treated "standard" and/or "high" concentration ESM models are not expected to meet and/or exceed the predetermined threshold obtained in at least one of the reference conditions, preferably at least in the "diseased" reference condition, which would not indicate that Eteplirsen has potency for treating DMD. This will be in line with expectations as mean dystrophin re-expression under Eteplirsen treatment was shown to be not more than 1% in muscle tissue (see, e.g.,Figure 6 in CHMP assessment report (EMEA/H/C/004355/0000)). As shown herein in **Figure 4,** more than 10% recovery of dystrophin expression was determined as essential to recover muscle function. However, in case at least the "high" treatment group will (unexpectedly) show a (mean) twitch tension value that will meet the respective predetermined threshold obtained in at least one of the reference conditions, preferably at least in the "diseased" reference condition, Eteplirsen will be assessed as having the potency for treating DMD by at least partially restoring the functionality of the physiological bodily function, measured as twitch tension in an ESM model.

### Example 9: Drug effect assessment on BENOs using MEA

Brain organoids are advantageous for modeling developmental malformations such as microcephaly and lissencephaly. BENOs exhibit an excitatory-inhibitory interplay that is advantageous in testing pro-convulsive and anti-convulsive medication.

#### Method

BioEngineered Neuronal Organoids (BENOs) were generated in line with the BENO generation protocol provided in Zafeiriou et al. (Nature Communications, 2020, 11:3791, https://doi.org/10.1038/s41467-020-17521-w).

The method used for this illustrative example was a multi-electrode array (MEA) recording. In particular, six-well plates with 64 platinum microelectrodes arrays per well (0.04 M$\Omega$/microelectrode, 30 $\mu$m microelectrode diameter, 200 $\mu$m spacing) were coated with Matrigel™ 1:120 diluted in PBS for 1 h at room temperature prior seeding. One or two slices (thickness: 300 $\mu$m) were placed into a MEA well and immobilized by a concentrically coiled tungsten ring. 10-15 min recordings/2 h were performed every other day for up to 60 days using a Maestro pro MEA system (Axion Biosystems). Data recordings were automatically scheduled with the Axion Software (AxIS Navigator) using the manufacturer's Spontaneous Neural Configuration. Data analysis was performed using the manufacturer's standalone tools, Neural Metric Tool, and Axis Metric Plotting Tool (Axion Biosystems). The spike detecting threshold was set to 5.5 standard deviations, and the electrodes that detected at least 5 spikes per min were classified as active. Spike bursts were identified using an ISI threshold requiring a minimum number of five spikes with a maximum ISI of 100 ms. Network Bursts (NB) were identified by an envelope algorithm with a threshold of 1.25, minimum IBI interval of 100 ms, 75% burst inclusion with a minimum of 10% active electrodes.

Treatments

Picrotoxin (PIC) was used as an illustrative pro-convulsive medication. Cyanquixalin (CNQX) was used as an illustrative example of a general excitatory neuron inhibitor. It is to be noted that PIC and CNQX may also be investigated as a positive control in a potency assay, as disclosed herein, when assessing potency of a pharmaceutical product with activity increasing and reducing effect, respectively. This may be especially the case for a disorder like epilepsy. Using PIC and/or CNQX as a positive control may be advantageous to investigate the building of interconnected BENO cells and/or of functional integrated networks. However, it is understood by the skilled artisan that the choice of a suitable positive control depends on the disease. For example, in case of Parkinson's Disease a positive control may be advantageous that dopamine production / release and/or dopaminergic neuron levels.

**Table 10** lists treatment details for **Example 9.**

**Table 10: Overview of treatment parameters.**

| | |
|---|---|
| Day of tissue analysis | 60 |
| Type of measurement | MEA Axion Maestro pro |
| Type of plate | 6 well, 60 electrodes |
| Type of treatment | Picrotoxin (PIC) 58 $\mu$M, Non-competitive GABA receptor antagonist, Pro-convulsive medication |
| | Cyanquixalin (CNQX) 15 $\mu$M, Competitive AMPA/Kainate receptor antagonist |
| Analysis software | Axis Navigator |
| Configuration | Neural Spontaneous |
| Duration of recording | 10min |
| Duration of treatment | 10min |
| Number of tissues | 4 |

Investigated parameters and their respective observed ranges are listed in Table 11.

**Table 11: Overview of investigated general parameters for the time period under study (day 50 to day 60 of BENO development).**

| Parameter | Observed range |
|---|---|
| Weighted Mean Firing Rate | 1.5 Hz $\pm$ 0.2 |
| Number of Active Electrodes | 32 $\pm$ 2 |
| Burst Frequency (Hz) | 0.08 $\pm$ 0.01 |
| Interspike Interval in a burst (s) | 0.02 $\pm$ 0.001 |
| Network Burst (NB) Frequency | 0.08 $\pm$ 0.01 |
| Interspike Interval in a NB (s) | 0.002 $\pm$ 0.0002 |
| NB Duration (s) | 3 $\pm$ 0.4 |
| NB percentage | 69 $\pm$ 2 |

Results

**Table 12** lists investigated physical parameters and respective thresholds expressed both as standard deviations (SD) and % change determined for untreated reference BENOs and for subsequent use as predetermined threshold for treated BENOs to assess PIC and CNQX potency.

**Table 12: Overview of investigated general parameters for the time period under study (day 50 to day 60 of BENO development) with parameter values observed for reference tissues**

| Parameter | Mean | SD | n | Thresholds | | | |
|---|---|---|---|---|---|---|---|
| | | | | 1xSD | % change | 2xSD | % change |
| Weighted Mean Firing Rate (Hz) | 1.7 | 0.5 | 4 | 0.5 | 29 | 1 | 59 |
| Number of Active Electrodes | 33 | 10 | 4 | 10 | 30 | 20 | 61 |
| Burst Frequency (Hz) | 0.04 | 0.01 | 4 | 0.01 | 25 | 0.02 | 50 |
| Interspike Interval in a burst (s) | 0.02 | 0.003 | 4 | 0.003 | 15 | 0.006 | 30 |
| Network Burst (NB) Frequency | 0.03 | 0.015 | 4 | 0.015 | 50 | 0.03 | 100 |
| Interspike Interval in a NB (s) | 0.002 | 0.0007 | 4 | 0.0007 | 35 | 0.0014 | 70 |
| NB Duration (s) | 1.8 | 0.1 | 3* | 0.1 | 6 | 0.2 | 11 |
| NB percentage | 72 | 20 | 4 | 20 | 28 | 40 | 56 |
| *Outlier identified by the ROUT method with a Q of 1%. | | | | | | | |

By applying a threshold of 2x SD of reference BENOs (thus requiring a physical parameter value deviating by more than 2x SD from reference BENOs in this example), picrotoxin (PIC) and Cyanquixalin (CNQX) were found to increase or decrease at least a fraction of the determined BENO activity parameters (summarized in **Table 13**).

**Table 13: Summary of effects of PIC and CNQX on BENO activity parameters; T: >2SD increase, ↓: >2SD decrease, ↔: <2SD change, -: no evaluation possible**

| Parameter | PIC | CNQX |
|---|---|---|
| Weighted Mean Firing Rate (Hz) | ↑ | ↓ |
| Number of Active Electrodes | ↔ | ↓ |
| Burst Frequency (Hz) | ↑ | ↔ |
| Interspike Interval in a burst (s) | ↔ | ↑ |
| Network Burst (NB) Frequency | ↑ | ↓ |
| Interspike Interval in a NB (s) | ↔ | - |
| NB Duration (s) | ↓ | - |
| NB percentage | ↔ | - |

Parameter value ranges theoretically to be expected as well as observed parameter values are summarized in **Table 14. Figure 6C** graphically depict the observed parameter values listed in the **Table 14.** As regards **Table 14**.it is noted that in case of the two physical parameters Interspike Interval in a Burst and Interspike Interval in a NB ranges are indicated from large to small as smaller values are better for the BENO functionality.

**Table 14: Overview of investigated general parameters for the time period under study (day 50 to day 60 of BENO development) with theoretically expected value ranges, parameter values observed for reference, PIC treatment and CNQX treatment, respectively.**

| | Range | Reference | PIC | CNQX |
|---|---|---|---|---|
| Weighted Mean Firing Rate (Hz) | 1.0-10.0 | 1.7 ± 0.5 | 4.7 | 0.6 |
| Number of Active Electrodes | 0-60 | 33 ± 10 | 23 | 2 |
| Burst Frequency (Hz) | 0-0.08 | 0.04 ± 0.01 | 0.2 | 0.02 |
| Interspike Interval in a burst (s) | 0.06-0.01 | 0.02 ± 0.003 | 0.02 | 0.03 |
| Network Burst (NB) Frequency | 0-0.5 | 0.03 ± 0.015 | 0.09 | 0 |
| Interspike Interval in a NB (s) | 0.03-0.001 | 0.002 ± 0.0007 | 0.002 | - |
| NB Duration (s) | 1.0-15.0 | 1.8 ± 0.1 | 1.4 ± 0.4 | - |
| NB percentage | 0-100 | 72 ± 20 | 43 ± 42 | - |

Picrotoxin treatment resulted in increased spontaneous activity. Weighted mean firing rate, burst and network burst frequency increased once the inhibitory GABAergic network was inhibited. Network burst complexity as demonstrated by network burst duration was decreased. This indicated that the GABAergic network may contribute to network complexity.

Binding of CNQX to the glutamatergic receptor (AMPA), inhibited the binding of gluatamate, resulting in diminishment of synaptic activity. This was exemplified by a decrease in weighted mean firing rate, NB frequency. Burst frequency was reduced to the predetermined threshold of $2\times$ SD, though not exceeding it as required in this Example. Interspike interval in a burst was higher after treatment due to silencing of the excitatory network. The few spikes still detected were spontaneously firing neurons. No NB were observed in CNQX treated BENO, therefore NB frequency was 0 and NB related parameters could not be evaluated.

Exemplary raster plots of neuronal network activity in a BENO on day 60 are depicted in **Figure 6.** (A) Example of a raster plot in which every electrode (EI) / channel activity is depicted in a line. Every spike is indicated with a black line on top of a respective plot, a burst by black lines in the main section of the respective plot and a network burst showing synchronicity between bursts is highlighted by a square with dashed lines.. All parameters as spike, burst, network burst duration, interspike interval, interburst interval are indicated in the graphic. (B) Representative activity raster plots of the same well prior and after picrotoxin treatment showing increase in firing rate, and burst frequency. This indicated that picrotoxin blocked the inhibitory network, leading to hyperactivity. (C) Bar graphs summarizing weighted mean firing rate in Hz (left) and burst frequency in Hz (right) with a PIC (picrotoxin) induced increase in both physical activities and a CNQX induced decrease in both physical activities. Horizontal lines indicate "healthy" baseline reference values (vehicle - solid line) and PIC as well as CNQX induced alterations from the baseline reference (striped lines). Potency of a therapeutic can be identified, e.g., if drug induced increases or decreases of activity would be normalized to "healthy" baseline reference values (vehicle - solid line).

Discussion

The obtained data showed that neural network function measured in BENO by MEA can be altered by proconvulsive medication such as Picrotoxin. Thus, potency assays based on BENOs are considered to be highly advantageous for assessing treatment and/or drug effectiveness.

This illustrative Example relates to a potential application of a potency assay as disclosed herein. In principle, genetic and non-genetic factors may lead to an altered function (preferably compared to a healthy reference) and any significant change, preferably (at leats partial) normalization to a healthy reference (mean) value, may be regarded as an indication of efficacy. Potency of a pharmaceutical product may be indicated, e.g. when (at least partial or full), normalization of function occurs.

The invention is also characterized by the following items:

1. A method for assessing the potency of a pharmaceutical product assumed to be effective in treating a disorder, preferably a genetic disorder or a non-genetic disorder, wherein the disorder results in an impaired functionality of a physiological bodily function of a subject having the disorder, wherein the method comprises:

(i) incubating an engineered tissue with the pharmaceutical product, wherein the engineered tissue is or has been modified to be representative for the disorder;
(ii) determining at least one physical parameter of the engineered tissue resulting from step (i), wherein said physical parameter is a measure of the functionality of the physiological bodily function, and
(iii) determining whether a value of the at least one physical parameter determined in step (ii) meets a predetermined threshold of said physical parameter, which determines whether the pharmaceutical product has potency,
wherein, if said value of the at least one physical parameter exceeds the predetermined threshold, potency of the pharmaceutical product is indicated for treating the disorder by at least partially restoring the functionality of the physiological bodily function.

2. The method of item 1, wherein the predetermined threshold is based on and/or obtained by a reference (experiment) using a statistical method, and wherein the predetermined threshold is preferably a value deviating from a (mean) reference value obtained for the at least one physical parameter in a reference (experiment) by 1x SD (standard deviation), 1.5x or 2x SD, optionally expressed as % change of the (mean) reference value.

3. The method of any one of the preceding items, wherein the engineered tissue comprises one or more of heart tissue, heart muscle, muscle tissue, skeletal muscle tissue, neuronal tissue or connective tissue.

4. The method of any one of the preceding items, wherein the engineered tissue has been genetically edited to comprise a mutation being seen as causative for the (genetic) disorder.

5. The method of any one of the preceding items, wherein the engineered tissue has been subjected to a non-genetic intervention to be representative for the disorder.

6. The method of any one of the preceding items, wherein the pharmaceutical product is an advanced therapy medicinal product (ATMP) such as a gene therapy medicinal product (GTMP), a somatic cell therapy medicinal product (SCTMP), or a tissue-engineered product (TEP); a small molecule, a peptide, a protein, a nucleic acid, synthetic RNA, non-coding RNA, a ribonucleoprotein particle (RNP) complex, or a read-through enhancer.

7. The method of item 6, wherein the GTMP is a viral vector, a nanoparticle preferably comprising a nucleic acid and/or proteins, preferably comprised in RNP, or a nucleic acid such as an antisense oligonucleotide.

8. The method of item 6 or 7, wherein the GTMP is a synthetic or a viral vector, such as a retrovirus such as an adenovirus or an adeno-associated virus (AAV) or a lentivirus, more preferably an AAV is selected from the group consisting of AAV1, AAV2, AAV3, AAV4, AAV5, AAV6, AAV7, AAV8, AAV9, AAV10, AAV11, AAVrh10, AAVrh74 or any combination thereof, preferably AAV2 or AAV9.

9. The method of any one of the preceding items, wherein the pharmaceutical product comprises a nucleic acid encoding a protein for gene editing or comprising an expression product thereof such as an endonuclease, e.g., a zinc-finger nuclease (ZFN), a transcription activator-like effector nuclease (TALEN) or a Clustered Regularly Interspaced Short Palindromic Repeats (CRISPR) nuclease.

10. The method of any one of items 7-9, wherein the GTMP

comprises an endonuclease such as Cas9 or Cas13, preferably *Streptococcus pyogenes* Cas9 (SpCas9), *S. aureus* Cas9, *S. auricularis* Cas9, *S. lugdunensis* Cas9, or *N. meningitides* Cas9, and a guide RNA (gRNA); or comprises a nucleic acid encoding an endonuclease such as Cas9 or Cas13, preferably *Streptococcus pyogenes* Cas9 (SpCas9), *S. aureus* Cas9, *S. auricularis* Cas9, *S. lugdunensis* Cas9, or *N. meningitides* Cas9, and one or more guide RNA (gRNA).

11. The method of any one of the preceding items, wherein the physical parameter is one or more selected from the group consisting of length, mass, time, electrical current, temperature, luminous intensity, and physical parameters derived from the foregoing.

12. The method of any one of the preceding items, wherein the physical parameter is one or more selected from the group consisting of force, movement, electrical current, mass, tissue structure (swelling/condensation, lucidity), or one or more biomechanical parameters (tissue compaction, contraction, stiffness, ultimate strength, elasticity, extensibility, excitability, resilience, toughness, etc), iii) one or more selected from the group consisting of tissue contraction, stiffness, force (development), spontaneous beating frequency, contraction time and/or velocity, relaxation time and/or velocity, force (of contraction), contractile force, and one or more MEA measures; preferably measured as pole deflection, Young's modulus, (EHM) shortening, spontaneous beating frequency, contraction time and/or velocity, relaxation time and/or velocity, force of contraction, twitch tension, resting tension, and one or more from the group consisting of (Weighted) Mean Firing Rate, Burst Frequency (Hz), Interspike Interval in a burst (ms), Network Burst (NB) Frequency, Interspike Interval in a NB (ms), and NB Duration (s), respectively.

13. The method of any one of the preceding items, wherein the at least one physical parameter is not a gene or protein expression level.

14. The method of any one of the preceding items, wherein the engineered tissue is human, from a non-human primate such as macaque or marmoset, from a pig, from a rodent such as mouse, rat or guinea pig.

15. The method of any one of the preceding items, wherein the engineered tissue comprises cells obtained from a patient, who will undergo a treatment with the pharmaceutical product.

16. The method of any one of items 1-14, wherein the engineered tissue comprises cells not obtained from the patient, who will undergo a treatment with the pharmaceutical product.

17. The method of any one of the preceding items, wherein the method further comprises the analysis of a biomarker such as gene or protein expression of the biomarker.

18. The method of any one of the preceding items, wherein the engineered tissue is heart muscle.

19. The method of any one of the preceding items, wherein the at least one physical parameter is force of the engineered tissue and/or movement of the engineered tissue.

20. The method of any one of items 18 or 19, wherein the one or more conditions comprise that the engineered tissue exerts a force of 0.01 mN or more, 0.05 mN or more, 0.1 mN or more, or 1 mN or more.

21. The method of any one of the preceding items, wherein the disorder is a genetic heart condition, preferably a genetic heart condition selected from the group consisting of hereditary heart disease such as hereditary forms of dilated, hypertrophic, and arrhythmogenic cardiomyopathies or fibroblastopathies as well as inborn errors of metabolism-associated cardiomyopathies, more preferably a condition selected from the group consisting of Duchenne muscular dystrophy (DMD), Noonan syndrome, diastolic cardiomyopathy, hypertrophic cardiomyopathy, arrhythmogenic right ventricular cardiomyopathy, long and short QT syndrome, Takotsubo syndrome, a lysosomal storage disorder, titinopathy and Barth syndrome.

22. The method of any one of items 1-20, wherein the disorder is a non-genetic heart condition, preferably a non-genetic heart condition induced by simulated neurohormonal and/or pharmacological stimulation (e.g. by a catecholamine, an angiotensin, and/or transforming growth factor β), by a drug such as doxorubicin, a tyrosine kinase inhibitor and/or a cardiotoxic drug, by mechanical injury such as crush injury, by thermal injury such as freezing or overheating, by a biophysical injury such as radiation injury, by infection such as a virus infection with cardiotropic virions such as coxsackie, SarsCov, cytomegaly virus and Dengue virus, or a parasitic infection such as Chagas disease with Trypanosoma cruzi, optionally by a bodily fluid such as a serum from a patient with a heart disease, and/or optionally by cells such as blood derived mononuclear cell cytes (e.g., T-cells, B-cells, NK-cells, macrophages) from patients with heart disease.

23. The method of any one of items 1-17, wherein the engineered tissue is neuronal tissue, preferably a neuronal organoid.

24. The method of any one of items 1-17 and 22, wherein the at least one physical parameter is electrical current and/or activity.

25. The method of any one of items 22 or 23, wherein the one or more conditions comprise local and coordinated electrical bursts, and/or clusters.

26. The method of any one of items 23-25, wherein the disorder is a neuronal condition, preferably selected from the group consisting of neurodegenerative diseases (e.g., dementia, Parkinson disease, M. Huntington), neuroinflammatory diseases (e.g., multiple sclerosis), brain inflammation disorders (e.g., meningitis), channelopathy (e.g., epilepsy) and psychiatric diseases (including autism and schizophrenia).

27. The method of any one of items 1-17, wherein the engineered tissue is muscle tissue, preferably skeletal muscle tissue.

28. The method of any one of items 1-17 and 27, wherein the at least one physical parameter is force or movement.

29. The method of any one of items 27 or 28, wherein the one or more conditions comprise that the engineered tissue exerts a force of 0.01 mN or more, 0.05 mN or more, 0.1 mN or more, or 1 mN or more.

30. The method of any one of items 27-29, wherein the disorder is a non-genetic or a genetic muscular disorder, preferably a genetic muscular disorder and/or a genetic muscular disorder selected from the group consisting of Duchenne muscular dystrophy, Facioscapulohumeral dystrophy, Becker dystrophy, Emery-Dreifuss dystrophy, myotonic dystrophy, limb-girdle dystrophy, oculopharyngeal muscular dystrophy, congenital dystrophies, congenital myopathies, myotonia congenita, familial periodic paralysis, and a heritable connective tissue disease such as Marfan syndrome, Ehlers-Danlos syndrome or Loeys-Dietz syndrome.

31. The method of any one of items 1-17 and 27-30, wherein the engineered tissue is a composition of different types of tissues, preferably a composition of:

(i) neurons and skeletal muscle, or
(ii) neurons and heart muscle;

wherein more preferably the composition of different types of tissues forms one or more neuro-muscular junction.

32. The method of any one of items 1-17, wherein

the engineered tissue is heart muscle or skeletal muscle,
the pharmaceutical product is an AAV2 and/or AAV9 viral vector comprising a nucleic acid encoding Cas9, preferably *S. aureus* Cas9, *S. auricularis* Cas9, *S. lugdunensis* Cas9, or *N. meningitides* Cas9, and one or two guide RNA(s) (gRNA), wherein the Cas9 gene is under the control of a muscle-specific promoter such as CK8e or TNNT2,
the disorder is Duchenne muscular dystrophy, and
said physical parameter is force,
preferably wherein the engineered tissue comprises cells obtained from a patient, who will undergo a treatment with the pharmaceutical product.

33. The method of any one of items 1-17, wherein the engineered tissue is connective tissue.

34. The method of any one of items 1-17 and 33, wherein the at least one physical parameter is a biomechanical parameter such as tissue compaction, contraction, stiffness, ultimate strength, elasticity, extensibility, excitability, resilience or toughness.

35. The method of any one of items 33 and 34, wherein the disorder is (i) a heart disease which presents with cardiac fibrosis, such as a heart failure (of unknown and/or genetic cause) with reduced or preserved ejection fraction, myocardial infarction, a congenital heart disease, a cardiomyopathy with known mutation(s), age- and/or senescence-induced cardiac fibrosis, a drug-induced cardiac disorder, a metabolic cardiac disorder, a (monogenetic or polygenetic) disorder with contribution of the heart and cardiac fibrosis, a disease involving reactive or replacement fibrosis in an organ such as kidney, liver, lung, and/or skin, etc., (ii) a disorder with impaired scaring and wound healing such as Diabetes mellitus, and/or (iii) a heritable connective tissue disease such as Marfan syndrome, Ehlers-Danlos syndrome or Loeys-Dietz syndrome.

It will be readily apparent to a person skilled in the art that varying substitutions and modifications may be made to the invention disclosed herein without departing from the scope and spirit of the invention.

The inventions illustratively described herein may suitably be practiced in the absence of any element or elements, limitation or limitations, not specifically disclosed herein. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

**Claims**

1. A method for assessing the potency of a pharmaceutical product assumed to be effective in treating a disorder, preferably a genetic disorder, wherein the disorder results in an impaired functionality of a physiological bodily function of a subject having the disorder, wherein the method comprises:

   (i) incubating an engineered tissue with the pharmaceutical product, wherein the engineered tissue is or has been modified to be representative for the disorder;
   (ii) determining at least one physical parameter of the engineered tissue resulting from step (i), wherein said physical parameter is a measure of the functionality of the physiological bodily function, and
   (iii) determining whether a value of the at least one physical parameter determined in step (ii) meets a predetermined threshold of said physical parameter, which determines whether the pharmaceutical product has potency,
   wherein, if said value of the at least one physical parameter exceeds the predetermined threshold, potency of the pharmaceutical product is indicated for treating the disorder by at least partially restoring the functionality of the physiological bodily function.

2. The method of claim 1, wherein the predetermined threshold is based on and/or obtained by a reference (experiment) using a statistical method, and wherein the predetermined threshold is preferably a value deviating from a (mean) reference value obtained for the at least one physical parameter in a reference (experiment) by (at least) between 0.5x and 2.5x standard deviations (SD) and/or by (at least) 0.5x SD, 1x SD, 1.5x or 2x SD, optionally expressed as % change of the (mean) reference value.

3. The method of any one of the preceding claims, wherein the engineered tissue comprises one or more of heart tissue, heart muscle, muscle tissue, skeletal muscle tissue, neuronal tissue or connective tissue.

4. The method of any one of the preceding claims, wherein the engineered tissue i) has been genetically edited to comprise a mutation being seen as causative for the (genetic) disorder, and/or ii) has been subjected to a non-genetic intervention to be representative for the disorder.

5. The method of any one of the preceding claims, wherein the pharmaceutical product is an advanced therapy medicinal product (ATMP) such as a gene therapy medicinal product (GTMP), a cell therapy medicinal product (CTMP), or a tissue-engineered product (TEP); a small molecule, a peptide, a protein, a nucleic acid, synthetic RNA, non-coding RNA, a ribonucleoprotein particle (RNP) complex, or a read-through enhancer; preferably wherein the GTMP is a viral vector, a nanoparticle preferably comprising a nucleic acid and/or proteins, preferably comprised in RNP, or a nucleic acid such as an antisense oligonucleotide, and/or wherein the GTMP is a synthetic or a viral vector, such as a retrovirus such as an adenovirus or an adeno-associated virus (AAV) or a lentivirus, more preferably an AAV is selected from the group consisting of AAV1, AAV2, AAV3, AAV4, AAV5, AAV6, AAV7, AAV8, AAV9, AAV10, AAV11, AAVrh10, AAVrh74 or any combination thereof, preferably AAV2 or AAV9.

**6.** The method of any one of the preceding claims, wherein the pharmaceutical product comprises a nucleic acid encoding a protein for gene editing or comprising an expression product thereof such as an endonuclease, e.g., a zinc-finger nuclease (ZFN), a transcription activator-like effector nuclease (TALEN) or a Clustered Regularly Interspaced Short Palindromic Repeats (CRISPR) nuclease.

**7.** The method of any one of claim 5 or 6, wherein the GTMP

comprises an endonuclease such as Cas9 or Cas13, preferably *Streptococcus pyogenes* Cas9 (SpCas9), *S. aureus* Cas9, *S. auricularis* Cas9, *S. lugdunensis* Cas9, or *N. meningitides* Cas9, and a guide RNA (gRNA); or comprises a nucleic acid encoding an endonuclease such as Cas9 or Cas13, preferably *Streptococcus pyogenes* Cas9 (SpCas9), *S. aureus* Cas9, *S. auricularis* Cas9, *S. lugdunensis* Cas9, or *N. meningitides* Cas9, and one or more guide RNA (gRNA).

**8.** The method of any one of the preceding claims, wherein said physical parameter is i) one or more selected from the group consisting of length, mass, time, electrical current, temperature, luminous intensity, and physical parameters derived from the foregoing, ii) one or more selected from the group consisting of force, movement, electrical current, mass, tissue structure (swelling/condensation, lucidity), or one or more biomechanical parameters (tissue compaction, contraction, stiffness, ultimate strength, elasticity, extensibility, excitability, resilience, toughness, etc), iii) one or more selected from the group consisting of tissue contraction, stiffness, force (development), spontaneous beating frequency, contraction time and/or velocity, relaxation time and/or velocity, force (of contraction), contractile force, and one or more multi-electrode array (MEA) measures; preferably measured as pole deflection, Young's modulus, (EHM) shortening, spontaneous beating frequency, contraction time and/or velocity, relaxation time and/or velocity, force of contraction, twitch tension, resting tension, and one or more from the group consisting of (Weighted) Mean Firing Rate, Burst Frequency (Hz), Interspike Interval in a burst (ms), Network Burst (NB) Frequency, Interspike Interval in a NB (ms), and NB Duration (s), respectively, and/or iv) not a gene or protein expression level.

**9.** The method of any one of the preceding claims, wherein i) the engineered tissue comprises cells obtained from a patient, who will undergo a treatment with the pharmaceutical product, or wherein ii) the engineered tissue comprises cells not obtained from the patient, who will undergo a treatment with the pharmaceutical product.

**10.** The method of any one of the preceding claims, wherein the engineered tissue is heart muscle, and i) wherein the at least one physical parameter is force of the engineered tissue and/or movement of the engineered tissue, ii) wherein the one or more conditions comprise that the engineered tissue exerts a force of 0.01 mN or more, 0.05 mN or more, 0.1 mN or more, or 1 mN or more, and/or iii) wherein the disorder is a genetic heart condition, preferably a genetic heart condition selected from the group consisting of hereditary heart disease such as hereditary forms of dilated, hypertrophic, and arrhythmogenic cardiomyopathies or fibroblastopathies as well as inborn errors of metabolism-associated cardiomyopathies, more preferably a condition selected from the group consisting of Duchenne muscular dystrophy (DMD), Noonan syndrome, diastolic cardiomyopathy, hypertrophic cardiomyopathy, arrhythmogenic right ventricular cardiomyopathy, long and short QT syndrome, Takotsubo syndrome, a lysosomal storage disorder, titinopathy, and Barth syndrome, or wherein the disorder is a non-genetic heart condition, preferably a non-genetic heart condition induced by simulated neurohormonal and/or pharmacological stimulation (e.g. by a catecholamine, an angiotensin, and/or transforming growth factor β), by a drug such as doxorubicin, a tyrosine kinase inhibitor and/or a cardiotoxic drug, by mechanical injury such as crush injury, by thermal injury such as freezing or overheating, by a biophysical injury such as radiation injury, by infection such as a virus infection with cardiotropic virions such as coxsackie, SarsCov, cytomegaly virus and Dengue virus, or a parasitic infection such as Chagas disease with Trypanosoma cruzi, optionally by a bodily fluid such as a serum from a patient with a heart disease, and/or optionally by cells such as blood derived mononuclear cell cytes (e.g., T-cells, B-cells, NK-cells, macrophages) from patients with heart disease.

**11.** The method of any one of claims 1-9, wherein the engineered tissue is neuronal tissue, preferably a neuronal organoid, and i) wherein the at least one physical parameter is electrical current and/or activity, ii) wherein the one or more conditions comprise local and coordinated electrical bursts, and/or clusters, and/or iii) wherein the disorder is a neuronal condition, preferably selected from the group consisting of neurodegenerative diseases (e.g., dementia, Parkinson disease, M. Huntington), neuroinflammatory diseases (e.g., multiple sclerosis), brain inflammation disorders (e.g., meningitis), channelopathy (e.g., epilepsy) and psychiatric diseases (including autism and schizophrenia).

**12.** The method of any one of claims 1-9, wherein the engineered tissue is muscle tissue, preferably skeletal muscle tissue, and i) wherein the at least one physical parameter is force or movement, ii) wherein the one or more conditions comprise that the engineered tissue exerts a force of 0.01 mN or more, 0.05 mN or more, 0.1 mN or more, or 1 mN or more, and/or iii) wherein the disorder is a genetic muscular disorder, preferably a genetic muscular disorder selected from the group consisting of Duchenne muscular dystrophy, Facioscapulohumeral dystrophy, Becker dystrophy, Emery-Dreifuss dystrophy, myotonic dystrophy, limb-girdle dystrophy, oculopharyngeal muscular dystrophy, congenital dystrophies, congenital myopathies, myotonia congenita, familial periodic paralysis, and a heritable connective tissue disease such as Marfan syndrome, Ehlers-Danlos syndrome or Loeys-Dietz syndrome.

13. The method of any one of claims 1-9, wherein the engineered tissue is a composition of different types of tissues, preferably a composition of:

(i) neurons and skeletal muscle, or
(ii) neurons and heart muscle;

wherein more preferably the composition of different types of tissues forms one or more neuro-muscular junction.

14. The method of any one of claims 1-9, wherein

the engineered tissue is heart muscle or skeletal muscle,
the pharmaceutical product is an AAV2 and/or AAV9 viral vector comprising a nucleic acid encoding Cas9, preferably *S. aureus* Cas9, *S. auricularis* Cas9, *S. lugdunensis* Cas9, or *N. meningitides* Cas9, and one or two guide RNA(s) (gRNA), wherein the Cas9 gene is under the control of a muscle-specific promoter such as CK8e or TNNT2,
the disorder is Duchenne muscular dystrophy, and
said physical parameter is force,
preferably wherein the engineered tissue comprises cells obtained from a patient, who will undergo a treatment with the pharmaceutical product.

15. The method of any one of claims 1-9, wherein the engineered tissue is connective tissue, and i) wherein the at least one physical parameter is a biomechanical parameter such as tissue compaction, contraction, stiffness, ultimate strength, elasticity, extensibility, excitability, resilience, or toughness and/or ii) wherein the disorder is (i) a heart disease which presents with cardiac fibrosis, such as a heart failure (of unknown and/or genetic cause) with reduced or preserved ejection fraction, myocardial infarction, a congenital heart disease, a cardiomyopathy with known mutation(s), age- and/or senescence-induced cardiac fibrosis, a drug-induced cardiac disorder, a metabolic cardiac disorder, a (monogenetic or polygenetic) disorder with contribution of the heart and cardiac fibrosis, a disease involving reactive or replacement fibrosis in an organ such as kidney, liver, lung, and/or skin, etc., (ii) a disorder with impaired scaring and wound healing such as Diabetes mellitus, and/or (iii) a heritable connective tissue disease such as Marfan syndrome, Ehlers-Danlos syndrome or Loeys-Dietz syndrome.

**Figure 1**

Figure 2

**Figure 3**

Figure 4

Figure 5

Figure 6 A)

**Figure 6 B)**

Figure 6 C)

wMean firing rate (Hz)

Burst Frequency (Hz)

| | Europäisches Patentamt European Patent Office Office européen des brevets | **EUROPEAN SEARCH REPORT** | Application Number EP 22 21 3839 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/117547 A1 (HARVARD COLLEGE [US]) 6 July 2017 (2017-07-06) | 1-5,8,9, 11,13 | INV. G01N33/50 |
| Y | * page 49, line 29 – page 52, line 34; claims 1-41 * | 1-15 | |
| X | CHENGZU LONG ET AL: "Correction of diverse muscular dystrophy mutations in human engineered heart muscle by single-site genome editing", SCIENCE ADVANCES, vol. 4, no. 1, 1 January 2018 (2018-01-01), pages 1-11, XP055523128, DOI: 10.1126/sciadv.aap9004 | 1-10,12, 14 | |
| Y | Results. Materials and methods.; * abstract * | 1-15 | |
| X | SANTOS GABRIELA LEÃO ET AL: "Inhibition of Rho-associated kinases suppresses cardiac myofibroblast function in engineered connective and heart muscle tissues", JOURNAL OF MOLECULAR AND CELLULAR CARDIOLOGY, ACADEMIC PRESS, GB, vol. 134, 22 June 2019 (2019-06-22), pages 13-28, XP085795976, ISSN: 0022-2828, DOI: 10.1016/J.YJMCC.2019.06.015 [retrieved on 2019-06-22] Results.; * abstract * | 1-5,8,9, 15 | |

TECHNICAL FIELDS SEARCHED (IPC)

G01N

-/--

The present search report has been drawn up for all claims

| Place of search | Date of completion of the search | Examiner |
|---|---|---|
| Munich | 17 May 2023 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 22 21 3839**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAUPT LUIS PETER ET AL: "Doxorubicin induces cardiotoxicity in a pluripotent stem cell model of aggressive B cell lymphoma cancer patients", BASIC RESEARCH IN CARDIOLOGY, SPRINGER BERLIN HEIDELBERG, BERLIN/HEIDELBERG, vol. 117, no. 1, 8 March 2022 (2022-03-08), XP037711686, ISSN: 0300-8428, DOI: 10.1007/S00395-022-00918-7 [retrieved on 2022-03-08] Results.; * abstract; figures 5, 9 * ----- | 1-5,8-10 | |
| | | | **TECHNICAL FIELDS SEARCHED (IPC)** |

The present search report has been drawn up for all claims

| Place of search | Date of completion of the search | Examiner |
|---|---|---|
| Munich | 17 May 2023 | Moreno de Vega, C |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
 
& : member of the same patent family, corresponding
   document

## ANNEX TO THE EUROPEAN SEARCH REPORT
## ON EUROPEAN PATENT APPLICATION NO.

EP 22 21 3839

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2023

| Patent document cited in search report | Publication date | Patent family member(s) | Publication date |
|---|---|---|---|
| WO 2017117547 A1 | 06-07-2017 | US 2019002835 A1 | 03-01-2019 |
| | | WO 2017117547 A1 | 06-07-2017 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2842581 A1
- US 9801817 B2
- WO 2015040142 A
- WO 20210741236 A
- EP 3945133 A
- WO 2018228948 A
- WO 2015025030 A
- WO 2021074126 A
- WO 2022023451 A1
- EP 20188364 A
- WO 2017207431 A
- DE 102016110328 B3

**Non-patent literature cited in the description**

- **LONG et al.** *Sci Adv,* 2018, vol. 4, eaap9004
- **GOULA et al.** *J Clin Med Res,* 2020, vol. 12 (12), 780-786
- **RABEA et al.** *ACS Med. Chem. Lett.,* 2019, vol. 10 (5), 726-731
- **LI et al.** *Pharm Res,* 2007, vol. 24 (3), 438-49
- **MLADENKA et al.** *Med Res Rev,* 2018, vol. 38, 1332-1403
- **TIBURCY et al.** *Circulation,* 2017, vol. 135 (19), 1832-1847
- **CHAMBERS ; FASANO et al.** *Nat. Biotechnol.,* 2009
- **LANCASTER ; KNOBLICH.** *Science,* 2014
- **QIAN ; NGUYEN et al.** *Cell,* 2016
- **ANDERSEN et al.** *Nature,* 2017
- **CRAWFORD ; ROELINK.** *Dev. Dyn.,* 2007
- **KRIKS ; SHIM et al.** *Nature,* 2011
- **LANCASTER et al.** *Nature,* 2013
- **ZAFEIRIOU et al.** *Nature Communications,* 2020, vol. 11, 3791
- **TIBURCY et al.** *FASEB Bioadv,* 2019, vol. 1 (12), 731-746
- **SHAHRIYARI et al.** *Sarcopenia Muscle,* 2022, vol. 13 (6), 3106-3121
- **SANTOS et al.** *J. Vis. Exp.,* 2021, vol. 174, e62700
- **SANTOS et al.** Fibroblast Derived Human Engineered Connective Tissue for Screening Applications. *J. Vis. Exp.,* 2021, e62700
- **SANTOS et al.** Using different geometries to modulate the cardiac fibroblast phenotype and the biomechanical properties of engineered connective tissues. *Biomater Adv.,* 2022, vol. 139, 213041
- **TIBURCY et al.** *STAR Protocols,* 2020
- **TIBURCY et al.** Defined Engineered Human Myocardium with Advanced Maturation for Applications in Heart Failure Modelling and Repair. *Circulation,* 2017, vol. 135, 1832-1847
- **TIBURCY et al.** Generation of Engineered Human Myocardium in a Multi-well Format. *STAR Protoc.,* 2020, vol. 1, 100032
- Engineering Heart Tissue for In Vitro and In Vivo Studies. **ZIMMERMANN et al.** Practical Methods in Cardiovascular Research. Springer, 2005
- **FOMIN et al.** *Science Translational Medicine,* 2021, vol. 13 (618
- **GRAMLICH et al.** *EMBO Mol Med,* 2015, vol. 7, 562-576
- **AARTSMA-RUS ; KRIEG.** *Nucleic Acid Therapeutics,* 2017, vol. 27 (1
- **SHAHRIYARI et al.** Engineered skeletal muscle recapitulates human muscle development, regeneration, and dystrophy. *Journal of Cachexia, Sarcopenia and Muscle,* 2022